(19) Europäisches Patentamt
European Patent Office
Office européen des brevets

(11) **EP 4 570 802 A1**

(12) **EUROPEAN PATENT APPLICATION**
published in accordance with Art. 153(4) EPC

(43) Date of publication:
**18.06.2025 Bulletin 2025/25**

(21) Application number: **23862284.9**

(22) Date of filing: **01.09.2023**

(51) International Patent Classification (IPC):
***C07D 487/04*** *(2006.01)* ***C07D 223/16*** *(2006.01)*
***A61K 31/55*** *(2006.01)*

(52) Cooperative Patent Classification (CPC):
**A61K 31/55; C07D 223/16; C07D 487/04**

(86) International application number:
**PCT/CN2023/116489**

(87) International publication number:
**WO 2024/051590 (14.03.2024 Gazette 2024/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **05.09.2022 CN 202211080826**

(71) Applicant: **SHANGHAI JEYOU PHARMACEUTICAL CO., LTD.**
**Shanghai 201315 (CN)**

(72) Inventors:
- **ZHANG, Yong**
  **Shanghai 201203 (CN)**
- **CAO, Cheng**
  **Shanghai 201203 (CN)**

(74) Representative: **Mewburn Ellis LLP**
**Aurora Building**
**Counterslip**
**Bristol BS1 6BX (GB)**

(54) **SALT TYPE AND CRYSTAL FORM OF BENZAZEPINE FUSED RING COMPOUND AND USE THEREOF**

(57) The present invention relates to a salt type and crystal form of a benzazepine fused ring compound and use thereof, and in particular to a maleate of a compound represented by formula (I), the structure of the maleate being represented by formula (II).

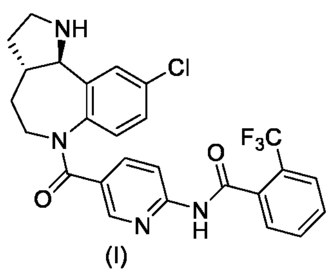
(I)

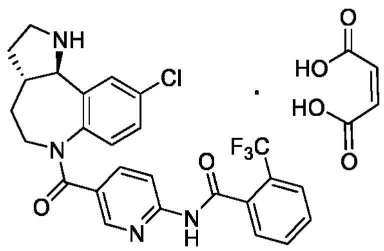
(II)

EP 4 570 802 A1

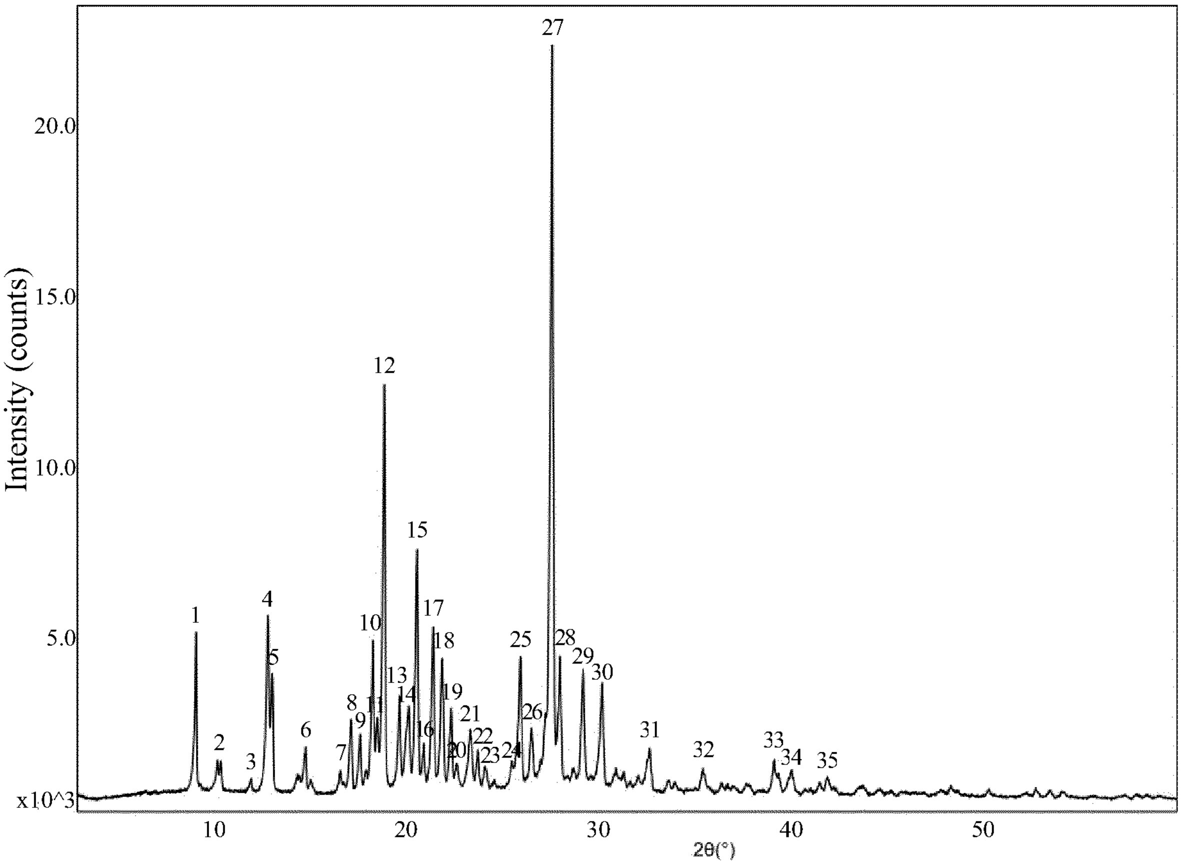
Intensity (counts)
20.0
15.0
10.0
5.0
x10^3
10
20
30
40
50
2θ(°)
1
2
3
4
5
6
7
8
9
10
11
12
13
14
15
16
17
18
19
20
21
22
23
24
25
26
27
28
29
30
31
32
33
34
35

FIG. 1

## Description

The present invention claims the right of the following priority: Application No. CN 202211080826.4, filed on September 5, 2022.

TECHNICAL FIELD

The present disclosure belongs to the field of medicinal chemistry. Specifically, the present disclosure relates to a salt type and crystal form of a benzazepine fused ring compound and a use thereof.

BACKGROUND

Hormones play an important role in the regulation of homeostasis in the human body, among which arginine vasopressin (AVP) is closely related to the regulation of water and sodium metabolism in the human body. Metabolic disturbances of arginine vasopressin (AVP) can cause a variety of diseases such as hyponatremia, syndrome of inappropriate antidiuretic hormone secretion, congestive heart failure, liver cirrhosis, kidney disease, hypertension, and edema. Arginine vasopressin (AVP) receptor antagonists can inhibit the binding of AVP to receptors, thereby providing a therapeutic effect on these diseases. Arginine vasopressin V2 receptor antagonists, represented by Tolvaptan, can increase the excretion of free water without affecting the metabolism of electrolytes, making them ideal drugs for the treatment of these diseases. However, the marketed AVP V2 receptor antagonists, such as Tolvaptan, are metabolized by liver metabolic enzymes, which produce a large number of metabolites in the body and lead to severe drug-induced hepatotoxicity. The FDA has issued a black box warning on the label of the drug product, limiting its application.

Application No. PCT/CN2022/079350, filed on March 4, 2022, provides a new AVP V2 receptor antagonist with the following structure:

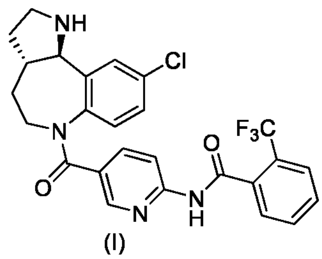

(I)

.

CONTENT OF THE PRESENT INVENTION

In one aspect of the present disclosure, the present disclosure provides a maleate of a compound represented by formula (I), whose structure is represented by formula (II),

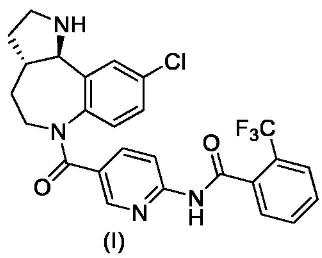

(I)

,

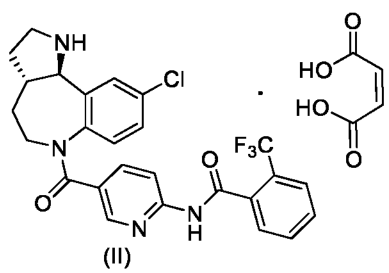

(II)

.

In another aspect of the present disclosure, the present disclosure provides a crystal form A of the maleate of the compound represented by formula (I) *(i.e.,* the compound represented by formula (II)), wherein the crystal form A has an X-ray powder diffraction pattern comprising characteristic diffraction peaks at the following 2θ angles: 9.14 ± 0.2°, 12.88 ± 0.2°, 18.31 ± 0.2°, 18.90 ± 0.2°, 20.60 ± 0.2°, and 27.61 ± 0.2°.

In some embodiments of the present disclosure, the X-ray powder diffraction pattern of the crystal form A comprises characteristic diffraction peaks at the following 2θ angles: 9.14 ± 0.2°, 12.88 ± 0.2°, 17.19 ± 0.2°, 18.31 ± 0.2°, 18.90 ± 0.2°, 20.60 ± 0.2°, 21.45 ± 0.2°, and 27.61 ± 0.2°.

In some embodiments of the present disclosure, the X-ray powder diffraction pattern of the crystal form A

comprises characteristic diffraction peaks at the following 2θ angles: 9.14 ± 0.2°, 12.88 ± 0.2°, 17.19 ± 0.2°, 18.31 ± 0.2°, 18.90 ± 0.2°, 19.70 ± 0.2°, 20.20 ± 0.2°, 20.60 ± 0.2°, 21.45 ± 0.2°, 21.91 ± 0.2°, and 27.61 ± 0.2°.

In some embodiments of the present disclosure, the X-ray powder diffraction pattern of the crystal form A comprises characteristic diffraction peaks at the following 2θ angles: 9.14 ± 0.2°, 12.88 ± 0.2°, 17.19 ± 0.2°, 18.31 ± 0.2°, 18.90 ± 0.2°, 19.70 ± 0.2°, 20.20 ± 0.2°, 20.60 ± 0.2°, 21.45 ± 0.2°, 21.91 ± 0.2°, 25.96 ± 0.2°, 26.53 ± 0.2°, 27.61 ± 0.2°, 29.22 ± 0.2°, and 30.20 ± 0.2°.

In some embodiments of the present disclosure, the X-ray powder diffraction pattern of the crystal form A has an X-ray powder diffraction pattern substantially as shown in FIG. 1.

In some embodiments of the present disclosure, the analytical data of the X-ray powder diffraction pattern of the crystal form A are as shown in Table 1 below.

Table 1

| Peak No. | 2θ (°) | Interplanar spacing (Å) | Relative intensity (%) |
|---|---|---|---|
| 1 | 9.143 | 9.6645 | 23.3 |
| 2 | 10.27 | 8.6066 | 6.6 |
| 3 | 12.003 | 7.3675 | 4.2 |
| 4 | 12.878 | 6.8684 | 25.5 |
| 5 | 13.095 | 6.7553 | 17.9 |
| 6 | 14.831 | 5.9682 | 8.3 |
| 7 | 16.63 | 5.3264 | 5.2 |
| 8 | 17.185 | 5.1557 | 11.8 |
| 9 | 17.657 | 5.0189 | 9.9 |
| 10 | 18.312 | 4.8408 | 22.2 |
| 11 | 18.53 | 4.7842 | 11.7 |
| 12 | 18.904 | 4.6905 | 55.6 |
| 13 | 19.695 | 4.5039 | 15 |
| 14 | 20.168 | 4.3992 | 13.6 |
| 15 | 20.602 | 4.3076 | 34.1 |
| 16 | 20.957 | 4.2354 | 8.7 |
| 17 | 21.45 | 4.1393 | 24 |
| 18 | 21.906 | 4.054 | 19.8 |
| 19 | 22.38 | 3.9692 | 13.3 |
| 20 | 22.642 | 3.9239 | 6.1 |
| 21 | 23.351 | 3.8063 | 10.6 |
| 22 | 23.764 | 3.7411 | 7.9 |
| 23 | 24.121 | 3.6865 | 5.7 |
| 24 | 25.578 | 3.4797 | 5.7 |
| 25 | 25.958 | 3.4297 | 20.1 |
| 26 | 26.531 | 3.3569 | 10.7 |
| 27 | 27.614 | 3.2276 | 100 |
| 28 | 28.012 | 3.1827 | 20.1 |
| 29 | 29.216 | 3.0542 | 18.4 |
| 30 | 30.203 | 2.9566 | 16.7 |
| 31 | 32.673 | 2.7385 | 8.1 |

(continued)

| Peak No. | 2θ (°) | Interplanar spacing (Å) | Relative intensity (%) |
|---|---|---|---|
| 32 | 35.42 | 2.5322 | 5.5 |
| 33 | 39.132 | 2.3001 | 6.6 |
| 34 | 40.022 | 2.251 | 5.3 |
| 35 | 41.9 | 2.1543 | 4.1 |

In another aspect of the present disclosure, the present disclosure also provides a crystal form B of the maleate of the compound represented by formula (I) (*i.e.,* the compound represented by formula (II)), wherein the crystal form B has an X-ray powder diffraction pattern comprising characteristic diffraction peaks at the following 2θ angles: 8.97 ± 0.2°, 15.73 ± 0.2°, 18.31 ± 0.2°, 20.15 ± 0.2°, 21.12 ± 0.2°, and 24.70 ± 0.2°.

In some embodiments of the present disclosure, the X-ray powder diffraction pattern of the crystal form B comprises characteristic diffraction peaks at the following 2θ angles: 7.40 ± 0.2°, 8.97 ± 0.2°, 10.11 ± 0.2°, 13.94 ± 0.2°, 15.73 ± 0.2°, 18.31 ± 0.2°, 19.02 ± 0.2°, 20.15 ± 0.2°, 21.12 ± 0.2°, and 24.70 ± 0.2°.

In some embodiments of the present disclosure, the X-ray powder diffraction pattern of the crystal form B has an X-ray powder diffraction pattern substantially as shown in FIG. 6.

In some embodiments of the present disclosure, the analytical data of the X-ray powder diffraction pattern of the crystal form B are as shown in Table 2 below.

Table 2

| 2θ | Interplanar spacing | Relative intensity (%) |
|---|---|---|
| 41.013° | 2.19889 Å | 6.1% |
| 33.595° | 2.66546 Å | 11.9% |
| 32.549° | 2.74872 Å | 11.9% |
| 31.827° | 2.80939 Å | 11.2% |
| 31.597° | 2.82929 Å | 9.8% |
| 30.769° | 2.90352 Å | 6.3% |
| 30.524° | 2.92629 Å | 13.4% |
| 30.088° | 2.96771 Å | 7.2% |
| 29.695° | 3.00609 Å | 4.6% |
| 29.463° | 3.02918 Å | 4.8% |
| 27.704° | 3.21738 Å | 11.5% |
| 27.075° | 3.29071 Å | 21.2% |
| 25.560° | 3.48230 Å | 9.6% |
| 25.369° | 3.50802 Å | 29.1% |
| 25.038° | 3.55359 Å | 28.7% |
| 24.690° | 3.60290 Å | 83.1% |
| 24.307° | 3.65881 Å | 15.2% |
| 23.929° | 3.71576 Å | 9.3% |
| 23.039° | 3.85720 Å | 37.9% |
| 21.697° | 4.09268 Å | 26.4% |
| 21.372° | 4.15414 Å | 16.3% |
| 21.122° | 4.20273 Å | 69.3% |
| 20.366° | 4.35702 Å | 4.3% |
| 20.150° | 4.40321 Å | 48.5% |

(continued)

| 2θ | Interplanar spacing | Relative intensity (%) |
|---|---|---|
| 19.172° | 4.62572 Å | 27.8% |
| 19.019° | 4.66241 Å | 33.3% |
| 18.665° | 4.75008 Å | 20.1% |
| 18.467° | 4.80072 Å | 27.7% |
| 18.307° | 4.84233 Å | 100.0% |
| 18.054° | 4.90943 Å | 20.0% |
| 16.956° | 5.22472 Å | 10.9% |
| 16.600° | 5.33610 Å | 10.8% |
| 15.733° | 5.62825 Å | 53.4% |
| 14.769° | 5.99320 Å | 5.6% |
| 13.942° | 6.34702 Å | 19.6% |
| 13.104° | 6.75096 Å | 10.8% |
| 12.564° | 7.03989 Å | 8.5% |
| 11.828° | 7.47621 Å | 7.2% |
| 10.689° | 8.27020 Å | 3.5% |
| 10.105° | 8.74649 Å | 23.5% |
| 8.966° | 9.85516 Å | 62.0% |
| 7.398° | 11.93973 Å | 12.3% |

In another aspect of the present disclosure, the present disclosure also discloses a fumarate of the compound represented by formula (I), whose structure is represented by formula (III),

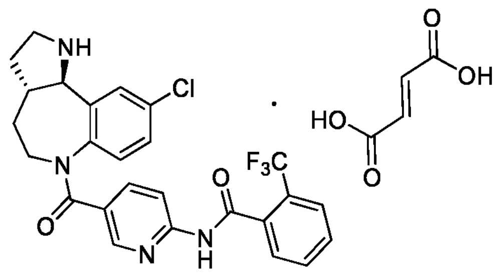

(III)

.

In another aspect of the present disclosure, the present disclosure also discloses a crystal form C of the fumarate of the compound represented by formula (I) *(i.e.,* the compound represented by formula (III)), wherein the crystal form C has an X-ray powder diffraction pattern comprising characteristic diffraction peaks at the following 2θ angles: 12.77 ± 0.2°, 14.44 ± 0.2°, 20.00 ± 0.2°, 20.64 ± 0.2°, 21.33 ± 0.2°, and 21.87 ± 0.2°.

In some embodiments of the present disclosure, the X-ray powder diffraction pattern of the crystal form C comprises characteristic diffraction peaks at the following 2θ angles: 12.77 ± 0.2°, 13.17 ± 0.2°, 14.44 ± 0.2°, 17.18 ± 0.2°, 20.00 ± 0.2°, 20.64 ± 0.2°, 21.33 ± 0.2°, 21.87 ± 0.2°, 23.43 ± 0.2°, and 25.86 ± 0.2°.

In some embodiments of the present disclosure, the X-ray powder diffraction pattern of the crystal form C has an X-ray powder diffraction pattern substantially as shown in FIG. 9.

In some embodiments of the present disclosure, the analytical data of the X-ray powder diffraction pattern of the crystal form C are as shown in Table 3 below.

Table 3

| 2θ | Interplanar spacing | Relative intensity (%) |
|---|---|---|
| 10.226° | 8.64329 Å | 7.8% |
| 11.928° | 7.41335 Å | 6.7% |
| 12.770° | 6.92687 Å | 55.3% |
| 13.172° | 6.71591 Å | 20.5% |
| 14.443° | 6.12800 Å | 40.8% |
| 15.255° | 5.80338 Å | 6.4% |
| 16.679° | 5.31086 Å | 11.7% |
| 17.177° | 5.15813 Å | 27.5% |
| 17.526° | 5.05624 Å | 5.5% |
| 18.198° | 4.87102 Å | 4.9% |
| 18.655° | 4.75276 Å | 8.4% |
| 19.767° | 4.48765 Å | 18.1% |
| 20.005° | 4.43479 Å | 34.1% |
| 20.637° | 4.30039 Å | 32.5% |
| 21.326° | 4.16310 Å | 100.0% |
| 21.873° | 4.06020 Å | 89.1% |
| 22.641° | 3.92412 Å | 18.5% |
| 23.431° | 3.79365 Å | 44.5% |
| 24.069° | 3.69452 Å | 10.7% |
| 24.508° | 3.62921 Å | 9.0% |
| 25.858° | 3.44283 Å | 18.5% |
| 26.151° | 3.40483 Å | 7.7% |
| 26.481° | 3.36324 Å | 5.5% |
| 26.920° | 3.30938 Å | 6.6% |
| 30.127° | 2.96395 Å | 10.1% |
| 31.295° | 2.85591 Å | 7.9% |
| 39.058° | 2.30434 Å | 7.1% |

In another aspect of the present disclosure, the present disclosure provides a hydrochloride of the compound represented by formula (I), whose structure is represented by formula (IV),

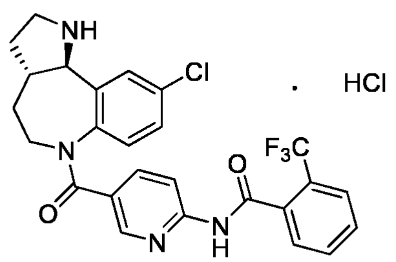

(IV) .

In another aspect of the present disclosure, the present disclosure provides a crystal form D of the hydrochloride of the compound represented by formula (I) (*i.e.,* the compound represented by formula (IV)), wherein the crystal form D has an X-ray powder diffraction pattern comprising characteristic diffraction peaks at the following 2θ angles: 8.13 ± 0.2°,

9.27 ± 0.2°, 9.91 ± 0.2°, 13.53 ± 0.2°, 16.37 ± 0.2°, and 17.09 ± 0.2°.

In some embodiments of the present disclosure, the X-ray powder diffraction pattern of the crystal form D comprises characteristic diffraction peaks at the following 2θ angles: 8.13 ± 0.2°, 9.27 ± 0.2°, 9.91 ± 0.2°, 12.86 ± 0.2°, 13.53 ± 0.2°, 16.37 ± 0.2°, 17.09 ± 0.2°, 18.67 ± 0.2°, 21.77 ± 0.2°, and 23.81 ± 0.2°.

In some embodiments of the present disclosure, the X-ray powder diffraction pattern of the crystal form D has an X-ray powder diffraction pattern substantially as shown in FIG. 12.

In some embodiments of the present disclosure, the analytical data of the X-ray powder diffraction pattern of the crystal form D are as shown in Table 4 below.

Table 4

| 2θ | Interplanar spacing | Relative intensity (%) |
|---|---|---|
| 8.129° | 10.86837 Å | 59.5% |
| 8.928° | 9.89720 Å | 7.4% |
| 9.271° | 9.53186 Å | 58.7% |
| 9.529° | 9.27384 Å | 37.5% |
| 9.908° | 8.92050 Å | 32.6% |
| 12.421° | 7.12059 Å | 8.2% |
| 12.673° | 6.97916 Å | 17.9% |
| 12.857° | 6.87999 Å | 22.3% |
| 13.534° | 6.53709 Å | 45.1% |
| 13.867° | 6.38091 Å | 5.5% |
| 14.206° | 6.22961 Å | 16.3% |
| 14.401° | 6.14545 Å | 9.9% |
| 15.748° | 5.62271 Å | 12.2% |
| 16.372° | 5.41000 Å | 54.6% |
| 16.513° | 5.36397 Å | 46.5% |
| 16.817° | 5.26772 Å | 42.9% |
| 17.090° | 5.18435 Å | 100.0% |
| 17.371° | 5.10099 Å | 51.4% |
| 17.966° | 4.93338 Å | 15.8% |
| 18.669° | 4.74915 Å | 61.9% |
| 19.211° | 4.61624 Å | 5.6% |
| 19.967° | 4.44324 Å | 32.4% |
| 20.234° | 4.38511 Å | 18.4% |
| 20.803° | 4.26644 Å | 10.9% |
| 21.340° | 4.16043 Å | 32.1% |
| 21.619° | 4.10733 Å | 57.6% |
| 21.774° | 4.07840 Å | 66.1% |
| 22.223° | 3.99700 Å | 12.4% |
| 22.493° | 3.94968 Å | 46.9% |
| 22.690° | 3.91588 Å | 43.4% |
| 23.420° | 3.79534 Å | 10.2% |
| 23.805° | 3.73482 Å | 80.5% |
| 24.274° | 3.66369 Å | 17.7% |

(continued)

| 2θ | Interplanar spacing | Relative intensity (%) |
|---|---|---|
| 24.648° | 3.60901 Å | 32.9% |
| 25.051° | 3.55189 Å | 16.1% |
| 25.820° | 3.44773 Å | 17.4% |
| 26.293° | 3.38686 Å | 27.6% |
| 26.671° | 3.33962 Å | 23.9% |
| 26.947° | 3.30607 Å | 12.4% |
| 27.616° | 3.22744 Å | 4.9% |
| 28.064° | 3.17694 Å | 9.6% |
| 28.684° | 3.10969 Å | 5.9% |
| 29.122° | 3.06389 Å | 13.9% |
| 29.447° | 3.03086 Å | 8.7% |
| 29.790° | 2.99669 Å | 16.2% |
| 30.187° | 2.95816 Å | 4.9% |
| 30.444° | 2.93383 Å | 8.0% |
| 31.152° | 2.86873 Å | 10.1% |
| 31.867° | 2.80598 Å | 7.8% |
| 32.490° | 2.75359 Å | 5.7% |
| 33.159° | 2.69951 Å | 8.1% |
| 33.434° | 2.67794 Å | 6.9% |
| 35.631° | 2.51773 Å | 7.1% |
| 36.500° | 2.45974 Å | 7.9% |
| 37.952° | 2.36893 Å | 9.7% |
| 41.801° | 2.15922 Å | 5.8% |
| 43.097° | 2.09724 Å | 5.5% |

In another aspect of the present disclosure, the present disclosure provides a crystal form E of the hydrochloride of the compound represented by formula (I) (*i.e.,* the compound represented by formula (IV)), wherein the crystal form E has an X-ray powder diffraction pattern comprising characteristic diffraction peaks at the following 2θ angles: 3.86 ± 0.2°, 13.60 ± 0.2°, 14.19 ± 0.2°, 18.06 ± 0.2°, 20.50 ± 0.2°, and 21.24 ± 0.2°.

In some embodiments of the present disclosure, the X-ray powder diffraction pattern of the crystal form E comprises characteristic diffraction peaks at the following 2θ angles: 3.86 ± 0.2°, 6.74 ± 0.2°, 11.73 ± 0.2°, 13.60 ± 0.2°, 14.19 ± 0.2°, 18.06 ± 0.2°, 20.50 ± 0.2°, 21.24 ± 0.2°, 23.72 ± 0.2°, and 24.06 ± 0.2°.

In some embodiments of the present disclosure, the X-ray powder diffraction pattern of the crystal form E has an X-ray powder diffraction pattern substantially as shown in FIG. 15.

In some embodiments of the present disclosure, the analytical data of the X-ray powder diffraction pattern of the crystal form E are as shown in Table 5 below.

Table 5

| 2θ | Interplanar spacing | Relative intensity (%) |
|---|---|---|
| 3.861° | 22.86835 Å | 65.3% |
| 6.739° | 13.10588 Å | 21.7% |
| 10.384° | 8.51254 Å | 7.6% |
| 11.734° | 7.53564 Å | 27.4% |

(continued)

| 2θ | Interplanar spacing | Relative intensity (%) |
|---|---|---|
| 13.063° | 6.77171 Å | 5.0% |
| 13.596° | 6.50767 Å | 40.9% |
| 14.188° | 6.23725 Å | 72.6% |
| 15.206° | 5.82217 Å | 13.6% |
| 17.168° | 5.16083 Å | 10.0% |
| 17.680° | 5.01250 Å | 21.6% |
| 18.056° | 4.90887 Å | 61.7% |
| 20.495° | 4.32996 Å | 100.0% |
| 21.237° | 4.18032 Å | 46.1% |
| 21.980° | 4.04061 Å | 16.7% |
| 22.701° | 3.91398 Å | 8.4% |
| 23.370° | 3.80344 Å | 20.9% |
| 23.721° | 3.74781 Å | 49.0% |
| 24.060° | 3.69577 Å | 63.7% |
| 24.680° | 3.60431 Å | 44.2% |
| 26.498° | 3.36111 Å | 36.9% |
| 27.127° | 3.28455 Å | 17.4% |
| 27.669° | 3.22147 Å | 15.6% |
| 28.534° | 3.12571 Å | 19.5% |
| 30.052° | 2.97119 Å | 10.7% |

In another aspect of the present disclosure, the present disclosure also provides a crystal form F of the hydrochloride of the compound represented by formula (I) (*i.e.,* the compound represented by formula (IV)), wherein the crystal form F has an X-ray powder diffraction pattern comprising characteristic diffraction peaks at the following 2θ angles: 5.84 ± 0.2°, 11.77 ± 0.2°, 13.29 ± 0.2°, 17.82 ± 0.2°, 20.49 ± 0.2°, and 20.94 ± 0.2°.

In some embodiments of the present disclosure, the X-ray powder diffraction pattern of the crystal form F comprises characteristic diffraction peaks at the following 2θ angles: 5.84 ± 0.2°, 11.77 ± 0.2°, 13.29 ± 0.2°, 14.34 ± 0.2°, 17.82 ± 0.2°, 18.67 ± 0.2°, 20.49 ± 0.2°, 20.94 ± 0.2°, 23.02 ± 0.2°, and 23.68 ± 0.2°.

In some embodiments of the present disclosure, the X-ray powder diffraction pattern of the crystal form F has an X-ray powder diffraction pattern substantially as shown in FIG. 18.

In some embodiments of the present disclosure, the analytical data of the X-ray powder diffraction pattern of the crystal form F are as shown in Table 6 below.

Table 6

| 2θ | Interplanar spacing | Relative intensity (%) |
|---|---|---|
| 5.844° | 15.10991 Å | 91.4% |
| 10.336° | 8.55177 Å | 7.8% |
| 11.768° | 7.51389 Å | 61.5% |
| 13.286° | 6.65876 Å | 16.9% |
| 14.435° | 6.13123 Å | 9.6% |
| 14.940° | 5.92506 Å | 3.1% |
| 16.093° | 5.50289 Å | 3.8% |
| 17.816° | 4.97466 Å | 22.2% |

(continued)

| 2θ | Interplanar spacing | Relative intensity (%) |
|---|---|---|
| 18.152° | 4.88335 Å | 10.1% |
| 18.667° | 4.74954 Å | 12.8% |
| 19.639° | 4.51668 Å | 3.1% |
| 20.497° | 4.32951 Å | 36.1% |
| 20.940° | 4.23897 Å | 100.0% |
| 22.510° | 3.94664 Å | 5.7% |
| 23.018° | 3.86066 Å | 16.7% |
| 23.683° | 3.75373 Å | 11.4% |
| 26.257° | 3.39135 Å | 10.2% |
| 27.842° | 3.20174 Å | 9.0% |
| 28.439° | 3.13595 Å | 5.0% |
| 28.615° | 3.11702 Å | 6.0% |
| 29.135° | 3.06259 Å | 7.0% |
| 29.855° | 2.99032 Å | 8.9% |
| 32.754° | 2.73198 Å | 8.8% |

In another aspect of the present disclosure, the present disclosure also provides a sulfate of the compound represented by formula (I), whose structure is represented by formula (V),

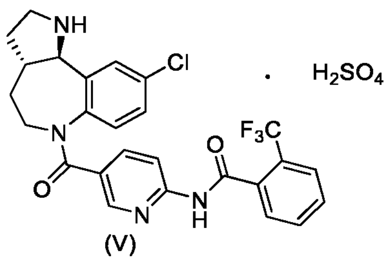

(V)

.

In another aspect of the present disclosure, the present disclosure also provides a crystal form G of the sulfate of the compound represented by formula (I) *(i.e.,* the compound represented by formula (V)), wherein the crystal form G has an X-ray powder diffraction pattern comprising characteristic diffraction peaks at the following 2θ angles: 10.30 ± 0.2°, 13.02 ± 0.2°, 16.60 ± 0.2°, 18.53 ± 0.2°, 20.67 ± 0.2°, and 22.26 ± 0.2°.

In some embodiments of the present disclosure, the X-ray powder diffraction pattern of the crystal form G comprises characteristic diffraction peaks at the following 2θ angles: 6.46 ± 0.2°, 10.30 ± 0.2°, 13.02 ± 0.2°, 16.60 ± 0.2°, 17.66 ± 0.2°, 18.53 ± 0.2°, 19.98 ± 0.2°, 20.67 ± 0.2°, 22.26 ± 0.2°, and 23.62 ± 0.2°.

In some embodiments of the present disclosure, the X-ray powder diffraction pattern of the crystal form G has an X-ray powder diffraction pattern substantially as shown in FIG. 21.

In some embodiments of the present disclosure, the analytical data of the X-ray powder diffraction pattern of the crystal form G of the sulfate are as shown in Table 7 below.

Table 7

| 2θ | Interplanar spacing | Relative intensity (%) |
|---|---|---|
| 6.459° | 13.67337 Å | 10.7% |
| 10.303° | 8.57922 Å | 14.8% |
| 12.169° | 7.26727 Å | 5.6% |
| 13.018° | 6.79500 Å | 29.8% |

(continued)

| 2θ | Interplanar spacing | Relative intensity (%) |
|---|---|---|
| 14.604° | 6.06056 Å | 7.2% |
| 15.192° | 5.82737 Å | 9.0% |
| 16.594° | 5.33793 Å | 34.7% |
| 17.200° | 5.15141 Å | 11.1% |
| 17.663° | 5.01733 Å | 14.2% |
| 18.096° | 4.89832 Å | 10.3% |
| 18.532° | 4.78391 Å | 28.9% |
| 19.102° | 4.64239 Å | 6.2% |
| 19.448° | 4.56066 Å | 12.3% |
| 19.975° | 4.44142 Å | 16.0% |
| 20.665° | 4.29473 Å | 67.8% |
| 21.864° | 4.06188 Å | 8.2% |
| 22.261° | 3.99021 Å | 100.0% |
| 22.538° | 3.94178 Å | 53.7% |
| 23.085° | 3.84970 Å | 9.5% |
| 23.616° | 3.76424 Å | 23.9% |
| 24.476° | 3.63395 Å | 12.8% |
| 24.970° | 3.56318 Å | 5.8% |
| 25.347° | 3.51097 Å | 7.0% |
| 25.783° | 3.45269 Å | 26.9% |
| 26.331° | 3.38205 Å | 17.5% |
| 27.028° | 3.29630 Å | 11.3% |
| 28.424° | 3.13751 Å | 20.9% |
| 29.462° | 3.02934 Å | 11.4% |
| 32.669° | 2.73891 Å | 6.0% |
| 34.073° | 2.62922 Å | 6.9% |

In another aspect of the present disclosure, the present disclosure also provides a succinate of the compound represented by formula (I), whose structure is represented by formula (VI),

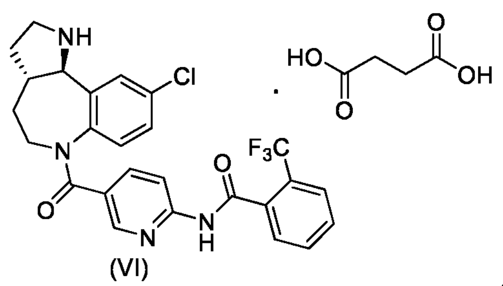

(VI)

.

In another aspect of the present disclosure, the present disclosure also provides a crystal form H of the succinate of the compound represented by formula (I) (*i.e.,* the compound represented by formula (VI)), wherein the crystal form H has an X-ray powder diffraction pattern comprising characteristic diffraction peaks at the following 2θ angles: 10.30 ± 0.2°, 14.63 ± 0.2°, 18.59 ± 0.2°, 20.13 ± 0.2°, 21.83 ± 0.2°, and 22.30 ± 0.2°.

In some embodiments of the present disclosure, the X-ray powder diffraction pattern of the crystal form H comprises characteristic diffraction peaks at the following 2θ angles: 10.30 ± 0.2°, 12.91 ± 0.2°, 14.63 ± 0.2°, 18.59 ± 0.2°, 19.41 ± 0.2°, 20.13 ± 0.2°, 20.69 ± 0.2°, 21.83 ± 0.2°, 22.30 ± 0.2°, and 23.65 ± 0.2°.

In some embodiments of the present disclosure, the X-ray powder diffraction pattern of the crystal form H has an X-ray powder diffraction pattern substantially as shown in FIG. 24.

In some embodiments of the present disclosure, the analytical data of the X-ray powder diffraction pattern of the crystal form H are as shown in Table 8 below.

Table 8

| 2θ | Interplanar spacing | Relative intensity (%) |
|---|---|---|
| 10.295° | 8.58547 Å | 46.9% |
| 12.909° | 6.85212 Å | 35.9% |
| 13.026° | 6.79111 Å | 24.9% |
| 14.633° | 6.04855 Å | 100.0% |
| 16.360° | 5.41391 Å | 10.5% |
| 16.584° | 5.34115 Å | 8.8% |
| 17.201° | 5.15094 Å | 21.6% |
| 17.979° | 4.92989 Å | 14.8% |
| 18.585° | 4.77032 Å | 53.3% |
| 19.134° | 4.63473 Å | 15.1% |
| 19.414° | 4.56857 Å | 34.8% |
| 20.128° | 4.40798 Å | 55.4% |
| 20.689° | 4.28977 Å | 42.9% |
| 21.326° | 4.16310 Å | 8.5% |
| 21.834° | 4.06742 Å | 83.6% |
| 22.301° | 3.98317 Å | 91.6% |
| 22.513° | 3.94620 Å | 8.4% |
| 23.037° | 3.85767 Å | 16.4% |
| 23.278° | 3.81826 Å | 6.5% |
| 23.647° | 3.75937 Å | 75.2% |
| 25.573° | 3.48047 Å | 10.7% |
| 25.740° | 3.45836 Å | 8.3% |
| 25.992° | 3.42538 Å | 20.9% |
| 26.386° | 3.37502 Å | 24.3% |
| 27.127° | 3.28460 Å | 4.7% |
| 27.544° | 3.23576 Å | 11.3% |
| 28.421° | 3.13783 Å | 5.2% |
| 29.565° | 3.01901 Å | 10.3% |
| 30.694° | 2.91051 Å | 21.9% |
| 31.254° | 2.85962 Å | 9.8% |
| 31.775° | 2.81389 Å | 6.1% |
| 35.613° | 2.51894 Å | 5.8% |
| 36.076° | 2.48767 Å | 6.8% |

(continued)

| 2θ | Interplanar spacing | Relative intensity (%) |
|---|---|---|
| 39.540° | 2.27733 Å | 5.3% |

In another aspect of the present disclosure, the present disclosure also provides a crystal form J of the succinate of the compound represented by formula (I) (*i.e.,* the compound represented by formula (VI)), wherein the crystal form J has an X-ray powder diffraction pattern comprising characteristic diffraction peaks at the following 2θ angles: 9.61 ± 0.2°, 11.56 ± 0.2°, 12.93 ± 0.2°, 17.12 ± 0.2°, 17.71 ± 0.2°, and 19.95 ± 0.2°.

In some embodiments of the present disclosure, the X-ray powder diffraction pattern of the crystal form J comprises characteristic diffraction peaks at the following 2θ angles: 9.61 ± 0.2°, 11.56 ± 0.2°, 12.93 ± 0.2°, 13.76 ± 0.2°, 17.12 ± 0.2°, 17.71 ± 0.2°, 19.51 ± 0.2°, 19.95 ± 0.2°, 21.83 ± 0.2°, and 22.42 ± 0.2°.

In some embodiments of the present disclosure, the X-ray powder diffraction pattern of the crystal form J has an X-ray powder diffraction pattern substantially as shown in FIG. 27.

In some embodiments of the present disclosure, the analytical data of the X-ray powder diffraction pattern of the crystal form J are as shown in Table 9 below.

Table 9

| 2θ | Interplanar spacing | Relative intensity (%) |
|---|---|---|
| 9.611° | 9.19525 Å | 23.4% |
| 9.939° | 8.89270 Å | 10.7% |
| 11.556° | 7.65153 Å | 60.5% |
| 12.932° | 6.84040 Å | 100.0% |
| 13.756° | 6.43214 Å | 33.3% |
| 14.006° | 6.31793 Å | 14.6% |
| 14.957° | 5.91855 Å | 12.3% |
| 15.592° | 5.67891 Å | 4.4% |
| 16.336° | 5.42185 Å | 4.5% |
| 16.560° | 5.34886 Å | 5.2% |
| 17.121° | 5.17500 Å | 86.6% |
| 17.707° | 5.00481 Å | 70.1% |
| 19.391° | 4.57384 Å | 38.1% |
| 19.512° | 4.54577 Å | 42.6% |
| 19.947° | 4.44768 Å | 87.5% |
| 20.251° | 4.38151 Å | 13.4% |
| 20.838° | 4.25935 Å | 7.5% |
| 21.233° | 4.18106 Å | 4.1% |
| 21.830° | 4.06807 Å | 15.3% |
| 22.423° | 3.96180 Å | 47.1% |
| 22.866° | 3.88605 Å | 22.7% |
| 23.392° | 3.79991 Å | 8.5% |
| 23.714° | 3.74896 Å | 10.6% |
| 23.917° | 3.71754 Å | 12.7% |
| 24.780° | 3.59009 Å | 6.6% |
| 25.126° | 3.54136 Å | 7.0% |
| 25.498° | 3.49052 Å | 17.6% |

(continued)

| 2θ | Interplanar spacing | Relative intensity (%) |
|---|---|---|
| 26.128° | 3.40787 Å | 16.8% |
| 26.812° | 3.32241 Å | 14.7% |
| 26.940° | 3.30693 Å | 11.0% |
| 27.415° | 3.25068 Å | 11.8% |
| 27.642° | 3.22451 Å | 22.1% |
| 27.820° | 3.20429 Å | 14.6% |
| 29.293° | 3.04644 Å | 18.5% |

In another aspect of the present disclosure, the present disclosure also provides a glycollate of the compound represented by formula (I), whose structure is represented by formula (VII),

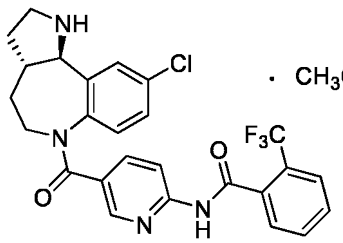

$\cdot\ CH_3CH_2OH$

(VII)

.

In another aspect of the present disclosure, the present disclosure also provides a crystal form K of the glycollate of the compound represented by formula (I) (*i.e.*, the compound represented by formula (VII)), wherein the crystal form K has an X-ray powder diffraction pattern comprising characteristic diffraction peaks at the following 2θ angles: 12.51 ± 0.2°, 15.99 ± 0.2°, 18.71 ± 0.2°, 20.18 ± 0.2°, 20.59 ± 0.2°, and 21.64 ± 0.2°.

In some embodiments of the present disclosure, the X-ray powder diffraction pattern of the crystal form K comprises characteristic diffraction peaks at the following 2θ angles: 12.51 ± 0.2°, 13.62 ± 0.2°, 15.99 ± 0.2°, 16.65 ± 0.2°, 18.71 ± 0.2°, 20.18 ± 0.2°, 20.59 ± 0.2°, 21.64 ± 0.2°, 22.62 ± 0.2°, and 24.53 ± 0.2°.

In some embodiments of the present disclosure, the X-ray powder diffraction pattern of the crystal form K has an X-ray powder diffraction pattern substantially as shown in FIG. 30.

In some embodiments of the present disclosure, the analytical data of the X-ray powder diffraction pattern of the crystal form K are as shown in Table 10 below.

Table 10

| 2θ | Interplanar spacing | Relative intensity (%) |
|---|---|---|
| 4.528° | 19.49824 Å | 7.9% |
| 9.126° | 9.68211 Å | 9.8% |
| 9.452° | 9.34926 Å | 16.0% |
| 12.514° | 7.06792 Å | 65.7% |
| 13.619° | 6.49668 Å | 27.9% |
| 14.273° | 6.20029 Å | 5.8% |
| 15.040° | 5.88582 Å | 10.2% |
| 15.242° | 5.80852 Å | 6.2% |
| 15.985° | 5.53991 Å | 50.4% |
| 16.649° | 5.32057 Å | 20.5% |
| 18.389° | 4.82092 Å | 9.8% |
| 18.713° | 4.73813 Å | 55.3% |

(continued)

| 2θ | Interplanar spacing | Relative intensity (%) |
|---|---|---|
| 19.027° | 4.66053 Å | 15.3% |
| 20.177° | 4.39755 Å | 100.0% |
| 20.498° | 4.32925 Å | 79.5% |
| 20.911° | 4.24475 Å | 9.6% |
| 21.642° | 4.10295 Å | 75.9% |
| 22.090° | 4.02082 Å | 8.0% |
| 22.619° | 3.92793 Å | 29.1% |
| 23.037° | 3.85763 Å | 29.1% |
| 23.723° | 3.74763 Å | 11.8% |
| 24.209° | 3.67336 Å | 6.0% |
| 24.533° | 3.62569 Å | 40.6% |
| 24.885° | 3.57521 Å | 12.2% |
| 25.170° | 3.53534 Å | 9.7% |
| 25.906° | 3.43657 Å | 12.3% |
| 26.781° | 3.32614 Å | 7.0% |
| 27.560° | 3.23391 Å | 8.3% |
| 27.753° | 3.21184 Å | 12.3% |
| 28.114° | 3.17146 Å | 7.9% |
| 28.579° | 3.12087 Å | 13.5% |
| 28.782° | 3.09933 Å | 14.3% |
| 29.013° | 3.07517 Å | 11.9% |
| 29.531° | 3.02236 Å | 9.6% |
| 31.325° | 2.85329 Å | 5.1% |
| 31.728° | 2.81799 Å | 5.8% |
| 32.439° | 2.75780 Å | 12.5% |
| 33.248° | 2.69249 Å | 4.7% |
| 33.613° | 2.66412 Å | 8.3% |
| 33.947° | 2.63867 Å | 6.1% |
| 34.998° | 2.56180 Å | 7.6% |
| 36.704° | 2.44650 Å | 5.2% |
| 37.799° | 2.37814 Å | 5.1% |
| 38.732° | 2.32300 Å | 6.0% |

In another aspect of the present disclosure, the present disclosure also provides a benzoate of the compound represented by formula (I), whose structure is represented by formula (VIII),

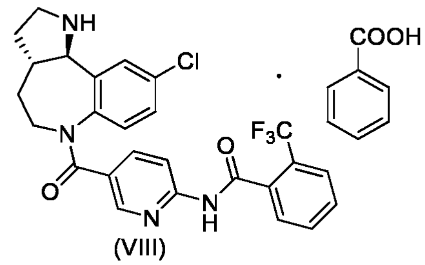

(VIII)

.

In another aspect of the present disclosure, the present disclosure also provides a eutectic crystal form L of the benzoate of the compound represented by formula (I), wherein the crystal form L has an X-ray powder diffraction pattern comprising characteristic diffraction peaks at the following 2θ angles: 8.14 ± 0.2°, 8.76 ± 0.2°, 9.55 ± 0.2°, 12.62 ± 0.2°, 16.43 ± 0.2°, and 18.05 ± 0.2°.

In some embodiments of the present disclosure, the X-ray powder diffraction pattern of the crystal form L comprises characteristic diffraction peaks at the following 2θ angles: 8.14 ± 0.2°, 8.76 ± 0.2°, 9.55 ± 0.2°, 12.62 ± 0.2°, 16.43 ± 0.2°, 17.68 ± 0.2°, 18.05 ± 0.2°, 18.95 ± 0.2°, 19.32 ± 0.2°, and 19.73 ± 0.2°.

In some embodiments of the present disclosure, the X-ray powder diffraction pattern of the crystal form L has an X-ray powder diffraction pattern substantially as shown in FIG. 33.

In some embodiments of the present disclosure, the analytical data of the X-ray powder diffraction pattern of the crystal form L are as shown in Table 11 below.

Table 11

| 2θ | Interplanar spacing | Relative intensity (%) |
|---|---|---|
| 8.143° | 10.84882 Å | 85.7% |
| 8.366° | 10.56043 Å | 35.0% |
| 8.764° | 10.08196 Å | 57.3% |
| 9.552° | 9.25168 Å | 100.0% |
| 12.621° | 7.00811 Å | 29.7% |
| 12.981° | 6.81437 Å | 10.9% |
| 16.431° | 5.39073 Å | 73.9% |
| 17.682° | 5.01183 Å | 21.8% |
| 18.050° | 4.91065 Å | 29.6% |
| 18.956° | 4.67789 Å | 29.8% |
| 19.328° | 4.58870 Å | 21.2% |
| 19.708° | 4.50104 Å | 18.5% |
| 21.069° | 4.21318 Å | 6.4% |
| 22.036° | 4.03058 Å | 19.8% |
| 22.391° | 3.96740 Å | 12.1% |
| 24.321° | 3.65676 Å | 33.9% |
| 24.920° | 3.57019 Å | 10.3% |
| 25.443° | 3.49798 Å | 7.8% |
| 26.741° | 3.33106 Å | 6.8% |
| 27.183° | 3.27789 Å | 14.0% |

In another aspect of the present disclosure, the present disclosure also provides a eutectic crystal form M of the benzoate of the compound represented by formula (I), wherein the crystal form M has an X-ray powder diffraction pattern comprising characteristic diffraction peaks at the following 2θ angles: 9.31 ± 0.2°, 13.77 ± 0.2°, 14.54 ± 0.2°, 19.84 ± 0.2°, 20.34 ± 0.2°, and 21.70 ± 0.2°.

In some embodiments of the present disclosure, the X-ray powder diffraction pattern of the crystal form M comprises characteristic diffraction peaks at the following 2θ angles: 9.31 ± 0.2°, 13.77 ± 0.2°, 14.54 ± 0.2°, 16.55 ± 0.2°, 17.66 ± 0.2°, 18.68 ± 0.2°, 19.84 ± 0.2°, 20.34 ± 0.2°, 21.70 ± 0.2°, and 23.32 ± 0.2°.
In some embodiments of the present disclosure, the X-ray powder diffraction pattern of the crystal form M has an X-ray powder diffraction pattern substantially as shown in FIG. 36.
In some embodiments of the present disclosure, the analytical data of the X-ray powder diffraction pattern of the crystal form M are as shown in Table 12 below.

Table 12

| 2θ | Interplanar spacing | Relative intensity (%) |
|---|---|---|
| 9.307° | 9.49506 Å | 44.3% |
| 10.894° | 8.11512 Å | 24.2% |
| 11.925° | 7.41539 Å | 9.4% |
| 12.205° | 7.24583 Å | 9.5% |
| 13.771° | 6.42529 Å | 47.4% |
| 14.535° | 6.08907 Å | 40.5% |
| 15.675° | 5.64886 Å | 17.5% |
| 16.549° | 5.35234 Å | 45.1% |
| 17.663° | 5.01741 Å | 48.3% |
| 18.678° | 4.74693 Å | 43.9% |
| 19.839° | 4.47156 Å | 52.1% |
| 20.344° | 4.36177 Å | 100.0% |
| 21.365° | 4.15546 Å | 44.2% |
| 21.697° | 4.09278 Å | 83.1% |
| 22.968° | 3.86898 Å | 42.4% |
| 23.319° | 3.81159 Å | 54.6% |
| 24.411° | 3.64352 Å | 34.3% |
| 24.803° | 3.58682 Å | 48.1% |
| 26.613° | 3.34685 Å | 22.6% |
| 27.392° | 3.25337 Å | 11.3% |
| 28.174° | 3.16481 Å | 16.7% |
| 29.855° | 2.99029 Å | 21.9% |
| 30.375° | 2.94029 Å | 18.3% |

In another aspect of the present disclosure, the present disclosure also provides a crystal form N of the compound represented by formula (I), wherein the crystal form N has an X-ray powder diffraction pattern comprising characteristic diffraction peaks at the following 2θ angles: 10.38 ± 0.2°, 13.54 ± 0.2°, 14.41 ± 0.2°, 16.32 ± 0.2°, 18.10 ± 0.2°, and 19.05 ± 0.2°.
In some embodiments of the present disclosure, the X-ray powder diffraction pattern of the crystal form N comprises characteristic diffraction peaks at the following 2θ angles: 10.38 ± 0.2°, 13.54 ± 0.2°, 14.41 ± 0.2°, 15.90 ± 0.2°, 16.32 ± 0.2°, 18.10 ± 0.2°, 19.05 ± 0.2°, 22.14 ± 0.2°, 22.91 ± 0.2°, and 23.66 ± 0.2°.
In some embodiments of the present disclosure, the X-ray powder diffraction pattern of the crystal form N has an X-ray powder diffraction pattern substantially as shown in FIG. 39.
In some embodiments of the present disclosure, the analytical data of the X-ray powder diffraction pattern of the crystal form N are as shown in Table 13 below.

Table 13

| 2θ | Interplanar spacing | Relative intensity (%) |
|---|---|---|
| 9.604° | 9.20210 Å | 7.7% |
| 10.378° | 8.51740 Å | 23.0% |
| 12.702° | 6.96338 Å | 4.2% |
| 12.954° | 6.82861 Å | 5.4% |
| 13.537° | 6.53564 Å | 28.4% |
| 14.109° | 6.27192 Å | 15.6% |
| 14.405° | 6.14410 Å | 24.9% |
| 14.643° | 6.04457 Å | 14.2% |
| 14.809° | 5.97737 Å | 6.1% |
| 15.180° | 5.83182 Å | 3.6% |
| 15.961° | 5.54825 Å | 36.5% |
| 16.317° | 5.42801 Å | 100.0% |
| 16.743° | 5.29085 Å | 3.7% |
| 17.094° | 5.18285 Å | 41.6% |
| 17.623° | 5.02856 Å | 29.2% |
| 18.104° | 4.89593 Å | 39.5% |
| 19.047° | 4.65567 Å | 67.5% |
| 19.343° | 4.58504 Å | 30.3% |
| 19.830° | 4.47364 Å | 7.7% |
| 20.409° | 4.34798 Å | 15.1% |
| 20.585° | 4.31118 Å | 25.1% |
| 20.883° | 4.25040 Å | 7.0% |
| 21.218° | 4.18399 Å | 5.9% |
| 21.718° | 4.08875 Å | 9.2% |
| 22.143° | 4.01132 Å | 31.6% |
| 22.907° | 3.87918 Å | 41.7% |
| 23.380° | 3.80181 Å | 30.3% |
| 23.663° | 3.75691 Å | 39.4% |
| 23.822° | 3.73226 Å | 12.4% |
| 24.841° | 3.58137 Å | 13.0% |
| 25.133° | 3.54046 Å | 7.3% |
| 25.617° | 3.47467 Å | 7.6% |
| 25.855° | 3.44316 Å | 27.1% |
| 26.439° | 3.36844 Å | 8.4% |
| 26.827° | 3.32060 Å | 5.4% |
| 27.110° | 3.28660 Å | 6.7% |
| 27.317° | 3.26213 Å | 10.2% |
| 27.899° | 3.19539 Å | 3.7% |
| 28.499° | 3.12942 Å | 3.9% |

(continued)

| 2θ | Interplanar spacing | Relative intensity (%) |
|---|---|---|
| 28.969° | 3.07972 Å | 6.9% |
| 29.366° | 3.03905 Å | 8.3% |
| 29.569° | 3.01858 Å | 5.3% |
| 29.900° | 2.98597 Å | 7.4% |
| 30.181° | 2.95880 Å | 11.6% |
| 32.185° | 2.77901 Å | 7.5% |

In another aspect of the present disclosure, the present disclosure also provides a crystal form O of the compound represented by formula (I), wherein the crystal form O has an X-ray powder diffraction pattern comprising characteristic diffraction peaks at the following 2θ angles: 4.75 ± 0.2°, 9.65 ± 0.2°, 15.70 ± 0.2°, 16.88 ± 0.2°, 18.00 ± 0.2°, and 18.97 ± 0.2°.

In some embodiments of the present disclosure, the X-ray powder diffraction pattern of the crystal form O comprises characteristic diffraction peaks at the following 2θ angles: 4.75 ± 0.2°, 9.65 ± 0.2°, 15.70 ± 0.2°, 16.88 ± 0.2°, 18.00 ± 0.2°, 18.97 ± 0.2°, 19.89 ± 0.2°, 21.86 ± 0.2°, 22.67 ± 0.2°, and 24.30 ± 0.2°.

In some embodiments of the present disclosure, the X-ray powder diffraction pattern of the crystal form O has an X-ray powder diffraction pattern substantially as shown in FIG. 42.

In some embodiments of the present disclosure, the analytical data of the X-ray powder diffraction pattern of the crystal form O are as shown in Table 14 below.

Table 14

| 2θ | Interplanar spacing | Relative intensity (%) |
|---|---|---|
| 4.751° | 18.58335 Å | 11.0% |
| 9.649° | 9.15933 Å | 20.8% |
| 11.219° | 7.88031 Å | 3.8% |
| 12.940° | 6.83581 Å | 2.6% |
| 13.494° | 6.55644 Å | 3.3% |
| 14.853° | 5.95955 Å | 3.7% |
| 15.697° | 5.64113 Å | 100.0% |
| 16.267° | 5.44455 Å | 7.3% |
| 16.881° | 5.24783 Å | 44.3% |
| 17.421° | 5.08644 Å | 6.4% |
| 18.004° | 4.92290 Å | 13.7% |
| 18.972° | 4.67398 Å | 19.2% |
| 19.885° | 4.46137 Å | 14.8% |
| 21.859° | 4.06272 Å | 10.3% |
| 22.668° | 3.91952 Å | 32.4% |
| 23.506° | 3.78164 Å | 6.7% |
| 24.295° | 3.66062 Å | 11.6% |
| 25.065° | 3.54985 Å | 9.7% |
| 25.843° | 3.44471 Å | 6.7% |
| 28.225° | 3.15917 Å | 7.8% |
| 29.632° | 3.01229 Å | 5.5% |
| 29.921° | 2.98393 Å | 6.2% |

In another aspect of the present disclosure, the present disclosure also provides a crystal form P of the compound represented by formula (I), wherein the crystal form P has an X-ray powder diffraction pattern comprising characteristic diffraction peaks at the following 2θ angles: 13.07 ± 0.2°, 17.98 ± 0.2°, 21.64 ± 0.2°, 23.78 ± 0.2°, 26.36 ± 0.2°, and 33.13 ± 0.2°.

In some embodiments of the present disclosure, the X-ray powder diffraction pattern of the crystal form P has an X-ray powder diffraction pattern substantially as shown in FIG. 45.

In some embodiments of the present disclosure, the analytical data of the X-ray powder diffraction pattern of the crystal form P are as shown in Table 15 below.

Table 15

| 2θ | Interplanar spacing | Relative intensity (%) |
|---|---|---|
| 13.067° | 6.76991 Å | 100.0% |
| 14.976° | 5.91077 Å | 1.3% |
| 15.443° | 5.73307 Å | 1.2% |
| 16.342° | 5.41972 Å | 0.9% |
| 16.764° | 5.28435 Å | 0.5% |
| 17.977° | 4.93039 Å | 9.2% |
| 18.178° | 4.87619 Å | 8.2% |
| 18.897° | 4.69245 Å | 2.4% |
| 19.363° | 4.58043 Å | 1.6% |
| 19.833° | 4.47291 Å | 0.7% |
| 21.207° | 4.18609 Å | 2.8% |
| 21.322° | 4.16391 Å | 1.9% |
| 21.643° | 4.10283 Å | 11.3% |
| 22.839° | 3.89050 Å | 2.8% |
| 23.526° | 3.77846 Å | 1.5% |
| 23.782° | 3.73848 Å | 17.6% |
| 24.402° | 3.64477 Å | 1.1% |
| 24.696° | 3.60213 Å | 1.3% |
| 24.958° | 3.56492 Å | 0.4% |
| 26.361° | 3.37827 Å | 92.9% |
| 26.525° | 3.35774 Å | 2.5% |
| 26.900° | 3.31179 Å | 1.2% |
| 27.603° | 3.22896 Å | 1.0% |
| 28.119° | 3.17090 Å | 0.8% |
| 29.258° | 3.04994 Å | 1.1% |
| 29.878° | 2.98808 Å | 0.7% |
| 30.318° | 2.94569 Å | 0.8% |
| 30.570° | 2.92202 Å | 0.7% |
| 30.900° | 2.89155 Å | 1.1% |
| 33.133° | 2.70162 Å | 24.7% |
| 36.299° | 2.47292 Å | 2.5% |

In another aspect of the present disclosure, the present disclosure also provides a crystal form Q of the

compound represented by formula (I), wherein the crystal form Q has an X-ray powder diffraction pattern comprising characteristic diffraction peaks at the following 2θ angles: 3.48 ± 0.2°, 10.60 ± 0.2°, 12.32 ± 0.2°, 15.41 ± 0.2°, 16.60 ± 0.2°, and 17.09 ± 0.2°.

In some embodiments of the present disclosure, the X-ray powder diffraction pattern of the crystal form Q comprises characteristic diffraction peaks at the following 2θ angles: 3.48 ± 0.2°, 10.60 ± 0.2°, 12.32 ± 0.2°, 15.41 ± 0.2°, 16.60 ± 0.2°, 17.09 ± 0.2°, 17.75 ± 0.2°, 18.79 ± 0.2°, 20.49 ± 0.2°, and 21.40 ± 0.2°.

In some embodiments of the present disclosure, the X-ray powder diffraction pattern of the crystal form Q has an X-ray powder diffraction pattern substantially as shown in FIG. 48.

In some embodiments of the present disclosure, the analytical data of the X-ray powder diffraction pattern of the crystal form Q are as shown in Table 16 below.

Table 16

| 2θ | Interplanar spacing | Relative intensity (%) |
|---|---|---|
| 3.478° | 25.38189 Å | 20.3% |
| 6.931° | 12.74332 Å | 4.0% |
| 7.997° | 11.04672 Å | 5.3% |
| 9.241° | 9.56225 Å | 3.4% |
| 10.601° | 8.33849 Å | 59.5% |
| 11.109° | 7.95845 Å | 15.1% |
| 12.324° | 7.17642 Å | 26.6% |
| 13.669° | 6.47283 Å | 9.9% |
| 13.961° | 6.33832 Å | 9.3% |
| 14.175° | 6.24295 Å | 10.5% |
| 14.642° | 6.04504 Å | 10.2% |
| 14.938° | 5.92574 Å | 15.7% |
| 15.119° | 5.85515 Å | 12.9% |
| 15.412° | 5.74472 Å | 100.0% |
| 16.604° | 5.33471 Å | 51.4% |
| 17.088° | 5.18469 Å | 84.8% |
| 17.754° | 4.99191 Å | 25.8% |
| 17.926° | 4.94416 Å | 13.7% |
| 18.176° | 4.87679 Å | 11.7% |
| 18.789° | 4.71902 Å | 77.6% |
| 19.663° | 4.51129 Å | 33.3% |
| 20.173° | 4.39825 Å | 13.4% |
| 20.491° | 4.33073 Å | 50.3% |
| 21.081° | 4.21092 Å | 21.8% |
| 21.401° | 4.14864 Å | 38.9% |
| 21.977° | 4.04128 Å | 4.4% |
| 22.226° | 3.99654 Å | 14.9% |
| 22.395° | 3.96663 Å | 17.6% |
| 22.738° | 3.90767 Å | 6.5% |
| 23.093° | 3.84831 Å | 8.1% |
| 23.961° | 3.71084 Å | 37.2% |

(continued)

| 2θ | Interplanar spacing | Relative intensity (%) |
|---|---|---|
| 24.477° | 3.63386 Å | 5.0% |
| 24.775° | 3.59084 Å | 9.5% |
| 24.998° | 3.55926 Å | 24.8% |
| 25.375° | 3.50727 Å | 10.7% |
| 25.744° | 3.45780 Å | 12.0% |
| 26.218° | 3.39636 Å | 21.4% |
| 26.477° | 3.36368 Å | 22.4% |
| 26.983° | 3.30170 Å | 6.3% |
| 27.595° | 3.22984 Å | 10.7% |
| 28.184° | 3.16376 Å | 9.3% |
| 29.045° | 3.07189 Å | 4.2% |
| 29.330° | 3.04264 Å | 8.5% |
| 29.551° | 3.02043 Å | 7.0% |
| 30.256° | 2.95163 Å | 5.3% |
| 31.876° | 2.80521 Å | 5.5% |
| 33.541° | 2.66962 Å | 3.2% |
| 34.690° | 2.58384 Å | 3.0% |
| 36.418° | 2.46507 Å | 2.4% |
| 37.924° | 2.37058 Å | 3.1% |

In another aspect of the present disclosure, the present disclosure also provides a crystal form R of the compound represented by formula (I), wherein the crystal form R has an X-ray powder diffraction pattern comprising characteristic diffraction peaks at the following 2θ angles: 6.70 ± 0.2°, 13.30 ± 0.2°, 18.15 ± 0.2°, 21.39 ± 0.2°, 22.97 ± 0.2°, and 26.71 ± 0.2°.

In some embodiments of the present disclosure, the X-ray powder diffraction pattern of the crystal form R has an X-ray powder diffraction pattern substantially as shown in FIG. 50.

In some embodiments of the present disclosure, the analytical data of the X-ray powder diffraction pattern of the crystal form R are as shown in Table 17 below.

Table 17

| 2θ | Interplanar spacing | Relative intensity (%) |
|---|---|---|
| 6.695 | 13.21358 | 7.7 |
| 10.064 | 8.81606 | 1.8 |
| 10.846 | 8.18694 | 2.2 |
| 13.302 | 6.69569 | 100.0 |
| 15.150 | 5.89501 | 4.0 |
| 17.453 | 5.13657 | 1.9 |
| 18.149 | 4.94600 | 7.9 |
| 19.061 | 4.71734 | 2.8 |
| 19.540 | 4.60625 | 2.1 |
| 19.974 | 4.50998 | 12.3 |
| 21.387 | 4.22471 | 20.8 |

(continued)

| 2θ | Interplanar spacing | Relative intensity (%) |
|---|---|---|
| 21.822 | 4.14455 | 2.8 |
| 22.973 | 3.94718 | 11.1 |
| 23.756 | 3.82435 | 2.9 |
| 23.973 | 3.79170 | 4.6 |
| 24.190 | 3.75966 | 5.0 |
| 24.560 | 3.70653 | 8.1 |
| 25.212 | 3.61671 | 1.8 |
| 25.951 | 3.52057 | 3.3 |
| 26.711 | 3.42738 | 29.6 |
| 27.081 | 3.38408 | 6.1 |
| 27.798 | 3.30347 | 10.2 |
| 29.428 | 3.13557 | 2.2 |
| 30.037 | 3.07779 | 3.9 |
| 30.493 | 3.03606 | 2.2 |
| 31.080 | 2.98432 | 11.9 |
| 33.144 | 2.81773 | 4.3 |
| 33.536 | 2.78864 | 5.0 |
| 34.427 | 2.72503 | 2.0 |
| 35.492 | 2.65354 | 2.1 |
| 36.274 | 2.60392 | 2.0 |
| 36.513 | 2.58922 | 1.4 |
| 37.795 | 2.51386 | 1.9 |
| 38.013 | 2.50165 | 1.6 |
| 38.730 | 2.46241 | 2.0 |
| 39.099 | 2.44282 | 1.4 |
| 40.447 | 2.37476 | 4.2 |
| 41.229 | 2.33753 | 5.4 |
| 42.859 | 2.26493 | 1.5 |

In another aspect of the present disclosure, the present disclosure also provides a crystal form S of the compound represented by formula (I), wherein the crystal form S has an X-ray powder diffraction pattern comprising characteristic diffraction peaks at the following 2θ angles: 14.97 ± 0.2°, 15.34 ± 0.2°, 17.97 ± 0.2°, 22.81 ± 0.2°, 23.54 ± 0.2°, and 24.69 ± 0.2°.

In some embodiments of the present disclosure, the X-ray powder diffraction pattern of the crystal form S has an X-ray powder diffraction pattern substantially as shown in FIG. 53.

In some embodiments of the present disclosure, the analytical data of the X-ray powder diffraction pattern of the crystal form S are as shown in Table 18 below.

Table 18

| 2θ | Interplanar spacing | Relative intensity (%) |
|---|---|---|
| 8.140° | 10.85304 Å | 1.3% |
| 9.936° | 8.89484 Å | 4.5% |

(continued)

| 2θ | Interplanar spacing | Relative intensity (%) |
|---|---|---|
| 13.149° | 6.72773 Å | 6.1% |
| 13.428° | 6.58845 Å | 1.5% |
| 14.973° | 5.91214 Å | 100.0% |
| 15.340° | 5.77164 Å | 16.3% |
| 16.371° | 5.41028 Å | 4.2% |
| 16.689° | 5.30772 Å | 0.7% |
| 17.248° | 5.13700 Å | 0.6% |
| 17.972° | 4.93177 Å | 84.8% |
| 18.727° | 4.73452 Å | 2.8% |
| 18.869° | 4.69924 Å | 3.7% |
| 19.008° | 4.66510 Å | 6.0% |
| 19.852° | 4.46879 Å | 3.2% |
| 19.995° | 4.43700 Å | 6.4% |
| 21.209° | 4.18582 Å | 9.5% |
| 21.332° | 4.16187 Å | 6.5% |
| 22.813° | 3.89488 Å | 19.9% |
| 22.968° | 3.86902 Å | 4.4% |
| 23.538° | 3.77667 Å | 51.4% |
| 24.025° | 3.70120 Å | 2.1% |
| 24.374° | 3.64895 Å | 1.7% |
| 24.690° | 3.60291 Å | 19.1% |
| 25.071° | 3.54903 Å | 2.3% |
| 25.477° | 3.49337 Å | 1.8% |
| 26.538° | 3.35606 Å | 2.6% |
| 26.885° | 3.31351 Å | 2.5% |
| 27.410° | 3.25129 Å | 2.5% |
| 27.615° | 3.22758 Å | 2.0% |
| 27.724° | 3.21511 Å | 1.3% |
| 28.091° | 3.17401 Å | 2.5% |
| 29.879° | 2.98795 Å | 1.2% |
| 30.261° | 2.95117 Å | 37.0% |

In another aspect of the present disclosure, the present disclosure also provides a crystal form T of the compound represented by formula (I), wherein the crystal form T has an X-ray powder diffraction pattern comprising characteristic diffraction peaks at the following 2θ angles: 15.84 ± 0.2°, 17.03 ± 0.2°, 17.60 ± 0.2°, 20.01 ± 0.2°, 22.22 ± 0.2°, and 22.82 ± 0.2°.

In some embodiments of the present disclosure, the X-ray powder diffraction pattern of the crystal form T comprises characteristic diffraction peaks at the following 2θ angles: 13.64 ± 0.2°, 14.70 ± 0.2°, 15.84 ± 0.2°, 17.03 ± 0.2°, 17.60 ± 0.2°, 19.01 ± 0.2°, 20.01 ± 0.2°, 22.22 ± 0.2°, 22.82 ± 0.2°, and 24.45 ± 0.2°.

In some embodiments of the present disclosure, the X-ray powder diffraction pattern of the crystal form T has an X-ray powder diffraction pattern substantially as shown in FIG. 56.

In some embodiments of the present disclosure, the analytical data of the X-ray powder diffraction pattern of the

crystal form T are as shown in Table 19 below.

Table 19

| 2θ | Interplanar spacing | Relative intensity (%) |
|---|---|---|
| 7.819° | 11.29786 Å | 7.0% |
| 9.612° | 9.19442 Å | 4.9% |
| 9.984° | 8.85246 Å | 12.6% |
| 11.362° | 7.78131 Å | 19.3% |
| 12.406° | 7.12908 Å | 6.2% |
| 12.720° | 6.95381 Å | 6.8% |
| 13.070° | 6.76822 Å | 12.3% |
| 13.636° | 6.48846 Å | 31.1% |
| 14.172° | 6.24442 Å | 11.2% |
| 14.482° | 6.11127 Å | 12.7% |
| 14.699° | 6.02151 Å | 26.9% |
| 14.991° | 5.90498 Å | 10.1% |
| 15.839° | 5.59078 Å | 80.6% |
| 17.029° | 5.20264 Å | 68.5% |
| 17.426° | 5.08496 Å | 36.6% |
| 17.574° | 5.04241 Å | 78.0% |
| 18.161° | 4.88081 Å | 19.2% |
| 18.484° | 4.79635 Å | 26.2% |
| 18.876° | 4.69756 Å | 12.7% |
| 19.015° | 4.66352 Å | 44.1% |
| 19.169° | 4.62626 Å | 34.7% |
| 20.011° | 4.43360 Å | 80.3% |
| 20.283° | 4.37469 Å | 41.7% |
| 20.469° | 4.33542 Å | 18.4% |
| 21.132° | 4.20091 Å | 14.6% |
| 21.289° | 4.17025 Å | 4.5% |
| 21.596° | 4.11167 Å | 9.3% |
| 22.026° | 4.03228 Å | 59.9% |
| 22.221° | 3.99732 Å | 100.0% |
| 22.816° | 3.89447 Å | 90.3% |
| 23.194° | 3.83178 Å | 18.1% |
| 23.308° | 3.81337 Å | 17.6% |
| 23.523° | 3.77894 Å | 21.8% |
| 23.642° | 3.76022 Å | 10.5% |
| 23.854° | 3.72723 Å | 16.5% |
| 23.990° | 3.70650 Å | 9.4% |
| 24.446° | 3.63839 Å | 36.2% |
| 25.193° | 3.53210 Å | 28.3% |

(continued)

| 2θ | Interplanar spacing | Relative intensity (%) |
|---|---|---|
| 25.501° | 3.49016 Å | 17.8% |
| 25.763° | 3.45522 Å | 19.7% |
| 25.990° | 3.42555 Å | 16.1% |
| 26.303° | 3.38555 Å | 32.9% |
| 26.673° | 3.33945 Å | 17.7% |
| 26.812° | 3.32246 Å | 30.4% |
| 27.859° | 3.19987 Å | 24.9% |
| 28.014° | 3.18257 Å | 26.2% |
| 28.334° | 3.14730 Å | 18.7% |
| 29.628° | 3.01273 Å | 11.7% |
| 29.757° | 2.99999 Å | 12.9% |
| 30.096° | 2.96689 Å | 12.5% |
| 31.206° | 2.86387 Å | 9.7% |
| 31.420° | 2.84488 Å | 11.3% |
| 31.748° | 2.81618 Å | 9.1% |
| 32.409° | 2.76031 Å | 7.8% |
| 36.566° | 2.45543 Å | 6.7% |
| 37.266° | 2.41094 Å | 8.0% |
| 40.084° | 2.24771 Å | 5.7% |
| 40.804° | 2.20969 Å | 8.1% |
| 41.861° | 2.15629 Å | 7.2% |

In another aspect of the present disclosure, the present disclosure also provides a crystal form U of the compound represented by formula (I), wherein the crystal form U has an X-ray powder diffraction pattern comprising characteristic diffraction peaks at the following 2θ angles: 8.01 ± 0.2°, 9.27 ± 0.2°, 12.68 ± 0.2°, 16.15 ± 0.2°, 17.94 ± 0.2°, and 19.31 ± 0.2°.

In some embodiments of the present disclosure, the X-ray powder diffraction pattern of the crystal form U comprises characteristic diffraction peaks at the following 2θ angles: 8.01 ± 0.2°, 9.27 ± 0.2°, 12.68 ± 0.2°, 16.15 ± 0.2°, 17.94 ± 0.2°, 19.31 ± 0.2°, 22.16 + 0.2°, 22.82 ± 0.2°, 23.80 ± 0.2°, and 24.08 ± 0.2°.

In some embodiments of the present disclosure, the X-ray powder diffraction pattern of the crystal form U has an X-ray powder diffraction pattern substantially as shown in FIG. 59.

In some embodiments of the present disclosure, the analytical data of the X-ray powder diffraction pattern of the crystal form U are as shown in Table 20 below.

Table 20

| 2θ | Interplanar spacing | Relative intensity (%) |
|---|---|---|
| 8.007° | 11.03265 Å | 84.4% |
| 8.403° | 10.51411 Å | 11.6% |
| 9.270° | 9.53232 Å | 67.8% |
| 12.676° | 6.97778 Å | 100.0% |
| 16.147° | 5.48477 Å | 68.8% |
| 17.183° | 5.15626 Å | 6.6% |
| 17.942° | 4.94000 Å | 24.6% |

(continued)

| 2θ | Interplanar spacing | Relative intensity (%) |
|---|---|---|
| 18.429° | 4.81041 Å | 10.8% |
| 19.314° | 4.59204 Å | 61.9% |
| 19.684° | 4.50648 Å | 50.1% |
| 20.499° | 4.32907 Å | 4.3% |
| 21.311° | 4.16593 Å | 3.9% |
| 22.163° | 4.00768 Å | 25.9% |
| 22.817° | 3.89420 Å | 19.3% |
| 23.145° | 3.83987 Å | 14.1% |
| 23.794° | 3.73653 Å | 46.0% |
| 24.084° | 3.69222 Å | 29.9% |
| 24.756° | 3.59350 Å | 12.4% |
| 25.432° | 3.49947 Å | 9.6% |
| 26.401° | 3.37320 Å | 29.8% |
| 26.871° | 3.31522 Å | 8.7% |
| 27.337° | 3.25978 Å | 12.0% |
| 33.374° | 2.68260 Å | 5.8% |

**Definition and description**

Unless otherwise specified, all of the technical and scientific terms used in the present disclosure have the same meaning as commonly understood by those skilled in the art to which the present disclosure belongs. All patents and publications referred to herein are incorporated in their entirety by reference. Although any methods and materials similar or identical to those described herein can be used in the practice or testing of the present disclosure, the preferred methods, devices, and materials are described herein.

"API" or "free state" refers to a free base form of the compound represented by formula (I).

"Crystal" or "crystal form" refers to a solid with a highly regular chemical structure, including, but not limited to, a single-component or multi-component crystal, and/or a polymorph, a solvate, a hydrate, a clathrate, a co-crystal, a salt, a solvate of the salt, a hydrate of the salt of a compound. The crystal form of the substance can be obtained by many methods known in the art. These methods include, but are not limited to, melt crystallization, melt cooling, solvent crystallization, crystallization in a confined space, for example, in nanopores or capillaries, crystallization on a surface or template, for example, on polymers, crystallization in the presence of an additive such as co-crystallized antimolecules, desolvation, dehydration, rapid evaporation, rapid cooling, slow cooling, vapor diffusion, sublimation, reactive crystallization, antisolvent addition, grinding, solvent drop grinding, *etc.*

"Amorphous" or "amorphous form" refers to a substance formed when its particles (molecules, atoms, ions) are arranged in three-dimensional space without periodicity, which is characterized by a diffuse X-ray powder diffraction pattern without sharp peaks. Amorphous is a special physical form of solid substance, and its locally ordered structural features suggest that it is inextricably linked to crystal substance. The amorphous form of the substance can be obtained by many methods known in the art. These methods include, but are not limited to, shock cooling, antisolvent flocculation, ball milling, spray drying, freeze drying, wet granulation, solid dispersion technology, *etc.*

"Solvent" refers to a substance (typically a liquid) that is capable of completely or partially dissolving another substance (typically a solid). Solvents used in the practice of the present disclosure include, but are not limited to, water, acetic acid, acetone, acetonitrile, benzene, chloroform, carbon tetrachloride, dichloromethane, dimethyl sulfoxide, 1,4-dioxane, ethanol, ethyl acetate, butanol, tert-butanol, N,N-dimethylacetamide, N,N-dimethylformamide, formamide, formic acid, heptane, hexane, isopropanol, methanol, methyl ethyl ketone, 1-methyl-2-pyrrolidone, mesitylene, nitromethane, polyethylene glycol, propanol, 2-propanone, pyridine, tetrahydrofuran, toluene, xylene, mixtures thereof, *etc.*

"Antisolvent" refers to a fluid that facilitates the precipitation of a product (or product precursor) from a solvent. The antisolvent may comprise a cold gas, or a fluid that facilitates the precipitation through a chemical reaction, or a fluid that reduces the solubility of the product in the solvent; it may be the same liquid as the solvent but at a different

temperature, or it may be a different liquid than the solvent.

"Solvate" means that the crystal has a solvent on the surface, or in the lattice, or both on the surface and in the lattice, wherein the solvent may be water, acetic acid, acetone, acetonitrile, benzene, chloroform, carbon tetrachloride, dichloromethane, dimethyl sulfoxide, 1,4-dioxane, ethanol, ethyl acetate, butanol, tert-butanol, N,N-dimethylacetamide, *N,N*-dimethylformamide, formamide, formic acid, heptane, hexane, isopropanol, methanol, methyl ethyl ketone, methyl pyrrolidone, mesitylene, nitromethane, polyethylene glycol, propanol, 2-propanone, pyridine, tetrahydrofuran, toluene, xylene, mixtures thereof, *etc*. A specific example of the solvate is a hydrate, wherein the solvent on the surface, or in the lattice, or both on the surface and in the lattice is water. On the surface of the substance, or in the lattice, or both on the surface and in the lattice, the hydrate may or may not have a solvent other than water.

The crystal form or amorphous form can be identified by a variety of techniques, such as X-ray powder diffraction (XRPD), infrared absorption spectroscopy (IR), melting point, differential scanning calorimetry (DSC), thermogravimetric analysis (TGA), nuclear magnetic resonance, Raman spectroscopy, X-ray single crystal diffraction, solution calorimetry, scanning electron microscopy (SEM), quantitative analysis, solubility, and dissolution rate.

X-ray powder diffraction (XRPD) is a common method for identifying crystal forms, which can detect changes in crystal form, crystallinity, crystal structure, and other information. The peak position of the XRPD pattern depends primarily on the structure of the crystal form and is relatively insensitive to experimental details, while its relative peak height depends on many factors related to sample preparation and instrument geometry. Therefore, in some examples, the crystal form of the present disclosure is characterized by an XRPD pattern with certain peak positions, which is substantially as shown in the XRPD pattern provided in the drawings of the present disclosure. At the same time, the measurement of 2θ of the XRPD pattern may have experimental errors, and the measurement of 2θ of the XRPD pattern may vary slightly between different instruments and different samples, so the value of 2θ cannot be regarded as absolute. According to the condition of the instrument used in the experiment of the present disclosure, there is an error tolerance of ± 0.2° for the diffraction peak.

Differential scanning calorimetry (DSC) is a program-controlled technique that measures the energy difference between a sample and an inert reference (commonly $\alpha$-$Al_2O_3$) as a function of temperature by continuously heating or cooling. The melting peak height of the DSC curve depends on many factors related to sample preparation and instrument geometry, while the peak position is relatively insensitive to experimental details. Therefore, in some examples, the crystal form of the present disclosure is characterized by a DSC pattern with characteristic peak positions, which is substantially as shown in the DSC pattern provided in the drawings of the present disclosure. At the same time, the DSC pattern may have experimental errors, and the peak position and the peak value of the DSC pattern may vary slightly between different instruments and different samples, so the peak position and the peak value of the DSC endothermic peak cannot be regarded as absolute. According to the condition of the instrument used in the experiment of the present disclosure, there is an error tolerance of ± 3°C for the melting peak.

Glass transition refers to the transition of an amorphous substance between a highly elastic state and a glass state, which is the inherent property of the substance; its corresponding transition temperature is the glass transition temperature (Tg), which is an important physical property of amorphous substances. The glass transition is a phenomenon related to molecular motion, and thus the glass transition temperature (Tg) depends primarily on the structure of the substance and is relatively insensitive to experimental details. In some examples, the amorphous glass transition temperature (Tg) of the present disclosure is determined by differential scanning calorimetry (DSC) and is characterized by having a glass transition temperature of 107.44°C. According to the condition of the instrument used in the experiment of the present disclosure, there is an error tolerance of ± 3°C for the glass transition temperature.

Differential scanning calorimetry (DSC) can also be used to detect and analyze whether the crystal form has the phenomenon of crystal transformation or mixed crystals.

Solids with the same chemical composition, under different thermodynamic conditions, often form isomers with different crystal structures, or variants, which is called polymorphism or homogeneous polyphase phenomenon. When temperature and pressure conditions change, the variants will transform into each other, which is called crystal transformation. Due to the crystal transformation, the mechanical, electrical, and magnetic properties of the crystal will change greatly. When the temperature of the crystal transformation is within a measurable range, the transformation process can be observed on a differential scanning calorimetry (DSC) pattern characterized by an exothermic peak reflecting the transformation process and two or more endothermic peaks at the same time, which are the characteristic endothermic peaks of different crystal forms before and after transformation. The crystal form or amorphous form of the compound of the present disclosure can undergo crystal transformation under appropriate conditions.

Thermogravimetric analysis (TGA) is a program-controlled technique for determining the mass of a substance as a function of temperature, which is suitable for checking the loss of solvent in crystals or the process of sublimation and decomposition of samples, and can infer the presence of crystallization water or crystallization solvent in crystals. The mass variation shown by the TGA curve depends on many factors such as sample preparation and instrument; the mass variation detected by TGA varies slightly between different instruments and different samples. According to the condition of the instrument used in the experiment of the present disclosure, there is an error tolerance of ± 0.3% for mass variation.

In the context of the present disclosure, the 2θ values in the X-ray powder diffraction pattern are all in degrees (°).

When referring to a pattern or/and data appearing in the pattern, "peak" refers to a feature that can be recognized by those skilled in the art and is not attributed to background noise.

The term "substantially as shown" means that at least 50%, or at least 60%, or at least 70%, or at least 80%, or at least 90%, or at least 95%, or at least 99% of the peaks in the X-ray powder diffraction pattern or DSC pattern or TGA result are shown in the pattern.

"Substantially pure" means that a crystal form is substantially free of one or more other crystal forms, *i.e.,* the crystal form has a purity of at least 80%, or at least 85%, or at least 90%, or at least 93%, or at least 95%, or at least 98%, or at least 99%, or at least 99.5%, or at least 99.6%, or at least 99.7%, or at least 99.8%, or at least 99.9%, or the crystal form contains other crystal forms whose percentage in the total volume or total weight of the crystal form is less than 20%, or less than 10%, or less than 5%, or less than 3%, or less than 1%, or less than 0.5%, or less than 0.1%, or less than 0.01%.

"Substantially free" means that the percentage of one or more other crystal forms in the total volume or total weight of the crystal form is less than 20%, or less than 10%, or less than 5%, or less than 4%, or less than 3%, or less than 2%, or less than 1%, or less than 0.5%, or less than 0.1%, or less than 0.01%.

"Relative intensity" refers to the ratio of the intensity of the other peaks to the intensity of the first strong peak among all diffraction peaks in the X-ray powder diffraction (XRPD) pattern when the intensity of the first strong peak is 100%.

In the context of the present disclosure, whether the word "about" or "approximately" is used or not, it means within 10%, appropriately within 5%, and especially within 1%, of a given value or range. Alternatively, for those of ordinary skill in the art, the term "about" or "approximately" means that it is within an acceptable standard error range of the mean. Whenever a number with a value of N is disclosed, any number with N +/- 1%, N +/- 2%, N +/- 3%, N +/- 5%, N +/- 7%, N +/- 8%, or N +/- 10% or less is explicitly disclosed, wherein "+/-" means plus or minus.

The term "comprises" is an open definition, which includes the contents specified in the present disclosure but does not exclude other aspects.

## BRIEF DESCRIPTION OF THE DRAWINGS

FIG. 1 shows the XRPD pattern of the crystal form A of the maleate according to an example of the present disclosure;
FIG. 2 shows the DSC and TGA pattern of the crystal form A of the maleate according to an example of the present disclosure;
FIG. 3 shows the NMR pattern of the crystal form A of the maleate according to an example of the present disclosure, wherein (a) shows the comparison with the free state and (b) shows the integration results;
FIG. 4 shows the DVS curve of the crystal form A of the maleate according to an example of the present disclosure;
FIG. 5 shows the XRPD pattern of the crystal form A of the maleate before and after DVS testing according to an example of the present disclosure;
FIG. 6 shows the XRPD pattern of the crystal form B of the maleate according to an example of the present disclosure;
FIG. 7 shows the DSC and TGA pattern of the crystal form B of the maleate according to an example of the present disclosure;
FIG. 8 shows the NMR pattern of the crystal form B of the maleate according to an example of the present disclosure;
FIG. 9 shows the XRPD pattern of the crystal form C of the fumarate according to an example of the present disclosure;
FIG. 10 shows the DSC and TGA pattern of the crystal form C of the fumarate according to an example of the present disclosure;
FIG. 11 shows the NMR pattern of the crystal form C of the fumarate according to an example of the present disclosure, wherein (a) shows the comparison with the free state and (b) shows the integration results;
FIG. 12 shows the XRPD pattern of the crystal form D of the hydrochloride according to an example of the present disclosure;
FIG. 13 shows the DSC and TGA pattern of the crystal form D of the hydrochloride according to an example of the present disclosure;
FIG. 14 shows the NMR pattern of the crystal form D of the hydrochloride according to an example of the present disclosure, wherein (a) shows the comparison with the free state and (b) shows the integration results;
FIG. 15 shows the XRPD pattern of the crystal form E of the hydrochloride according to an example of the present disclosure;
FIG. 16 shows the DSC and TGA pattern of the crystal form E of the hydrochloride according to an example of the present disclosure;
FIG. 17 shows the NMR pattern of the crystal form E of the hydrochloride according to an example of the present disclosure, wherein (a) shows the comparison with the free state and (b) shows the integration results;

FIG. 18 shows the XRPD pattern of the crystal form F of the hydrochloride according to an example of the present disclosure;

FIG. 19 shows the DSC and TGA pattern of the crystal form F of the hydrochloride according to an example of the present disclosure;

FIG. 20 shows the NMR pattern of the crystal form F of the hydrochloride according to an example of the present disclosure, wherein (a) shows the comparison with the free state and (b) shows the integration results;

FIG. 21 shows the XRPD pattern of the crystal form G of the sulfate according to an example of the present disclosure;

FIG. 22 shows the DSC and TGA pattern of the crystal form G of the sulfate according to an example of the present disclosure;

FIG. 23 shows the NMR pattern of the crystal form G of the sulfate according to an example of the present disclosure, wherein (a) shows the comparison with the free state and (b) shows the integration results;

FIG. 24 shows the XRPD pattern of the crystal form H of the succinate according to an example of the present disclosure;

FIG. 25 shows the DSC and TGA pattern of the crystal form H of the succinate according to an example of the present disclosure;

FIG. 26 shows the NMR pattern of the crystal form H of the succinate according to an example of the present disclosure, wherein (a) shows the comparison with the free state and (b) shows the integration results;

FIG. 27 shows the XRPD pattern of the crystal form J of the succinate according to an example of the present disclosure;

FIG. 28 shows the DSC and TGA pattern of the crystal form J of the succinate according to an example of the present disclosure;

FIG. 29 shows the NMR pattern of the crystal form J of the succinate according to an example of the present disclosure, wherein (a) shows the comparison with the free state and (b) shows the integration results;

FIG. 30 shows the XRPD pattern of the crystal form K of the glycollate according to an example of the present disclosure;

FIG. 31 shows the DSC and TGA pattern of the crystal form K of the glycollate according to an example of the present disclosure;

FIG. 32 shows the NMR pattern of the crystal form K of the glycollate according to an example of the present disclosure, wherein (a) shows the comparison with the free state and (b) shows the integration results;

FIG. 33 shows the XRPD pattern of the eutectic crystal form L of the benzoate according to an example of the present disclosure;

FIG. 34 shows the DSC and TGA pattern of the eutectic crystal form L of the benzoate according to an example of the present disclosure;

FIG. 35 shows the NMR pattern of the eutectic crystal form L of the benzoate according to an example of the present disclosure, wherein (a) shows the comparison with the free state and (b) shows the integration results;

FIG. 36 shows the XRPD pattern of the eutectic crystal form M of the benzoate according to an example of the present disclosure;

FIG. 37 shows the DSC and TGA pattern of the eutectic crystal form M of the benzoate according to an example of the present disclosure;

FIG. 38 shows the NMR pattern of the eutectic crystal form M of the benzoate according to an example of the present disclosure, wherein (a) shows the comparison with the free state and (b) shows the integration results;

FIG. 39 shows the XRPD pattern of the crystal form N according to an example of the present disclosure;

FIG. 40 shows the DSC and TGA pattern of the crystal form N according to an example of the present disclosure;

FIG. 41 shows the NMR pattern of the crystal form N according to an example of the present disclosure;

FIG. 42 shows the XRPD pattern of the crystal form O according to an example of the present disclosure;

FIG. 43 shows the DSC and TGA pattern of the crystal form O according to an example of the present disclosure;

FIG. 44 shows the NMR pattern of the crystal form O according to an example of the present disclosure;

FIG. 45 shows the XRPD pattern of the crystal form P according to an example of the present disclosure;

FIG. 46 shows the DSC and TGA pattern of the crystal form P according to an example of the present disclosure;

FIG. 47 shows the NMR pattern of the crystal form P according to an example of the present disclosure;

FIG. 48 shows the XRPD pattern of the crystal form Q according to an example of the present disclosure;

FIG. 49 shows the DSC and TGA pattern of the crystal form Q according to an example of the present disclosure;

FIG. 50 shows the XRPD pattern of the crystal form R according to an example of the present disclosure;

FIG. 51 shows the DSC and TGA pattern of the crystal form R according to an example of the present disclosure;

FIG. 52 shows the NMR pattern of the crystal form R according to an example of the present disclosure;

FIG. 53 shows the XRPD pattern of the crystal form S according to an example of the present disclosure;

FIG. 54 shows the DSC and TGA pattern of the crystal form S according to an example of the present disclosure;

FIG. 55 shows the NMR pattern of the crystal form S according to an example of the present disclosure;

FIG. 56 shows the XRPD pattern of the crystal form T according to an example of the present disclosure;
FIG. 57 shows the DSC and TGA pattern of the crystal form T according to an example of the present disclosure;
FIG. 58 shows the NMR pattern of the crystal form T according to an example of the present disclosure;
FIG. 59 shows the XRPD pattern of the crystal form U according to an example of the present disclosure;
FIG. 60 shows the DSC and TGA pattern of the crystal form U according to an example of the present disclosure;
FIG. 61 shows the NMR pattern of the crystal form U according to an example of the present disclosure, wherein (a) shows the comparison with the free state and (b) shows the integration results;
FIG. 62 shows the XRPD pattern of the crystal form A of the maleate in the stability study according to an example of the present disclosure;
FIG. 63 shows the XRPD pattern of the crystal form C of the fumarate in the stability study according to an example of the present disclosure;
FIG. 64 shows the XRPD pattern of the crystal form N in the stability study according to an example of the present disclosure;
FIG. 65 shows the XRPD pattern of the crystal form S in the stability study according to an example of the present disclosure;
FIG. 66 shows the XRPD comparison pattern of the remaining solid after 24 hours of oscillation in a medium for the crystal form A of the maleate according to an example of the present disclosure;
FIG. 67 shows the XRPD comparison pattern of the remaining solid after 24 hours of oscillation in a medium for the crystal form C of the fumarate according to an example of the present disclosure.

## DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and its specific embodiments have also been disclosed. It will be apparent to those skilled in the art that various variations and improvements can be made to the specific embodiments of the present disclosure without departing from the spirit and scope of the present disclosure.
The starting materials used in the present disclosure are all commercially available unless otherwise specified.
The present disclosure is described in detail by the examples below, but it does not mean that there are any adverse restrictions on the present disclosure. The present disclosure has been described in detail herein, and its specific embodiments have also been disclosed. It will be apparent to those skilled in the art that various variations and improvements can be made to the specific embodiments of the present disclosure without departing from the spirit and scope of the present disclosure.

General analysis methods:

1. Nuclear magnetic analysis ($^1$H NMR)

Several milligrams of solid sample were dissolved in dimethyl sulfoxide-$d_6$ solvent and subjected to NMR analysis on a Bruker AVANCE-III (Bruker, GER).

2. X-ray powder diffraction (XRPD)

Instrument model: Bruker D8 ADVANCE (Unique instrument number: IARC-031-PXRD-01).
Test standard: Chinese Pharmacopoeia, 2020 Edition, Volume IV, General Principle 0451.
Sample preparation: The sample was placed in the center of the groove in a sample holder, making the surface of the sample flush with the surface of the sample holder.
Experimental conditions: Cu*Ka* 40kv 40mA, divergence slit: 0.6 mm, Soller slit: 4.0°, continuous scanning, detector: LynxEye.

Step size: 0.02.
Scanning speed: 4°/min.

3. Thermogravimetric analysis (TGA)

The thermogravimetric analyzer is TA Discovery 55 (TA, US). 2 to 5 mg of sample was placed in a balanced open aluminum sample pan and automatically weighed in a TGA furnace. The sample was heated to a final temperature at a rate of 10°C/min with a nitrogen purge rate of 60 mL/min at the sample and 40 mL/min at the balance.

4. Differential scanning calorimetry (DSC)

The differential scanning calorimeter is TA Discovery 2500 (TA, US). 1 to 2 mg of sample was accurately weighed and placed in a perforated DSC Tzero sample pan, and heated to a final temperature at a rate of 10°C/min with a nitrogen purge rate of 50 mL/min in the furnace.

5. Dynamic vapor sorption analysis (DVS)

Dynamic vapor sorption analysis was performed using DVS Intrinsic (SMS, UK). The test was performed in gradient mode with a humidity change of 50%-95%-0%-50%, and 10% humidity change per gradient in the range of 0% to 90%. The gradient endpoint was determined by dm/dt, and was defined as when dm/dt was less than 0.002% and maintained for 10 minutes. After the test was completed, the sample was analyzed by XRPD to confirm whether the solid morphology had changed.

6. Polarized light microscopy (PLM)

The polarizing microscope is Nikon Ci-POL (Nikon, JP). A small amount of sample was placed on a slide and a suitable lens was selected to observe the sample morphology.

7. High-performance liquid chromatography (HPLC)

The high-performance liquid chromatography is LC-2030C 3D Plus (Shimadzu, JP), and the test conditions are shown in Table 21.

Table 21

| | | | |
|---|---|---|---|
| Chromatographic column | YMC Pack Pro C18 4.6 × 150 mm, 5 μm | | |
| Mobile phase | A: 0.01 mol/L diammonium phosphate solution, pH adjusted to 7 with phosphoric acid<br>B: Acetonitrile | | |
| Gradient | Time (min) | Mobile phase A (%) | Mobile phase B (%) |
| | 0 | 70 | 30 |
| | 1.5 | 50 | 50 |
| | 2 | 50 | 50 |
| | 10 | 20 | 80 |
| | 12 | 70 | 30 |
| Post-run time | 3 min | | |
| Flow rate | 1.0 mL/min | | |
| Injection volume | 10 μL | | |
| Detection wave-length | 240 nm | | |
| Column tempera-ture | 25°C | | |

The HPLC conditions used for testing the maleate content are shown in Table 22 below.

Table 22

| | |
|---|---|
| Chromatographic column | Xbridge Xselect CSH C18 4.6 × 150 mm, 3.5 μm |
| Mobile phase A | 0.1% formic acid solution |
| Mobile phase B | Acetonitrile solution |
| Column temperature | 40°C |

(continued)

| | |
|---|---|
| Flow rate | 1 mL/min |
| Elution method | Isocratic elution, 15% B |
| Run time | 8 min |
| Detection wavelength | 220 nm |
| Injection volume | 10 μL |
| Sample concentration | 0.2 mg/mL |
| Control concentration | 0.04 mg/mL |
| Diluent | 50% methanol |

8. Ion chromatography (IC)

The ion chromatography is ICS 5000 (Thermo Fisher, US), and the instrument parameters are shown in Table 23.

Table 23

| | |
|---|---|
| Chromatographic column | Dionex IonPacTM AS11-HC Analytical (4 × 250 mm) |
| Ion for detection | Chloride ion |
| Mobile phase | 20 mmol/L sodium hydroxide solution |
| Flow rate | 1.0 mL/min |
| Injection volume | 50 μL |
| Column temperature | 30°C |
| Run time | 20 in |

General test methods:

1. Solubility test for starting materials

Approximately 20 mg of sample was weighed and added to an EP tube, followed by the addition of a certain amount of solvent in batches at room temperature (about 25°C), and the mixture was stirred to observe whether the solid was completely dissolved. If the solid was not dissolved after adding up to 10.0 mL of solvent, the experiment was terminated. The solubility of the compound in the solvent was estimated based on the volume of solvent in which the solid was completely dissolved.

2. Reactive crystallization

2.1. 1 equivalent addition

Approximately 26 mg (0.05 mmol) of sample and 1 equivalent of acidic compound were added to a certain amount of selected solvent and suspended at room temperature for 2 days. The suspension was centrifuged, and the solid was dried under vacuum at room temperature. If the solution was clarified, the solution was placed in a refrigerator (-15°C); if a solid phase was precipitated, the solution was centrifuged to remove the supernatant, and the solid was dried under vacuum at room temperature. If no solid phase was precipitated after cooling, antisolvent was added dropwise to the solution until a solid was precipitated.

2.2. 2 equivalents addition

Approximately 26 mg (0.05 mmol) of sample and 2 equivalents of acidic compound were added to a certain amount of selected solvent and suspended at room temperature for 2 days. The suspension was centrifuged, and the solid was dried under vacuum at room temperature.

3. Solvent evaporation method

The clarified solution obtained from the solubility test was left in an open container at room temperature until the solvent was completely evaporated to obtain a solid. Alternatively, approximately 20 mg of the starting material was weighed, then an appropriate amount of selected solvent was added dropwise to a certain amount of poor solvent until it was completely dissolved, and the solution was left at room temperature to evaporate until the solvent was completely evaporated.

4. Suspension method

4.1. Suspension at room temperature

Using different crystal forms as starting materials, a certain amount of sample was added to the selected single solvent or binary solvent until a suspension was formed. The suspension was stirred at room temperature for a certain period of time, then centrifuged, and the solid was dried under vacuum at room temperature.

4.2. Suspension at 50°C

Using different crystal forms as starting materials, a certain amount of sample was added to the selected solvent until a suspension was formed. The suspension was stirred at 50°C for 1 day, then centrifuged, and the solid was dried under vacuum at room temperature.

5. Dilution crystallization

5.1. Dilution crystallization

Approximately 20 mg of sample was weighed, then a certain amount of good solvent was added dropwise at room temperature to completely dissolve the sample or prepare a saturated solution of good solvent, and the solution was added dropwise to 5 to 10 times the volume of poor solvent. After stirring for 1 hour to precipitate a solid, the system was centrifuged, and the solid was dried under vacuum at room temperature. The clarified solution was then stirred for 24 hours, and if no solid was precipitated, the system was placed in a refrigerator at 4 or -15°C. If a solid was precipitated, the system was centrifuged, and the solid was dried under vacuum at room temperature.

5.2. Binary solvent dropwise method

Approximately 20 mg of sample was weighed, then a certain amount of good solvent was added dropwise at room temperature to completely dissolve the sample or prepared as a saturated solution of good solvent, and poor solvent was added dropwise until a solid was precipitated. After stirring at room temperature for 15 minutes to precipitate a solid, the system was centrifuged, and the solid was dried under vacuum at room temperature. The clarified solution was then stirred for 24 hours, and if no solid was precipitated, the system was placed in a refrigerator at 4 or -15°C. If a solid was precipitated, the system was centrifuged, and the solid was dried under vacuum at room temperature.

6. Cooling method

6.1. Single solvent cooling

Approximately 20 mg of sample was weighed, and the preheated selected solvent was added dropwise at 50°C until the solid was just completely dissolved. The solution was quickly transferred to room temperature for cooling. The solution was left at room temperature for more than 2 hours. If no sufficient solid was precipitated, the solution was left at 4°C for further cooling. If still no sufficient solid was precipitated, the solution was left at -15°C for further cooling. If sufficient solid was precipitated, the system was centrifuged, and the solid was dried under vacuum at room temperature.

6.2. Binary solvent cooling

Approximately 20 mg of sample was weighed and mixed with a certain amount of poor solvent at 50°C to form a suspension. The preheated good solvent was gradually added dropwise until the solid was just completely dissolved, and the solution was transferred to room temperature for cooling. The solution was left at room temperature for more than 2 hours. If no sufficient solid was precipitated, the solution was left at 4°C for further cooling. If still no sufficient solid was

precipitated, the solution was left at -15°C for further cooling. If sufficient solid was precipitated, the system was centrifuged, and the solid was dried under vacuum at room temperature.

7. Vapor phase diffusion method

Approximately 20 mg of sample was weighed and dissolved in good solvent or prepared as a saturated solution of good solvent, and the clarified solution was left at room temperature under an atmosphere of poor solvent until a solid was precipitated. If a solid was precipitated, the solution was removed from the system using a syringe, and the wet sample was subjected to XRPD testing.

8. Solid/vapor phase diffusion

Approximately 20 mg of amorphous sample was weighed and left at room temperature or low temperature under an atmosphere of selected solvent for 7 days, during which the solid was regularly observed for properties in the glass vial and subjected to XRPD testing.

9. Thermal transformation

Thermal transformation was carried out using an Instec HCS424GXY hot stage (Instec Inc., USA), in which 6 to 8 mg of sample was placed on a glass slide on the hot stage, heated to the target temperature at a rate of 20°C/min and maintained for 5 to 10 minutes, and then naturally cooled to room temperature to obtain a solid.

10. Competitive water activity suspension experiment

An equal amount of selected crystal form sample was weighed and added to a certain volume of water/-isopropanol saturated solution with varying water contents (0%, 30%, 60%, volume percentage of water), and the mixture was suspended and stirred at room temperature and 60°C for a certain period of time. The suspension was centrifuged, and the wet sample was subjected to XRPD characterization.

11. Stability study

Approximately 20 mg of sample was weighed into a weighing bottle and exposed to high temperature (60°C), high humidity (25°C/92.5% RH), light (25°C/4500 Lux), and accelerated (40°C/75% RH) conditions, followed by sampling for XRPD characterization after 7 days and 15 days.

12. Solubility test

The preparation process of biological media is shown in Table 24. Samples of different crystal forms were added to biological media and shaken at a constant temperature of 37°C for 24 hours, followed by sampling at 0.5 hours, 2 hours, and 24 hours, respectively, and the sampled solution was filtered using a 0.22 μm hydrophilic filter membrane. Some of the samples with higher concentrations were appropriately diluted with a diluent, and the signal peak area of the solution was measured by HPLC. Finally, the concentration of the compound in the solution was calculated based on the peak area, the HPLC standard curve of the starting material, and the dilution factor. In addition, the 24-hour supernatant was tested for pH, and the remaining solid was subjected to XRPD testing.

Table 24

| Biological media | Preparation process |
|---|---|
| FaSSIF | a. Using a 50 mL volumetric flask, 2.0906 g of FaSSIF solution concentrate was weighed, and the volume was fixed with purified water;<br>b. 111.5 mg of FaSSIF/FeSSIF/FaSSGF powder was weighed, and the volume was fixed to 50 mL (pH 6.5) with the solution prepared in step a. |
| FeSSIF | a. Using a 50 mL volumetric flask, 4.0762 g of FeSSIF solution concentrate was weighed, and the volume was fixed with purified water;<br>b. 559.5 mg of FaSSIF/FeSSIF/FaSSGF powder was weighed, and the volume was fixed to 50 mL (pH 5.0) with the solution prepared in step a. |

(continued)

| Biological media | Preparation process |
|---|---|
| FaSSGF | a. Using a 100 mL volumetric flask, 3.66724 g of FaSSGF solution concentrate was weighed, and the volume was fixed with purified water;<br>b. 6.0 mg of FaSSIF/FeSSIF/FaSSGF powder was added to the solution prepared in step a and mixed well (pH 1.6). |

**Example 1: Preparation of compound represented by formula (I)**

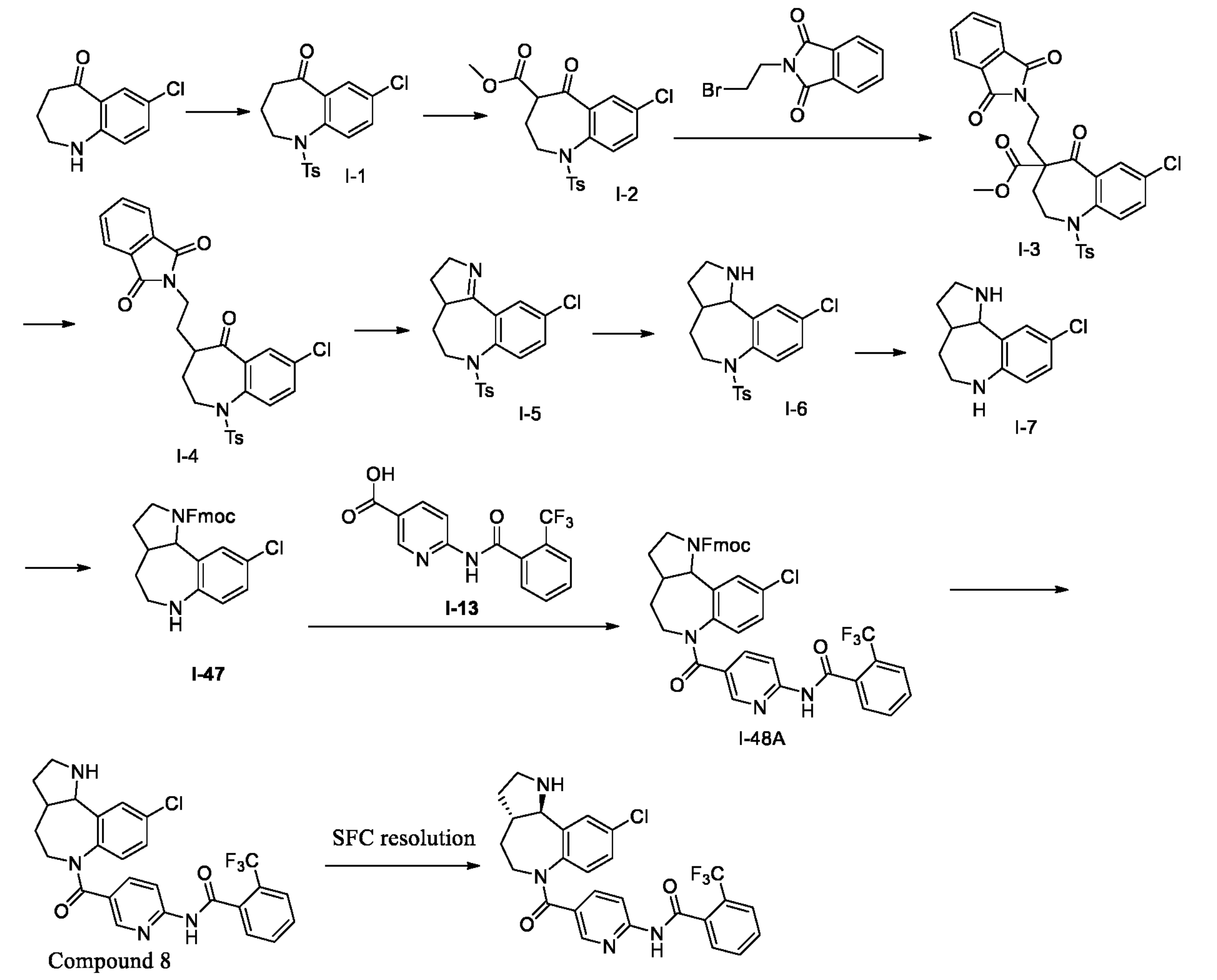


I-1 I-2 I-3 I-4 I-5 I-6 I-7 I-47 I-13 I-48A

Compound 8

Compound represented by formula (I)

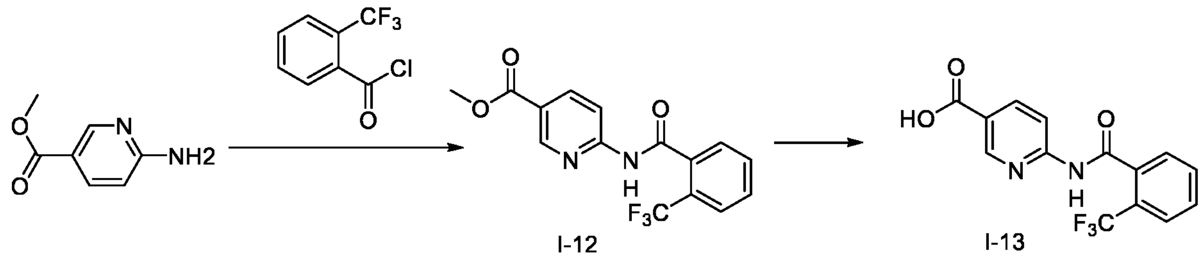

I-12 I-13

To a solution of 7-chloro-1,2,3,4-tetrahydrobenzo[B]azepin-5-one (15 g, 76.7 mmol) in pyridine (150 mL) was added *p*-toluenesulfonyl chloride (21.9 g, 115 mmol) at room temperature. The reaction mixture was reacted at room

temperature for 16 hours. The reaction mixture was concentrated under reduced pressure, poured into water (200 mL), extracted with ethyl acetate (100 mL × 3), and the organic phases were combined. The organic phase was washed with saturated sodium chloride solution (100 mL), dried over anhydrous sodium sulfate, and filtered. The filtrate was concentrated under reduced pressure to remove the organic solvent, and the residue was purified by silica gel chromatography to obtain intermediate I-1.

LC-MS (ESI) $[M+H]^+$ 349.9.

$^1H$ NMR (400 MHz, $CDCl_3$) δ 7.66 (d, *J* = 2.4 Hz, 1H), 7.58 (d, *J* = 8.3 Hz, 2H), 7.47 (dd, *J* = 8.6, 2.5 Hz, 1H), 7.43 (d, *J* = 8.5 Hz, 1H), 7.28 (d, *J* = 8.0 Hz, 2H), 3.83 (t, *J* = 6.5 Hz, 2H), 2.43 (s, 3H), 2.40 - 2.35 (m, 2H), 2.00 - 1.91 (m, 2H).

Intermediate I-1 (37.00 g, 106.00 mmol) was dissolved in anhydrous tetrahydrofuran (350 mL) at 25°C, and sodium hydride (6.36 g, 60% wt, 159.00 mmol) was added thereto in batches in an ice-water bath under an argon atmosphere. After cooling and stirring in an ice-water bath for 1 hour, dimethyl carbonate (19.08 g, 212.00 mmol) was added thereto, and the reaction mixture was heated to 50°C and stirred for 24 hours. After cooling, the reaction mixture was slowly poured into cold saturated ammonium chloride aqueous solution (500 mL), concentrated to remove most of the tetrahydrofuran, and filtered. The filter cake was washed with purified water, then slurried with petroleum ether, filtered, and dried under reduced pressure to obtain intermediate I-2.

LC-MS (ESI) $[M+H]^+$ 408.0.

Intermediate I-2 (19.00 g, 46.68 mmol) was dissolved in anhydrous *N,N*-dimethylformamide (187 mL) at 25°C, then sodium carbonate (14.84 g, 140.00 mmol) and 2-(2-bromoethyl)isoindoline-1,3-dione (23.71 g, 93.36 mmol) were sequentially added thereto, and the mixture was stirred overnight at 90°C under an argon atmosphere. The reaction mixture was cooled, then diluted with ethyl acetate (500 mL), washed with water (150 mL × 3) and saturated brine (100 mL), dried over anhydrous sodium sulfate, and filtered. The filtrate was concentrated under reduced pressure to obtain a crude product. The crude product was purified by silica gel chromatography to obtain intermediate I-3.

LC-MS (ESI) $[M+H]^+$ 581.2.

Intermediate I-3 (20.00 g, 34.48 mmol) was dissolved in dimethyl sulfoxide/water (130 mL/13 mL) at 25°C, and sodium chloride (16.70 g, 28.60 mmol) was added thereto. After the system was replaced with argon three times, the mixture was stirred at 150°C for 10 hours under an argon atmosphere. The reaction mixture was cooled, then diluted with ethyl acetate (400 mL), washed with water (150 mL × 3) and saturated brine (100 mL), dried over anhydrous sodium sulfate, and filtered. The filtrate was concentrated under reduced pressure to obtain a crude product. The crude product was purified by silica gel chromatography to obtain intermediate I-4.

LC-MS (ESI) $[M+H]^+$ 523.2.

Intermediate I-4 (200 mg, 0.38 mmol) was dissolved in ethanol (7 mL) at 25°C, then 85% hydrazine hydrate (0.35 mL) was added thereto, and the reaction mixture was stirred at 35°C for 4 hours. The reaction mixture was concentrated under reduced pressure to remove most of the ethanol, diluted with ethyl acetate (50 mL), sequentially washed with water (20 mL × 3) and saturated brine (20 mL), dried over anhydrous sodium sulfate, and filtered. The filtrate was concentrated under reduced pressure to obtain intermediate I-5. The crude product was directly used in the next reaction step.

LC-MS (ESI) $[M+H]^+$ 375.2.

Intermediate I-5 (170 mg, 0.45 mmol) was dissolved in methanol (10 mL) at 25°C, and sodium borohydride (190 mg, 5.00 mmol) was slowly added thereto under cooling in an ice-water bath. The reaction mixture was stirred at room temperature for 1 hour, concentrated under reduced pressure to remove most of the methanol, diluted with ethyl acetate (50 mL), sequentially washed with water (20 mL × 3) and saturated brine (20 mL), dried over anhydrous sodium sulfate, and filtered. The filtrate was concentrated under reduced pressure to obtain crude intermediate I-6. The crude product was directly used in the next reaction step.

LC-MS (ESI) $[M+H]^+$ 377.2.

Intermediate I-6 (150 mg, 0.40 mmol) was dissolved in anhydrous methanol (20 mL) at 25°C, and magnesium chips (2.00 g, 83.33 mmol) were added thereto. The reaction system was replaced with nitrogen three times, and stirred overnight at 70°C under a nitrogen atmosphere (balloon). After cooling, the reaction mixture was filtered through diatomite. The filtrate was concentrated to dryness, dissolved in a mixture of dichloromethane/methanol (10/1, 50 mL), washed with saturated ammonium chloride aqueous solution (20 mL × 3) and water (20 mL × 2), dried over anhydrous sodium sulfate, and filtered. The filtrate was concentrated under reduced pressure to obtain crude intermediate I-7. The crude product was directly used in the next reaction step.

LC-MS (ESI) $[M+H]^+$ 223.0.

Intermediate I-7 (7.30 g, 32.89 mmol) and triethylamine (10.10 g, 100.00 mmol) were dissolved in anhydrous dichloromethane (100 mL) at 25°C. 9-Fluorenylmethyl chloroformate (12.73 g, 49.33 mmol) was slowly added thereto in an ice-water bath under an argon atmosphere, and the mixture was stirred at 25°C for 16 hours. The reaction mixture was concentrated under reduced pressure at room temperature, slurried with petroleum ether, and filtered. The filter cake was washed with water (20 mL) and dried under reduced pressure to obtain intermediate I-47.

LC-MS (ESI) $[M+H]^+$ 444.8.

Methyl 6-aminonicotinate (1.0 g, 6.57 mmol) was dissolved in pyridine (20 mL) at room temperature, and 2-trifluoromethylbenzoyl chloride (1.51 g, 7.25 mmol) was added thereto. After the addition was completed, the reaction mixture was stirred at room temperature for 1 hour. The reaction mixture was poured into ice water (100 mL) and extracted with ethyl acetate (50 mL × 3). The combined organic phase was washed with water (50 mL × 5), dried over anhydrous sodium sulfate, and filtered. The filtrate was concentrated under reduced pressure, and the residue was purified by silica gel chromatography to obtain intermediate I-12.

LC-MS (ESI) $[M+H]^+$ 325.0.

Intermediate I-12 (1.35 g, 4.16 mmol) was dissolved in tetrahydrofuran (10 mL) at room temperature, and a solution of sodium hydroxide (499 mg, 12.5 mmol) in water (2 mL) was added thereto. After the addition was completed, the reaction mixture was stirred at 70°C for 1 hour. After the reaction was completed, the reaction mixture was added with 1 N hydrochloric acid to adjust the pH to 5 to 6. After filtration, the solid was dried to obtain intermediate 1-13.

LC-MS (ESI) $[M+H]^+$ 311.0.

Intermediate I-47 (4.70 g, 10.58 mmol) was dissolved in anhydrous tetrahydrofuran (35 mL) at 25°C, then pyridine (8.37 g, 106.00 mmol), intermediate I-13 (4.92 g, 15.87 mmol), and propylphosphonic anhydride (50% wt ethyl acetate solution, 20.00 g, 31.74 mmol) were sequentially added thereto, and the mixture was stirred overnight at 65°C under an argon atmosphere. The reaction mixture was cooled, concentrated to remove most of the tetrahydrofuran, and diluted with ethyl acetate (150 mL). The reaction mixture was sequentially washed with 1 N hydrochloric acid (100 mL × 2), saturated sodium bicarbonate aqueous solution (100 mL × 3), water (100 mL), and saturated sodium chloride aqueous solution (100 mL), dried over anhydrous sodium sulfate, and filtered. The filtrate was concentrated under reduced pressure to remove the organic solvent to obtain a crude product. The crude product was purified by silica gel chromatography to obtain intermediate I-48A (Rt = 1.541 min).

LCMS analysis method: Chromatographic column: Waters acquity UPLC CSH 2.1 × 50 mm, 1.7 μm.

Mobile phase: A: water (0.01% trifluoroacetic acid); B: acetonitrile (0.01% trifluoroacetic acid).

Elution gradient: 5% to 95% B, 0.7 minutes; 95% B, 0.8 minutes; then 5% B, 0.5 minutes.

Flow rate: 1.0 mL/min.

Column temperature: 60°C.

Mass spectrum scanning range: 100 to 1000.

Intermediate I-48A (Rt = 1.541 min) LC-MS (ESI) $[M+H]^+$ 737.3.

Intermediate I-48A (14 mg, 0.019 mmol) was dissolved in N,Ndimethylformamide (3 mL) at 25°C, then pyrrolidine (35.50 mg, 0.50 mmol) was added thereto, and the mixture was stirred at 25°C for 1 hour. The reaction mixture was diluted with ethyl acetate (20 mL), washed with water (10 mL × 3), dried over anhydrous sodium sulfate, and filtered. The filtrate was concentrated under reduced pressure and purified by C18 reverse phase chromatography (formic acid system) to obtain compound 8.

LC-MS (ESI) $[M+H]^+$ 515.2.

$^1H$ NMR (400 MHz, DMSO-$d_6$) δ 11.18 (s, 1H), 8.13 - 7.90 (m, 2H), 7.88 - 7.52 (m, 6H), 7.09 (dd, *J* = 8.3, 2.7 Hz, 1H), 6.89 (d, *J* = 8.3 Hz, 1H), 4.90 (dt, *J* = 13.7, 3.3 Hz, 1H), 4.14 (d, *J* = 9.2 Hz, 1H), 3.80 - 3.35 (m, 1H), 3.15 - 3.03 (m, 1H), 3.03 - 2.92 (m, 1H), 2.66 (t, *J* = 12.7 Hz, 1H), 2.09 (dd, *J* = 12.1, 4.1 Hz, 2H), 1.86 - 1.64 (m, 2H), 1.64 - 1.51 (m, 1H).

Compound 8 was subjected to SFC chiral resolution to obtain compound 10A (Rt = 1.424 min).

Chiral resolution method:

Instrument: MG II preparative SFC (SFC-14).
Chromatographic column: ChiralPakAD, 250 × 30 mm I.D., 10 μm.
Mobile phase: A: carbon dioxide; B: ethanol (0.1% ammonia water).
Elution gradient: 35% B.
Flow rate: 80 mL/min.
Back pressure: 100 bar.
Column temperature: 38°C.
Detection wavelength: 220 nm.
Cycle time: about 8 minutes.
Chiral analysis method:
Instrument: Waters UPC2 analytical SFC (SFC-H).
Chromatographic column: ChiralPak AD, 150 × 4.6 mm I.D., 3 μm.
Mobile phase: A: carbon dioxide; B: ethanol (0.05% diethylamine).
Elution gradient: 40% B.
Flow rate: 2.5 mL/min.
Back pressure: 1500 psi.

Column temperature: 35°C.
Detection wavelength: 220 nm.
Compound 10A:
Rt = 1.424 min.

LC-MS (ESI) $[M+H]^+$ 515.2.

$^1H$ NMR (400 MHz, DMSO-$d_6$) δ 11.18 (s, 1H), 8.30 - 7.88 (m, 2H), 7.88 - 7.55 (m, 6H), 7.10 (dd, *J* = 8.4, 2.7 Hz, 1H), 6.89 (d, *J* = 8.3 Hz, 1H), 4.90 (dt, *J* = 13.5, 3.2 Hz, 1H), 4.14 (d, *J* = 9.2 Hz, 1H), 3.40 - 3.39 (m, 1H), 3.13 - 2.90 (m, 2H), 2.66 (t, *J* = 12.3 Hz, 1H), 2.16 - 2.05 (m, 2H), 1.87 - 1.49 (m, 3H).

**Example 2: $IC_{50}$ test of inhibition of vasopressin-induced activation of vasopressin receptor V2R by compound represented by formula (I)**

(1) Cell

HeLa cell line stably expressing human vasopressin receptor V2R (HeLa-V2R): constructed by Shanghai Genechem Co., Ltd. using lentivirus infection method and verified to stably express human V2R by qPCR.

(2) Reagent

DMEM cell medium: Brand: Gibco, Cat. No.: 11995065; Fetal bovine serum: Brand: Genetimes, Cat. No.: FND500; 0.25% Trypsin: Brand: Gibco, Cat. No.: 25200072; Puromycin Dihydrochloride: Brand: Gibco, Cat. No.: A1113803; cAMP-GS HIRANGE kit: Brand: Cisbio, Cat. No.: 62AM6PEC; IBMX: Brand: Sigma, Cat. No.: i5879; vasopressin AVP: customized by GL Biochem (Shanghai), Ltd.

(3) Test method

HeLa-V2R cells were cultured in DMEM medium supplemented with 10% fetal bovine serum at 37°C with 5% $CO_2$, and the medium was added with 2 μg/mL puromycin to continuously screen cells expressing V2R. On the day of the experiment, the cells were digested with trypsin, washed twice with stimulation buffer in the cAMP-GS HIRANGE kit, resuspended and counted to make a concentration of $1.6 \times 10^6$ cells/mL, and IBMX was added until a final concentration of 0.5 mM. 5 μL/well of cell suspension was transferred to a 384-well plate. The corresponding wells were added with 2.5 μL of various concentrations of the test compound (3-fold dilution starting from 10 μM, 10 concentration gradients) or DMSO (minimum value Min, maximum value Max as a control), respectively. After incubating at room temperature for 30 minutes, the test compound well and the maximum value well were added with 2.5 μL of vasopressin AVP solution until a final concentration of 2.25 nM, and the minimum value well was added with 2.5 μL of stimulation buffer. The plate was incubated at 25°C for 60 minutes. At the same time, cAMP standard samples (3-fold dilution starting from 5.6 μM, 10 concentration points) were prepared, and 10 μL of cAMP standard was transferred to the corresponding wells of the 384-well plate. The cAMP-d2 fluorescence and anti-cAMP antibody probes provided in the cAMP-GS HIRANGE kit were 20-fold diluted with lysis buffer in the kit. 5 μL of each diluted reagent was sequentially added to each well of the 384-well plate and mixed well. The plate was then centrifuged briefly and incubated at 25°C for 2 hours before testing. The sample was assayed for fluorescence intensity at 615 nm and 665 nm on an Envision microplate reader using the HTRF method. Each test sample was tested in duplicate, with 32 replicates for both Min and Max, respectively.

(4) Data processing

The fluorescence intensity ratio $FI_{665/615}$ at 665 nm and 615 nm was calculated for the sample in each well. Taking the logarithm of the concentration of the standard as the X value and $FI_{665/615} \times 1000$ as the Y value, the standard curve was obtained by fitting the "log(inhibitor) vs response - variable slope (four parameters)" model in Prism 8.0 software. Taking $FI_{665/615} \times 1000$ of the test well as the Y value, the cAMP concentration corresponding to each sample was calculated based on the above standard curve in Prism 8.0 software.

%Inhibition (inhibition percentage) is calculated as follows:

$$\%\ \text{inhibition} = (\overline{\text{Cmax}} - \text{Ccmp}d)/(\overline{Cmax} - \overline{Cmin}) \times 100$$

wherein $\overline{\text{Cmax}}$ is the average calculated value of cAMP concentration in all maximum wells; $\overline{Cmin}$ is the average calculated

value of cAMP concentration in all minimum wells; Ccmpd is the calculated value of cAMP concentration of the test compound.

Taking %Inhibition (inhibition percentage) as the Y value and the logarithmic value of the compound concentration as the X value, the $IC_{50}$ was calculated by nonlinear regression with "log(inhibitor) vs response - variable slope (four parameters)" model in Prism 8.0 software, wherein $Y = Bottom + (Top - Bottom)/(1 + 10^{((LogIC_{50} - X) \times Hill Slope)})$.

The experimental results are shown in Table 25:

Table 25: Evaluation of compounds on inhibition of cAMP increase in human cervical cancer cells (Human V2R Hela-Stable cell line OE2)

| Compound No. | $IC_{50}$ (nM) |
|---|---|
| Compound represented by formula (I) | 30.97 |

**Example 3: Salt type screening**

Fifteen acidic compounds were selected for salt type screening by reactive crystallization. The results are shown in Tables 26 and 27 below.

Table 26: Salt type preliminary screening experiment by solution suspension method (1 equivalent)

| Solvent / Acidic compound | Isopropanol | Methyl *tert*-butyl ether | Cyclohexane |
|---|---|---|---|
| Hydrochloric acid | Crystal form D of hydrochloride | Amorphous | Crystal form E of hydrochloride |
| Sulfuric acid | Amorphous | Amorphous | Crystal form G of sulfate |
| Methanesulfonic acid | Amorphous* | Amorphous | Amorphous |
| *p*-Toluenesulfonic acid | Amorphous* | Amorphous | Amorphous |
| Maleic acid | Crystal form A of maleate | Crystal form A of maleate | Crystal form A of maleate |
| Phosphoric acid | Amorphous | Amorphous | Amorphous |
| Tartaric acid | Amorphous | Amorphous | Crystal form R of free base |
| Fumaric acid | Crystal form C of fumarate | Crystal form C of fumarate | Amorphous + fumaric acid |
| Citric acid | Amorphous | Amorphous | Amorphous + citric acid |
| Malic acid | Amorphous | Amorphous | Amorphous |
| Glycolic acid | Amorphous* | Crystal form K of glycollate | Crystal form K of glycollate |
| Benzoic acid | Eutectic crystal form L of benzoate | Amorphous | Eutectic crystal form M of benzoate |
| Succinic acid | Crystal form H of succinate | Crystal form J of succinate | Crystal form J of succinate |
| Ascorbic acid | Amorphous | Crystal form N of free base + ascorbic acid | Crystal form N of free base + ascorbic acid |
| Propionic acid | Amorphous* | Crystal form R of free base** | Crystal form U of free base |

Note: "*" stands for clarification after suspension and solid precipitation at -15°C; "**" stands for clarification after suspension and cooling to -15°C and solid precipitation after addition of *n*-heptane.

Table 27: Salt type preliminary screening experiment by solution suspension method (2 equivalents)

| Acidic compound \ Solvent | Isopropanol |
|---|---|
| Maleic acid | Crystal form A of maleate |
| Fumaric acid | Crystal form C of fumarate |
| Hydrochloric acid | Crystal form D of hydrochloride |

It can be seen from the above table that the compound represented by formula (I) can be formed into 6 kinds of salts, namely hydrochloride, sulfate, maleate, fumarate, succinate, and glycollate.

**Example 4: Preparation of crystal form A of maleate**

Method 1: 513.6 mg of the compound represented by formula (I) and 1 equivalent of maleic acid were weighed and added to 16 mL of isopropanol, and the mixture was stirred at room temperature for 2 days. The suspension was centrifuged, and the solid was dried under vacuum at 40°C for 3 days to obtain 611.5 mg of crystal form A of maleate.

Method 2: Methanol (30 mL, 10.0 v/w) was added to a jacketed reactor R1 at room temperature. The system was replaced with nitrogen three times. Free amine (3.0 g, 1 w, 5.82 mmol, 1.0 eq.) was added to the jacketed reactor R1 at room temperature and stirring was initiated. The mixture was stirred until clarified. A solution of maleic acid in isopropanol was prepared in a jacketed reactor R2. Specifically, maleic acid (0.71 g, 0.236 w, 6.11 mmol, 1.05 eq.) was dissolved in isopropanol (60 mL, 20.0 v/w) at 25 to 30°C. The prepared maleic acid/isopropanol solution was added dropwise to the reactor R1 for 20 minutes with the internal temperature controlled at 20 to 30°C. After the dropwise addition was completed, the mixture was reacted at 20 to 30°C for 2 to 5 hours. After filtration, the filter cake was washed once with isopropanol (9 mL, 3.0 v/w) and dried to obtain crystal form A of maleate.

Method 3: 10 L of methanol was added to a 100 L reactor, followed by the addition of 4.2 kg of free base and 6.8 L of methanol, and the mixture was heated to 60°C. 993 g of maleic acid was dissolved in 33.6 L of isopropanol, and the above solution was slowly added dropwise thereto. The mixture was cooled to 25°C and filtered to obtain 4.7 kg of crystal form A of maleate.

The crystal form A of maleate was characterized, and the results are shown in FIGs. 1 to 5. The XRPD (FIG. 1) results showed that the crystal form A of maleate is a solid with good crystallinity. The TGA (FIG. 2) results showed that the crystal form A of maleate had a weight loss of 0.5% when heated to 150°C, with possible decomposition above 200°C. The DSC (FIG. 2) results showed that the crystal form A of maleate had an endothermic peak at 229°C. The NMR (FIG. 3) results showed peak shifts at 1.50 to 1.90 ppm, 2.98 ppm, 3.07 ppm, 4.11 ppm, 6.90 ppm, and 7.08 ppm compared to the free state, suggesting salt formation for the sample. The signal peak for maleic acid was observed at 6.03 ppm, and based on the integration results, the ratio of starting material to maleic acid was calculated to be 1:1. The solvent peak for isopropanol was observed at 1.04 ppm, suggesting a small amount of residual isopropanol solvent in the sample. The DVS (FIG. 4) results showed that the crystal form A of maleate had a weight gain of 0.09% at 80% RH, a weight gain of 0.23% at 95% RH, and a weight loss of 0.24% at 0% RH, indicating that the crystal form A of maleate has almost no hygroscopicity. The XRPD (FIG. 5) results showed no morphological change in the sample after DVS testing. It can be seen that the crystal form A of maleate is an anhydrous form with good crystallinity and has almost no hygroscopicity.

**Example 5: Preparation of crystal form B of maleate**

21.7 mg of crystal form A of maleate was dissolved in 0.5 mL of DMF, and 1.5 mL of isopropanol was added thereto. The mixture was heated to 60°C, then slowly cooled to 15°C, and filtered to obtain crystal form B of maleate.

The XRPD (FIG. 6) results showed that the crystal form B is a solid with good crystallinity. The TGA (FIG. 7) results showed that the crystal form B had a weight loss of 9.7% when heated from room temperature to 170°C, with possible decomposition above 220°C. The DSC (FIG. 7) results showed that the crystal form B had an endothermic signal corresponding to desolvation at around 145°C and an endothermic peak at around 234°C. The NMR (FIG. 8) results were basically consistent with the starting material, and the solvent peaks for DMF were observed at 2.73, 2.89, and 7.95 ppm. Based on the integration results, the ratio of compound to DMF was calculated to be 1:0.9, and the DMF content was basically consistent with the TGA weight loss. The experimental results of thermal transformation showed that the crystal form B was transformed into the crystal form A when heated to 170°C for desolvation. In summary, the crystal form B is a DMF solvate.

**Example 6: Preparation of crystal form C of fumarate**

25.6 mg of the compound represented by formula (I) was added to 0.8 mL of isopropanol, followed by the addition of 1 equivalent of fumaric acid, and the mixture was stirred at room temperature for 2 days and centrifuged to obtain crystal form C of fumarate.

The XRPD (FIG. 9) results showed that the crystal form C of fumarate is a solid with good crystallinity. The TGA (FIG. 10) results showed that the sample had a weight loss of 0.7% when heated to 150°C, with possible decomposition above 190°C. The DSC (FIG. 10) results showed an endothermic signal at around 217°C. The NMR (FIG. 11) results showed peak shifts at 1.50 to 1.90 ppm, 2.98 ppm, 3.07 ppm, 4.11 ppm, 6.90 ppm, and 7.08 ppm compared to the free state, suggesting salt formation for the sample. The signal peak for fumaric acid was observed at 6.57 ppm, and based on the integration results, the ratio of API to fumaric acid was calculated to be 1:1. The solvent peak for isopropanol was observed at 1.04 ppm, suggesting a small amount of residual isopropanol solvent in the sample. In summary, the crystal form C of fumarate is an anhydrous form.

**Example 7: Preparation of crystal form D of hydrochloride**

25.3 mg of the compound represented by formula (I) was added to 0.8 mL of isopropanol, followed by the addition of 1 equivalent of concentrated hydrochloric acid, and the mixture was stirred at room temperature for 2 days and centrifuged to obtain crystal form D of hydrochloride.

The XRPD (FIG. 12) results showed that the crystal form D of hydrochloride is a solid with good crystallinity. The TGA (FIG. 13) results showed that the sample had a weight loss of 1.6% (corresponding to a weight loss of 0.5 water molecules) when heated to 150°C, with possible decomposition above 230°C. The DSC (FIG. 13) results showed endothermic signals at around 71°C and 262°C. The NMR (FIG. 14) results showed multiple peak shifts at 1.50 to 1.90 ppm, 2.98 ppm, 3.07 ppm, 4.11 ppm, 6.90 ppm, and 7.08 ppm compared to the free state, suggesting salt formation for the sample. The solvent peak for isopropanol was observed at 1.04 ppm, suggesting a small amount of residual isopropanol solvent in the sample. The experimental results of thermal transformation showed that the crystal form D of hydrochloride was transformed into crystal form F of hydrochloride when heated to 150°C. The results of ion chromatography showed that the salt formation ratio for the crystal form D of hydrochloride was 1:1. In summary, the crystal form D of hydrochloride is a hydrate.

**Example 8: Preparation of crystal form E of hydrochloride**

25.3 mg of the compound represented by formula (I) was added to 1.0 mL of cyclohexane, followed by the addition of 1 equivalent of concentrated hydrochloric acid, and the mixture was stirred at room temperature for 2 days and centrifuged to obtain crystal form E of hydrochloride.

The XRPD (FIG. 15) results showed that the crystal form E of hydrochloride is a solid with poor crystallinity. The TGA (FIG. 16) results showed that the sample had a sustained weight loss during the heating process. The DSC (FIG. 16) results showed endothermic signals at around 89°C and 201°C. The NMR (FIG. 17) results showed peak shifts at 1.50 to 1.90 ppm, 2.98 ppm, 3.07 ppm, 6.90 ppm, and 7.08 ppm compared to the free state, suggesting salt formation for the sample. The solvent peak for cyclohexane was observed at 1.39 ppm, suggesting a small amount of residual cyclohexane solvent in the sample. In summary, the crystal form E of hydrochloride may be an anhydride or a hydrate that absorbs water.

**Example 9: Preparation of crystal form F of hydrochloride**

25.4 mg of crystal form N was added to 0.8 mL of isopropanol, followed by the addition of 1 equivalent of hydrochloric acid, and the mixture was stirred at room temperature for 2 days. The suspension was centrifuged, and the solid was dried under vacuum at room temperature to obtain crystal form F of hydrochloride.

The XRPD (FIG. 18) results showed that the crystal form F of hydrochloride is a solid with good crystallinity. The TGA (FIG. 19) results showed that the sample had a weight loss of 0.5% when heated to 150°C, with possible decomposition above 230°C. The DSC (FIG. 19) results showed endothermic signals at around 316°C and 320°C. The NMR (FIG. 20) results showed peak shifts at 1.50 to 1.90 ppm, 2.98 ppm, 3.07 ppm, 4.11 ppm, 6.90 ppm, and 7.08 ppm compared to the free state, suggesting salt formation for the sample. The solvent peak for isopropanol was observed at 1.04 ppm, suggesting a small amount of residual isopropanol solvent in the sample. The results of ion chromatography showed that the salt formation ratio for the crystal form F of hydrochloride was 1:1. In summary, the crystal form F of hydrochloride is an anhydrous form.

**Example 10: Preparation of crystal form G of sulfate**

26.1 mg of the compound represented by formula (I) was added to 1.0 mL of cyclohexane, followed by the addition of 1 equivalent of concentrated sulfuric acid, and the mixture was stirred at room temperature for 2 days and centrifuged to obtain crystal form G of sulfate.
The XRPD (FIG. 21) results showed that the crystal form G of sulfate is a solid with good crystallinity. The TGA (FIG. 22) results showed that the sample had a weight loss of 3.7% when heated to 150°C, with possible decomposition above 240°C. The DSC (FIG. 22) results showed endothermic signals at around 282°C and 298°C. The NMR (FIG. 23) results showed peak shifts at 1.50 to 1.90 ppm, 2.98 ppm, 3.07 ppm, 6.90 ppm, and 7.08 ppm compared to the free state, suggesting salt formation for the sample. The solvent peak for cyclohexane was observed at 1.39 ppm, suggesting a very small amount of residual cyclohexane solvent in the sample. In summary, the crystal form G of sulfate may be an anhydride or a hydrate that absorbs water.

**Example 11: Preparation of crystal form H of succinate**

24.1 mg of the compound represented by formula (I) was added to 0.8 mL of isopropanol, followed by the addition of 1 equivalent of succinic acid, and the mixture was stirred at room temperature for 2 days and centrifuged to obtain crystal form H of succinate.
The XRPD (FIG. 24) results showed that the crystal form H of succinate is a solid with good crystallinity. The TGA (FIG. 25) results showed that the sample had a weight loss of 0.2% when heated to 120°C, with possible decomposition above 170°C. The DSC (FIG. 25) results showed an endothermic signal at around 186°C. The NMR (FIG. 26) results showed peak shifts at 1.50 to 1.90 ppm, 2.98 ppm, 3.07 ppm, 4.11 ppm, 6.90 ppm, and 7.08 ppm compared to the free state, suggesting salt formation for the sample. The signal peak for succinic acid was observed at 2.39 ppm, and based on the integration results, the ratio of API to succinic acid was calculated to be 1:1. The solvent peak for isopropanol was observed at 1.04 ppm, suggesting a small amount of residual isopropanol solvent in the sample. In summary, the crystal form H of succinate is an anhydrous form.

**Example 12: Preparation of crystal form J of succinate**

24.7 mg of the compound represented by formula (I) was added to 1.0 mL of methyl *tert-butyl* ether (MTBE), followed by the addition of 1 equivalent of succinic acid, and the mixture was stirred at room temperature for 2 days and centrifuged to obtain crystal form J of succinate.
The XRPD (FIG. 27) results showed that the crystal form J of succinate is a solid with good crystallinity. The TGA (FIG. 28) results showed that the sample had a weight loss of 0.6% when heated to 150°C, with possible decomposition above 170°C. The DSC results showed an endothermic signal at around 177°C. The NMR (FIG. 29) results showed peak shifts at 1.50 to 1.90 ppm, 2.98 ppm, 3.07 ppm, 4.11 ppm, 6.90 ppm, and 7.08 ppm compared to the free state, suggesting salt formation for the sample. The signal peak for succinic acid was observed at 2.39 ppm, and based on the integration results, the ratio of API to succinic acid was calculated to be 1:1. The solvent peak for MTBE was observed at 1.10 ppm, suggesting a small amount of residual MTBE solvent in the sample. In summary, the crystal form J of succinate is an anhydrous form.

**Example 13: Preparation of crystal form K of glycollate**

25.9 mg of the compound represented by formula (I) was added to 1.0 mL of MTBE, followed by the addition of 1 equivalent of glycolic acid, and the mixture was stirred at room temperature for 2 days and centrifuged to obtain crystal form K of glycollate.
The XRPD (FIG. 30) results showed that the crystal form K of glycollate is a solid with good crystallinity. The TGA (FIG. 31) results showed that the sample had a weight loss of 0.7% when heated to 70°C and a sustained weight loss during the subsequent heating process, with possible decomposition above 150°C. The DSC results showed an endothermic signal at around 98°C. The NMR (FIG. 32) results showed peak shifts at 2.98 ppm, 3.07 ppm, 4.11 ppm, 6.90 ppm, and 7.08 ppm compared to the free state, suggesting salt formation for the sample. The signal peak for glycolic acid was observed at 3.87 ppm. The solvent peak for MTBE was observed at 1.10 ppm, suggesting a very small amount of residual MTBE solvent in the sample. In summary, the crystal form K of glycollate may be an anhydride/a hydrate.

**Example 14: Preparation of eutectic crystal form L of benzoate**

24.7 mg of the compound represented by formula (I) was added to 0.8 mL of isopropanol, followed by the addition of 1 equivalent of benzoic acid, and the mixture was stirred at room temperature for 2 days and centrifuged to obtain

eutectic crystal form L of benzoate.

The XRPD (FIG. 33) results showed that the eutectic crystal form L of benzoate is a solid with good crystallinity. The TGA (FIG. 34) results showed that the sample had a weight loss of 1.6% when heated to 100°C, with possible decomposition above 135°C. The DSC (FIG. 34) results showed endothermic signals at around 165°C and 177°C. The NMR (FIG. 35) results showed no peak shift compared to the free state, suggesting no salt formation and possible eutectic formation for the sample. The signal peak for benzoic acid was observed at 7.0 to 8.5 ppm, and based on the integration results, the ratio of API to benzoic acid was calculated to be 1:1. The solvent peak for isopropanol was observed at 1.04 ppm, suggesting a small amount of residual isopropanol solvent in the sample. In summary, the eutectic crystal form L of benzoate is an anhydrous form.

**Example 15: Preparation of eutectic crystal form M of benzoate**

25.2 mg of the compound represented by formula (I) was added to 1.0 mL of cyclohexane, followed by the addition of 1 equivalent of benzoic acid, and the mixture was stirred at room temperature for 2 days and centrifuged to obtain eutectic crystal form M of benzoate.

The XRPD (FIG. 36) results showed that the eutectic crystal form M of benzoate is a solid with poor crystallinity. The TGA (FIG. 37) results showed that the sample had a weight loss of 1.0% when heated to 100°C, with possible decomposition above 135°C. The DSC (FIG. 37) results showed an endothermic signal at around 175°C. The NMR (FIG. 38) results showed no peak shift compared to the free state, suggesting no salt formation and possible eutectic formation for the sample. The signal peak for benzoic acid was observed at 7.0 to 8.5 ppm, and based on the integration results, the ratio of starting material to benzoic acid was calculated to be 1:1. The solvent peak for cyclohexane was observed at 1.39 ppm, suggesting a small amount of residual cyclohexane solvent in the sample. In summary, the eutectic crystal form M of benzoate is an anhydrous form.

**Example 16: Preparation of crystal form N**

20.4 mg of the compound represented by formula (I) was added to 0.5 mL of toluene. The mixture was suspended and stirred at 50°C for 1 day, and centrifuged to obtain crystal form N.

The XRPD (FIG. 39) results showed that the crystal form N is a solid with good crystallinity. The TGA (FIG. 40) results showed that the crystal form N had a weight loss of 0.6% when heated to 150°C, with possible decomposition above 300°C. The DSC (FIG. 40) results showed that the crystal form N had a melting endothermic peak at around 208°C. The NMR (FIG. 41) results showed that the solvent peak for toluene was observed at 2.30 ppm, suggesting a small amount of residual toluene solvent in the sample. In summary, the crystal form N is an anhydrous form.

**Example 17: Preparation of crystal form O**

19.5 mg of the compound represented by formula (I) was dissolved in 0.6 mL of ethanol, and 9.0 mL of n-heptane was slowly added thereto. The mixture was suspended and stirred for 15 minutes, and centrifuged to obtain crystal form O.

The XRPD (FIG. 42) results showed that the crystal form O is a solid with good crystallinity. The TGA (FIG. 43) results showed that the crystal form O had a weight loss of 2.6% when heated to 200°C, with possible decomposition above 300°C. The DSC (FIG. 43) results showed that the crystal form O had an exothermic peak at around 196°C, an endothermic signal at around 190°C, and a melting endothermic peak at around 208°C. The experimental results of thermal transformation showed that the crystal form O was recrystallized into the crystal form N after desolvation. The NMR (FIG. 44) results showed that the solvent peak for ethanol was observed at 1.06 ppm. Based on the integration results, the ratio of compound to ethanol was calculated to be 1:0.25, which was consistent with the TGA weight loss (a theoretical weight loss of 2.2%). In summary, the crystal form O is an ethanol/n-propanol solvate, or an anhydrous form containing ethanol/n-propanol.

**Example 18: Preparation of crystal form P**

20.9 mg of the compound represented by formula (I) was dissolved in 1.4 mL of acetonitrile and 1.0 mL of water. The mixture was left at room temperature to evaporate until the solvent was completely evaporated, and centrifuged to obtain crystal form P.

The XRPD (FIG. 45) results showed that the crystal form P is a solid with good crystallinity and obvious preferred orientation. The TGA (FIG. 46) results showed that the crystal form P had a weight loss of 3.3% when heated to 175°C, with possible decomposition above 300°C. The DSC (FIG. 46) results showed that the crystal form P had an exothermic peak at around 158°C, an endothermic signal at around 145°C, and a melting endothermic peak at around 211°C. The experimental results of thermal transformation showed that the crystal form P was recrystallized into the crystal form

N after desolvation. The NMR (FIG. 47) results showed that the sample had no obvious organic solvent peak. In summary, the crystal form P is a hydrate.

**Example 19: Preparation of crystal form Q**

19.9 mg of the compound represented by formula (I) was dissolved in 4.5 mL of diethyl ether. The mixture was left in an open container at room temperature until the solvent was completely evaporated to obtain crystal form Q.

The XRPD (FIG. 48) results showed that the crystal form Q is a solid with good crystallinity. The TGA (FIG. 49) results showed that the crystal form Q had a weight loss of 4.6% when heated to 180°C, with possible decomposition above 300°C. The DSC (FIG. 49) results showed that the crystal form Q had an exothermic peak at around 181°C, endothermic signals at around 171°C and 198°C, and a melting endothermic peak at around 207°C. The experimental results of thermal transformation showed that the crystal form Q was recrystallized into the crystal form N after desolvation.

**Example 20: Preparation of crystal form R**

0.8 mL of acetonitrile was added dropwise to 19.9 mg of the compound represented by formula (I) at room temperature until it was completely dissolved, and 3.0 mL of water was added dropwise thereto until a solid was precipitated. After suspension at room temperature for 15 minutes, the system was centrifuged and dried under vacuum at room temperature to obtain crystal form R.

The XRPD (FIG. 50) results showed that the crystal form R is a solid with good crystallinity. The TGA (FIG. 51) results showed that the crystal form R had a weight loss of 3.7% when heated to 150°C, with possible decomposition above 300°C. The DSC (FIG. 51) results showed that the crystal form R had an exothermic peak at around 161°C, an endothermic signal at around 154°C, and a melting endothermic peak at around 211°C. The experimental results of thermal transformation showed that the crystal form R was recrystallized into the crystal form N after desolvation. The NMR (FIG. 52) results showed no obvious organic solvent peak. In summary, the crystal form R is a hydrate.

**Example 21: Preparation of crystal form S**

400.0 mg of the compound represented by formula (I) was placed in a glass vial, then 10.5 mL of a mixed solvent of isopropanol/water (1/6, v/v) was added thereto, and the mixture was suspended at 50°C for 1 day. The resulting solid was centrifuged and dried under vacuum at 40°C for 1 day to obtain crystal form S.

The XRPD (FIG. 53) results showed that the crystal form S is a solid with good crystallinity. The TGA (FIG. 54) results showed that the crystal form S had a weight loss of 3.6% when heated to 150°C, with possible decomposition above 300°C. The DSC (FIG. 54) results showed that the crystal form S had an exothermic peak at around 155°C, an endothermic signal at around 126°C, and a melting endothermic peak at around 210°C. The experimental results of thermal transformation showed that the crystal form S was recrystallized into the crystal form N after desolvation. In summary, the crystal form S is a hydrate. The NMR results are shown in FIG. 55.

**Example 22: Preparation of crystal form T**

20.4 mg of the compound represented by formula (I) was dissolved in 0.1 mL of dichloromethane, and the mixture was evaporated to dryness in an open container at room temperature to obtain crystal form T.

The XRPD (FIG. 56) results showed that the crystal form T is a solid with good crystallinity. The TGA (FIG. 57) results showed that the crystal form T had a weight loss of 6.7% when heated to 200°C, with possible decomposition above 300°C. The DSC (FIG. 57) results showed that the crystal form T had an exothermic peak at around 191°C, an endothermic signal at around 186°C, and a melting endothermic peak at around 209°C. The NMR (FIG. 58) results showed no change in the structure of the compound, with solvent peaks for dichloromethane and a small amount of dioxane observed at 5.76 ppm and 3.57 ppm. Based on the integration results, the ratio of compound to dichloromethane was calculated to be 1:0.25. In summary, the crystal form T is a dichloromethane solvate or an anhydrous form containing dichloromethane.

**Example 23: Preparation of crystal form U**

23.5 mg of the compound represented by formula (I) was added to 1.0 mL of cyclohexane, followed by the addition of 1 equivalent of propionic acid, and the mixture was stirred at room temperature for 2 days. The suspension was centrifuged, and the solid was dried under vacuum at room temperature to obtain crystal form U.

The XRPD (FIG. 59) results showed that the crystal form U is a solid with good crystallinity. The TGA (FIG. 60) results showed that the sample had a weight loss of 18.0% when heated to 130°C, corresponding to a weight loss of approximately 2 propionic acid molecules. The DSC (FIG. 60) results showed endothermic signals at around 109°C,

121°C, and 204°C. The NMR (FIG. 61) results showed no peak shift compared to the free state, suggesting no salt formation for the sample. The signal peaks for propionic acid were observed at 0.99 ppm and 2.20 ppm, and based on the integration results, the ratio of API to propionic acid was calculated to be 1:2. The solvent peak for cyclohexane was observed at 1.39 ppm, suggesting a small amount of residual cyclohexane solvent in the sample. In summary, the crystal form U is a propionic acid solvate.

**Example 24: Stability study**

The stability study was performed on crystal form A of maleate, crystal form C of fumarate, crystal form N, and crystal form S under high temperature (60°C), high humidity (25°C/92.5% RH), light (25°C/4500 Lux), and accelerated (40°C/75% RH) conditions. Samples were taken for XRPD characterization after 7 days and 15 days. The results are shown in Table 28 and FIGs. 62 to 65. The XRPD results showed that the crystal form A of maleate and crystal form C of fumarate were stable under high temperature, high humidity, light, and accelerated conditions for 15 days without any crystal transformation.

Table 28

| Salt type | Condition | Results after 7 days | Results after 15 days |
|---|---|---|---|
| Crystal form A of maleate | High temperature, 60°C | No change | No change |
| | High humidity, 25°C/92.5% RH | No change | No change |
| | Light, 25°C/4500 Lux | No change | No change |
| | Accelerated, 40°C/75% RH | No change | No change |
| Crystal form C of fumarate | High temperature, 60°C | No change | No change |
| | High humidity, 25°C/92.5% RH | No change | No change |
| | Light, 25°C/4500 Lux | No change | No change |
| | Accelerated, 40°C/75% RH | No change | No change |
| Crystal form N | High temperature, 60°C | No change | No change |
| | High humidity, 25°C/92.5% RH | No change | No change |
| | Light, 25°C/4500 Lux | No change | No change |
| | Accelerated, 40°C/75% RH | No change | No change |
| Crystal form S | High temperature, 60°C | No change | No change |
| | High humidity, 25°C/92.5% RH | No change | No change |
| | Light, 25°C/4500 Lux | No change | No change |
| | Accelerated, 40°C/75% RH | No change | No change |

**Example 25: Solubility test in biological media**

Dynamic solubility measurements were performed in three biological media (FaSSIF, FeSSIF, and FaSSGF). The results are shown in Table 29 and FIGs. 66 and 67 below.

Table 29

| Salt type | Medium | pH | Sample amount (mg) | Volume (mL) | Solubility (mg/mL) | | | Final pH | XRPD for remaining solid |
|---|---|---|---|---|---|---|---|---|---|
| | | | | | 0.5 h | 2h | 24 h | | |
| Crystal form A of maleate | FaSSIF | 6.5 | 20.2 | 4.0 | 0.66 | 0.69 | 0.57 | 6.1 | Obvious change |
| | FeSSIF | 5.1 | 19.9 | 4.0 | 1.08 | 1.28 | 0.31 | 5.0 | Obvious change |
| | FaSSGF | 1.6 | 20.2 | 4.0 | 1.44 | 1.43 | 1.02 | 1.7 | Crystal form A of maleate + new peak |

(continued)

| Salt type | Medium | pH | Sample amount (mg) | Volume (mL) | Solubility (mg/mL) | | | Final pH | XRPD for remaining solid |
|---|---|---|---|---|---|---|---|---|---|
| | | | | | 0.5 h | 2h | 24 h | | |
| Crystal form C of fumarate | FaSSIF | 6.5 | 20.2 | 4.0 | 0.38 | 0.35 | 0.28 | 6.1 | Crystal form C of fumarate + new peak |
| | FeSSIF | 5.1 | 19.9 | 4.0 | 0.66 | 0.74 | 0.68 | 5.0 | Crystal form C of fumarate + new peak |
| | FaSSGF | 1.6 | 20.2 | 4.0 | 0.65 | 0.70 | 0.71 | 1.7 | Obvious change |
| Crystal form N | FaSSIF | 6.5 | 20.1 | / | 0.06 | 0.07 | 0.08 | 6.5 | Crystal form N |
| | FeSSIF | 5.0 | 20.0 | / | 0.80 | 0.97 | 0.94 | 5.1 | Crystal form N |
| | FaSSGF | 1.6 | 40.0 | / | > 7.61 | > 7.89 | > 7.98 | 2.0 | No solid |
| | Water | - | 20.0 | / | 0.004 | 0.011 | 0.004 | 7.5 | Crystal form N |
| Crystal form S | FaSSIF | 6.5 | 20.0 | / | 0.03 | 0.04 | 0.04 | 6.5 | Crystal form S |
| | FeSSIF | 5.0 | 19.7 | / | 0.40 | 0.44 | 0.43 | 5.1 | Crystal form S |
| | FaSSGF | 1.6 | 38.9 | / | > 7.19 | > 7.35 | 2.15 | 2.0 | Amorphous* |
| | Water | - | 19.2 | / | < LOD | 0.001 | 0.004 | 7.4 | Crystal form S |
| *The sample was first dissolved in FaSSGF and then shaken for 24 hours to precipitate a solid, which was amorphous. | | | | | | | | | |

In this specification, the descriptions of the terms such as "an embodiment", "some embodiments", "example", "specific example", or "some examples" mean that a specific feature, structure, material, or characteristic described in reference to the embodiment or example is included in at least one embodiment or example of the present disclosure. In this specification, the schematic descriptions of the above terms do not necessarily refer to the same embodiment or example. Furthermore, the specific feature, structure, material, or characteristic described can be combined in any suitable manner in any one or more embodiments or examples. In addition, various embodiments or examples and features of various embodiments or examples described in this specification can be combined by those skilled in the art to the extent that they do not contradict each other.

Although the examples of the present disclosure have been illustrated and described above, it can be understood that the above examples are illustrative and should not be construed as limiting the present disclosure. Those skilled in the art can make changes, modifications, substitutions, and variations based on the above examples within the scope of the present disclosure.

## Claims

**1.** A maleate of a compound represented by formula (I), whose structure is represented by formula (II),

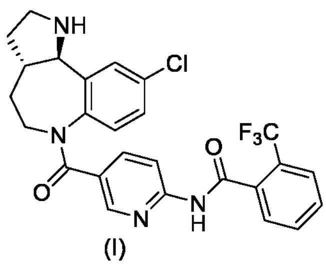

(I)

,

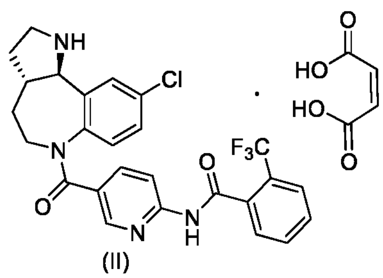

(II)

.

**2.** A crystal form A of a maleate of a compound represented by formula (I), wherein the crystal form A has an X-ray

powder diffraction pattern comprising characteristic diffraction peaks at the following 2θ angles: 9.14 ± 0.2°, 12.88 ± 0.2°, 18.31 ± 0.2°, 18.90 ± 0.2°, 20.60 ± 0.2°, and 27.61 ± 0.2°;

optionally, the X-ray powder diffraction pattern of the crystal form A comprises characteristic diffraction peaks at the following 2θ angles: 9.14 ± 0.2°, 12.88 ± 0.2°, 17.19 + 0.2°, 18.31 ± 0.2°, 18.90 ± 0.2°, 20.60 ± 0.2°, 21.45 ± 0.2°, and 27.61 ± 0.2°;
optionally, the X-ray powder diffraction pattern of the crystal form A comprises characteristic diffraction peaks at the following 2θ angles: 9.14 ± 0.2°, 12.88 ± 0.2°, 17.19 ± 0.2°, 18.31 ± 0.2°, 18.90 ± 0.2°, 19.70 ± 0.2°, 20.20 ± 0.2°, 20.60 ± 0.2°, 21.45 ± 0.2°, 21.91 ± 0.2°, and 27.61 ± 0.2°;
optionally, the X-ray powder diffraction pattern of the crystal form A comprises characteristic diffraction peaks at the following 2θ angles: 9.14 ± 0.2°, 12.88 ± 0.2°, 17.19 ± 0.2°, 18.31 ± 0.2°, 18.90 ± 0.2°, 19.70 ± 0.2°, 20.20 ± 0.2°, 20.60 ± 0.2°, 21.45 ± 0.2°, 21.91 ± 0.2°, 25.96 ± 0.2°, 26.53 ± 0.2°, 27.61 ± 0.2°, 29.22 ± 0.2°, and 30.20 ± 0.2°;
optionally, the X-ray powder diffraction pattern of the crystal form A has an X-ray powder diffraction pattern substantially as shown in FIG. 1.

**3.** A crystal form B of a maleate of a compound represented by formula (I), wherein the crystal form B has an X-ray powder diffraction pattern comprising characteristic diffraction peaks at the following 2θ angles: 8.97 ± 0.2°, 15.73 ± 0.2°, 18.31 ± 0.2°, 20.15 ± 0.2°, 21.12 ± 0.2°, and 24.70 ± 0.2°;

optionally, the X-ray powder diffraction pattern of the crystal form B comprises characteristic diffraction peaks at the following 2θ angles: 7.40 ± 0.2°, 8.97 ± 0.2°, 10.11 ± 0.2°, 13.94 ± 0.2°, 15.73 ± 0.2°, 18.31 ± 0.2°, 19.02 ± 0.2°, 20.15 ± 0.2°, 21.12 ± 0.2°, and 24.70 ± 0.2°;
optionally, the X-ray powder diffraction pattern of the crystal form B has an X-ray powder diffraction pattern substantially as shown in FIG. 6.

**4.** A fumarate of a compound represented by formula (I), whose structure is represented by formula (III),

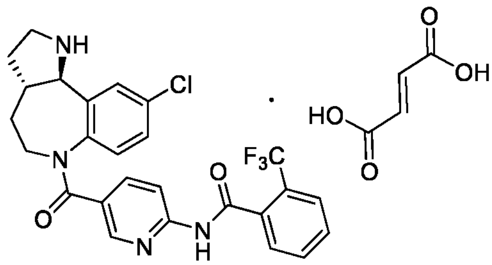

(III)

.

**5.** A crystal form C of a fumarate of a compound represented by formula (I), wherein the crystal form C has an X-ray powder diffraction pattern comprising characteristic diffraction peaks at the following 2θ angles: 12.77 ± 0.2°, 14.44 ± 0.2°, 20.00 ± 0.2°, 20.64 ± 0.2°, 21.33 ± 0.2°, and 21.87 ± 0.2°;

optionally, the X-ray powder diffraction pattern of the crystal form C comprises characteristic diffraction peaks at the following 2θ angles: 12.77 ± 0.2°, 13.17 ± 0.2°, 14.44 ± 0.2°, 17.18 ± 0.2°, 20.00 ± 0.2°, 20.64 ± 0.2°, 21.33 ± 0.2°, 21.87 ± 0.2°, 23.43 ± 0.2°, and 25.86 ± 0.2°;
optionally, the X-ray powder diffraction pattern of the crystal form C has an X-ray powder diffraction pattern substantially as shown in FIG. 9.

**6.** A hydrochloride of a compound represented by formula (IV), whose structure is represented by formula (IV),

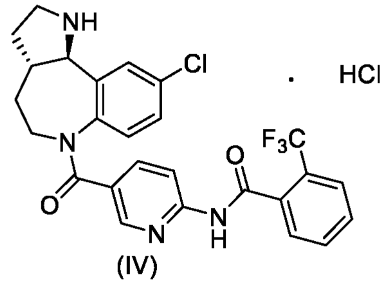

(IV)

.

7. A crystal form D of a hydrochloride of a compound represented by formula (I), wherein the crystal form D has an X-ray powder diffraction pattern comprising characteristic diffraction peaks at the following 2θ angles: 8.13 ± 0.2°, 9.27 ± 0.2°, 9.91 ± 0.2°, 13.53 ± 0.2°, 16.37 ± 0.2°, and 17.09 ± 0.2°;

optionally, the X-ray powder diffraction pattern of the crystal form D comprises characteristic diffraction peaks at the following 2θ angles: 8.13 ± 0.2°, 9.27 ± 0.2°, 9.91 ± 0.2°, 12.86 ± 0.2°, 13.53 ± 0.2°, 16.37 ± 0.2°, 17.09 ± 0.2°, 18.67 ± 0.2°, 21.77 ± 0.2°, and 23.81 ± 0.2°;
optionally, the X-ray powder diffraction pattern of the crystal form D has an X-ray powder diffraction pattern substantially as shown in FIG. 12.

8. A crystal form E of a hydrochloride of a compound represented by formula (I), wherein the crystal form E has an X-ray powder diffraction pattern comprising characteristic diffraction peaks at the following 2θ angles: 3.86 ± 0.2°, 13.60 ± 0.2°, 14.19 ± 0.2°, 18.06 ± 0.2°, 20.50 ± 0.2°, and 21.24 ± 0.2°;

optionally, the X-ray powder diffraction pattern of the crystal form E comprises characteristic diffraction peaks at the following 2θ angles: 3.86 ± 0.2°, 6.74 ± 0.2°, 11.73 ± 0.2°, 13.60 ± 0.2°, 14.19 ± 0.2°, 18.06 ± 0.2°, 20.50 ± 0.2°, 21.24 ± 0.2°, 23.72 ± 0.2°, and 24.06 ± 0.2°;
optionally, the X-ray powder diffraction pattern of the crystal form E has an X-ray powder diffraction pattern substantially as shown in FIG. 15.

9. A crystal form F of a hydrochloride of a compound represented by formula (I), wherein the crystal form F has an X-ray powder diffraction pattern comprising characteristic diffraction peaks at the following 2θ angles: 5.84 ± 0.2°, 11.77 ± 0.2°, 13.29 ± 0.2°, 17.82 ± 0.2°, 20.49 ± 0.2°, and 20.94 ± 0.2°;

optionally, the X-ray powder diffraction pattern of the crystal form F comprises characteristic diffraction peaks at the following 2θ angles: 5.84 ± 0.2°, 11.77 ± 0.2°, 13.29 ± 0.2°, 14.34 ± 0.2°, 17.82 ± 0.2°, 18.67 ± 0.2°, 20.49 ± 0.2°, 20.94 ± 0.2°, 23.02 ± 0.2°, and 23.68 ± 0.2°;
optionally, the X-ray powder diffraction pattern of the crystal form F has an X-ray powder diffraction pattern substantially as shown in FIG. 18.

10. A sulfate of a compound represented by formula (I), whose structure is represented by formula (V),

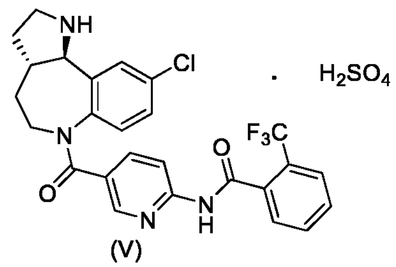

(V)

.

11. A crystal form G of a sulfate of a compound represented by formula (I), wherein the crystal form G has an X-ray powder diffraction pattern comprising characteristic diffraction peaks at the following 2θ angles: 10.30 ± 0.2°, 13.02 ± 0.2°, 16.60 ± 0.2°, 18.53 ± 0.2°, 20.67 ± 0.2°, and 22.26 ± 0.2°;

optionally, the X-ray powder diffraction pattern of the crystal form G comprises characteristic diffraction peaks at

the following 2θ angles: 6.46 ± 0.2°, 10.30 ± 0.2°, 13.02 ± 0.2°, 16.60 ± 0.2°, 17.66 ± 0.2°, 18.53 ± 0.2°, 19.98 ± 0.2°, 20.67 ± 0.2°, 22.26 ± 0.2°, and 23.62 ± 0.2°;
optionally, the X-ray powder diffraction pattern of the crystal form G has an X-ray powder diffraction pattern substantially as shown in FIG. 21.

**12.** A succinate of a compound represented by formula (I), whose structure is represented by formula (VI),

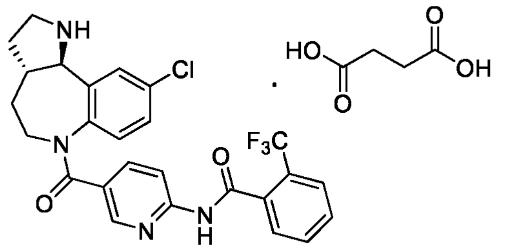

(VI)

.

**13.** A crystal form H of a succinate of a compound represented by formula (I), wherein the crystal form H has an X-ray powder diffraction pattern comprising characteristic diffraction peaks at the following 2θ angles: 10.30 ± 0.2°, 14.63 ± 0.2°, 18.59 ± 0.2°, 20.13 ± 0.2°, 21.83 ± 0.2°, and 22.30 ± 0.2°;

optionally, the X-ray powder diffraction pattern of the crystal form H comprises characteristic diffraction peaks at the following 2θ angles: 10.30 ± 0.2°, 12.91 ± 0.2°, 14.63 ± 0.2°, 18.59 ± 0.2°, 19.41 ± 0.2°, 20.13 ± 0.2°, 20.69 ± 0.2°, 21.83 ± 0.2°, 22.30 ± 0.2°, and 23.65 ± 0.2°;
optionally, the X-ray powder diffraction pattern of the crystal form H has an X-ray powder diffraction pattern substantially as shown in FIG. 24.

**14.** A crystal form J of a succinate of a compound represented by formula (I), wherein the crystal form J has an X-ray powder diffraction pattern comprising characteristic diffraction peaks at the following 2θ angles: 9.61 ± 0.2°, 11.56 ± 0.2°, 12.93 ± 0.2°, 17.12 ± 0.2°, 17.71 ± 0.2°, and 19.95 ± 0.2°;

optionally, the X-ray powder diffraction pattern of the crystal form J comprises characteristic diffraction peaks at the following 2θ angles: 9.61 ± 0.2°, 11.56 ± 0.2°, 12.93 ± 0.2°, 13.76 ± 0.2°, 17.12 ± 0.2°, 17.71 ± 0.2°, 19.51 ± 0.2°, 19.95 ± 0.2°, 21.83 ± 0.2°, and 22.42 ± 0.2°;
optionally, the X-ray powder diffraction pattern of the crystal form J has an X-ray powder diffraction pattern substantially as shown in FIG. 27.

**15.** A glycollate of a compound represented by formula (I), whose structure is represented by formula (VII),

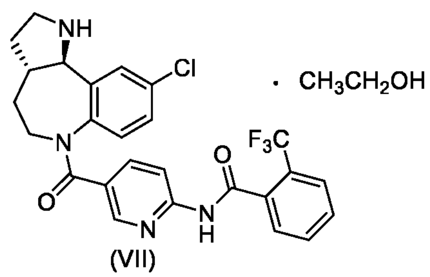

(VII)

.

**16.** A crystal form K of a glycollate of a compound represented by formula (I), wherein the crystal form K has an X-ray powder diffraction pattern comprising characteristic diffraction peaks at the following 2θ angles: 12.51 ± 0.2°, 15.99 ± 0.2°, 18.71 ± 0.2°, 20.18 ± 0.2°, 20.59 ± 0.2°, and 21.64 ± 0.2°;

optionally, the X-ray powder diffraction pattern of the crystal form K comprises characteristic diffraction peaks at the following 2θ angles: 12.51 ± 0.2°, 13.62 ± 0.2°, 15.99 ± 0.2°, 16.65 ± 0.2°, 18.71 ± 0.2°, 20.18 ± 0.2°, 20.59 ± 0.2°, 21.64 ± 0.2°, 22.62 ± 0.2°, and 24.53 ± 0.2°;
optionally, the X-ray powder diffraction pattern of the crystal form K has an X-ray powder diffraction pattern

substantially as shown in FIG. 30.

**17.** A benzoate of a compound represented by formula (I), whose structure is represented by formula (VIII),

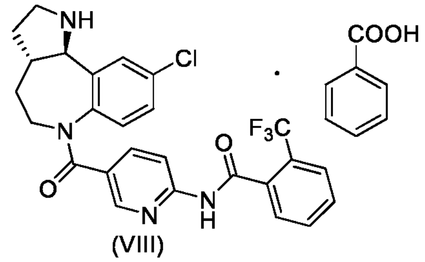

(VIII)

.

**18.** A eutectic crystal form M of a benzoate of a compound represented by formula (I), wherein the crystal form M has an X-ray powder diffraction pattern comprising characteristic diffraction peaks at the following 2θ angles: 9.31 ± 0.2°, 13.77 ± 0.2°, 14.54 ± 0.2°, 19.84 ± 0.2°, 20.34 ± 0.2°, and 21.70 ± 0.2°;

optionally, the X-ray powder diffraction pattern of the crystal form M comprises characteristic diffraction peaks at the following 2θ angles: 9.31 ± 0.2°, 13.77 ± 0.2°, 14.54 ± 0.2°, 16.55 ± 0.2°, 17.66 ± 0.2°, 18.68 ± 0.2°, 19.84 ± 0.2°, 20.34 ± 0.2°, 21.70 ± 0.2°, and 23.32 ± 0.2°;
optionally, the X-ray powder diffraction pattern of the crystal form M has an X-ray powder diffraction pattern substantially as shown in FIG. 36.

**19.** A crystal form N of a compound represented by formula (I), wherein the crystal form N has an X-ray powder diffraction pattern comprising characteristic diffraction peaks at the following 2θ angles: 10.38 ± 0.2°, 13.54 ± 0.2°, 14.41 ± 0.2°, 16.32 ± 0.2°, 18.10 ± 0.2°, and 19.05 ± 0.2°;

optionally, the X-ray powder diffraction pattern of the crystal form N comprises characteristic diffraction peaks at the following 2θ angles: 10.38 ± 0.2°, 13.54 ± 0.2°, 14.41 ± 0.2°, 15.90 ± 0.2°, 16.32 ± 0.2°, 18.10 ± 0.2°, 19.05 ± 0.2°, 22.14 ± 0.2°, 22.91 ± 0.2°, and 23.66 ± 0.2°;
optionally, the X-ray powder diffraction pattern of the crystal form N has an X-ray powder diffraction pattern substantially as shown in FIG. 39.

**20.** A crystal form O of a compound represented by formula (I), wherein the crystal form O has an X-ray powder diffraction pattern comprising characteristic diffraction peaks at the following 2θ angles: 4.75 ± 0.2°, 9.65 ± 0.2°, 15.70 ± 0.2°, 16.88 ± 0.2°, 18.00 ± 0.2°, and 18.97 ± 0.2°;

optionally, the X-ray powder diffraction pattern of the crystal form O comprises characteristic diffraction peaks at the following 2θ angles: 4.75 ± 0.2°, 9.65 ± 0.2°, 15.70 ± 0.2°, 16.88 ± 0.2°, 18.00 ± 0.2°, 18.97 ± 0.2°, 19.89 ± 0.2°, 21.86 ± 0.2°, 22.67 ± 0.2°, and 24.30 + 0.2°;
optionally, the X-ray powder diffraction pattern of the crystal form O has an X-ray powder diffraction pattern substantially as shown in FIG. 42.

**21.** A crystal form P of a compound represented by formula (I), wherein the crystal form P has an X-ray powder diffraction pattern comprising characteristic diffraction peaks at the following 2θ angles: 13.07 ± 0.2°, 17.98 ± 0.2°, 21.64 ± 0.2°, 23.78 ± 0.2°, 26.36 ± 0.2°, and 33.13 ± 0.2°;
optionally, the X-ray powder diffraction pattern of the crystal form P has an X-ray powder diffraction pattern substantially as shown in FIG. 45.

**22.** A crystal form Q of a compound represented by formula (I), wherein the crystal form Q has an X-ray powder diffraction pattern comprising characteristic diffraction peaks at the following 2θ angles: 3.48 ± 0.2°, 10.60 ± 0.2°, 12.32 ± 0.2°, 15.41 ± 0.2°, 16.60 ± 0.2°, and 17.09 ± 0.2°;

optionally, the X-ray powder diffraction pattern of the crystal form Q comprises characteristic diffraction peaks at the following 2θ angles: 3.48 ± 0.2°, 10.60 ± 0.2°, 12.32 ± 0.2°, 15.41 ± 0.2°, 16.60 ± 0.2°, 17.09 ± 0.2°, 17.75 ± 0.2°, 18.79 ± 0.2°, 20.49 ± 0.2°, and 21.40 ± 0.2°;
optionally, the X-ray powder diffraction pattern of the crystal form Q has an X-ray powder diffraction pattern

substantially as shown in FIG. 48.

**23.** A crystal form R of a compound represented by formula (I), wherein the crystal form R has an X-ray powder diffraction pattern comprising characteristic diffraction peaks at the following 2θ angles: 6.70 ± 0.2°, 13.30 ± 0.2°, 18.15 ± 0.2°, 21.39 ± 0.2°, 22.97 ± 0.2°, and 26.71 ± 0.2°;
optionally, the X-ray powder diffraction pattern of the crystal form R has an X-ray powder diffraction pattern substantially as shown in FIG. 50.

**24.** A crystal form S of a compound represented by formula (I), wherein the crystal form S has an X-ray powder diffraction pattern comprising characteristic diffraction peaks at the following 2θ angles: 14.97 ± 0.2°, 15.34 ± 0.2°, 17.97 ± 0.2°, 22.81 ± 0.2°, 23.54 ± 0.2°, and 24.69 ± 0.2°;
optionally, the X-ray powder diffraction pattern of the crystal form S has an X-ray powder diffraction pattern substantially as shown in FIG. 53.

**25.** A crystal form T of a compound represented by formula (I), wherein the crystal form T has an X-ray powder diffraction pattern comprising characteristic diffraction peaks at the following 2θ angles: 15.84 ± 0.2°, 17.03 ± 0.2°, 17.60 ± 0.2°, 20.01 ± 0.2°, 22.22 ± 0.2°, and 22.82 ± 0.2°;

optionally, the X-ray powder diffraction pattern of the crystal form T comprises characteristic diffraction peaks at the following 2θ angles: 13.64 ± 0.2°, 14.70 ± 0.2°, 15.84 ± 0.2°, 17.03 ± 0.2°, 17.60 ± 0.2°, 19.01 ± 0.2°, 20.01 ± 0.2°, 22.22 ± 0.2°, 22.82 ± 0.2°, and 24.45 ± 0.2°;
optionally, the X-ray powder diffraction pattern of the crystal form T has an X-ray powder diffraction pattern substantially as shown in FIG. 56.

**26.** A crystal form U of a compound represented by formula (I), wherein the crystal form U has an X-ray powder diffraction pattern comprising characteristic diffraction peaks at the following 2θ angles: 8.01 ± 0.2°, 9.27 ± 0.2°, 12.68 ± 0.2°, 16.15 ± 0.2°, 17.94 ± 0.2°, and 19.31 ± 0.2°;

optionally, the X-ray powder diffraction pattern of the crystal form U comprises characteristic diffraction peaks at the following 2θ angles: 8.01 ± 0.2°, 9.27 ± 0.2°, 12.68 ± 0.2°, 16.15 ± 0.2°, 17.94 ± 0.2°, 19.31 ± 0.2°, 22.16 ± 0.2°, 22.82 ± 0.2°, 23.80 ± 0.2°, and 24.08 ± 0.2°;
optionally, the X-ray powder diffraction pattern of the crystal form U has an X-ray powder diffraction pattern substantially as shown in FIG. 59.

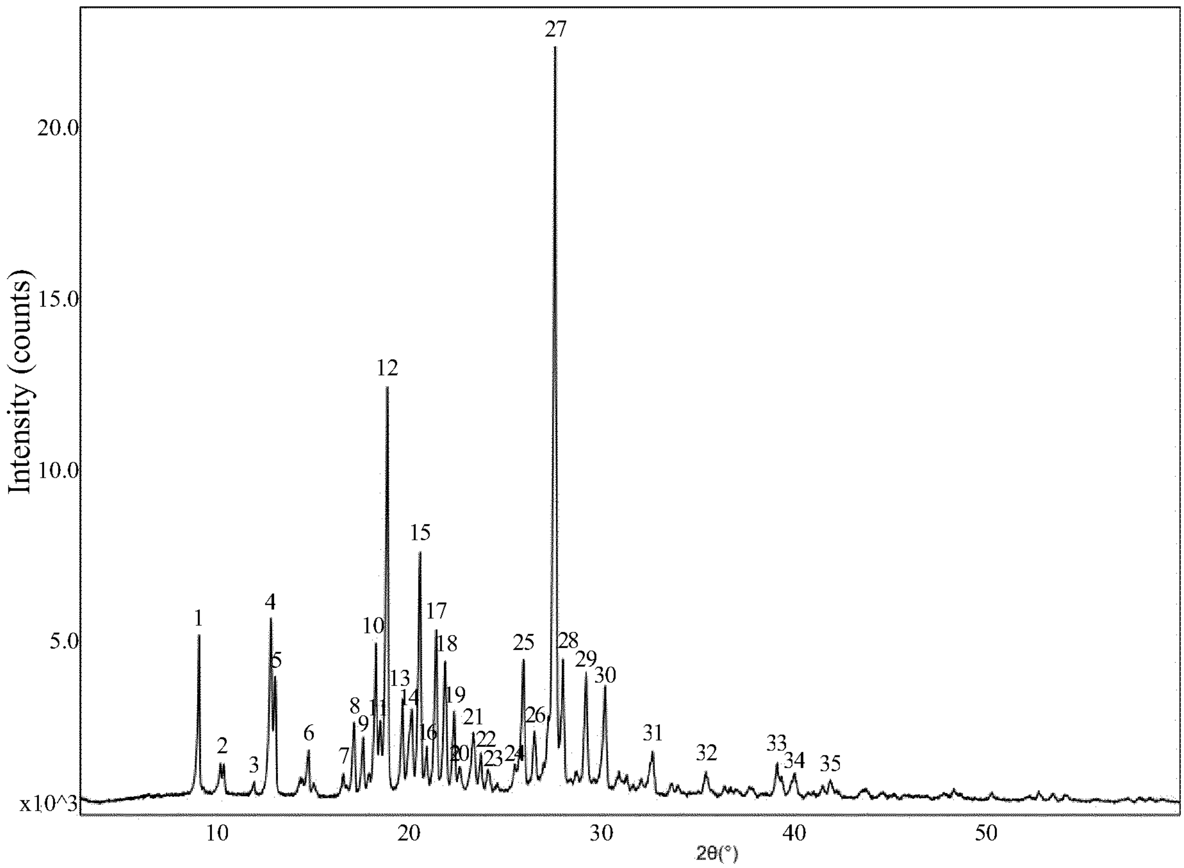


FIG. 1

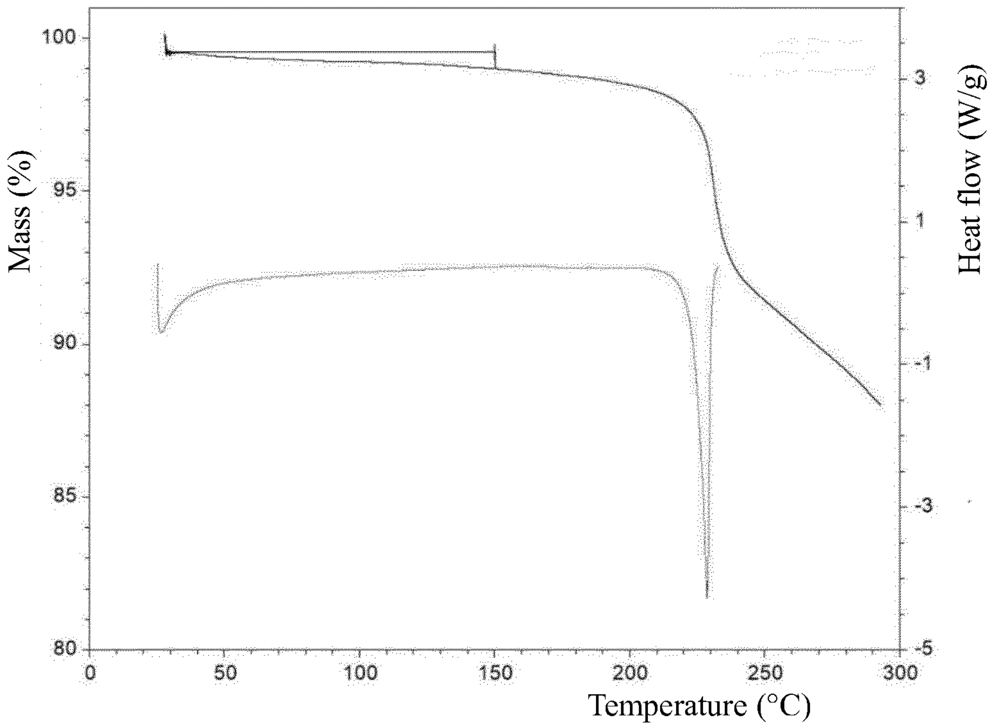


FIG. 2

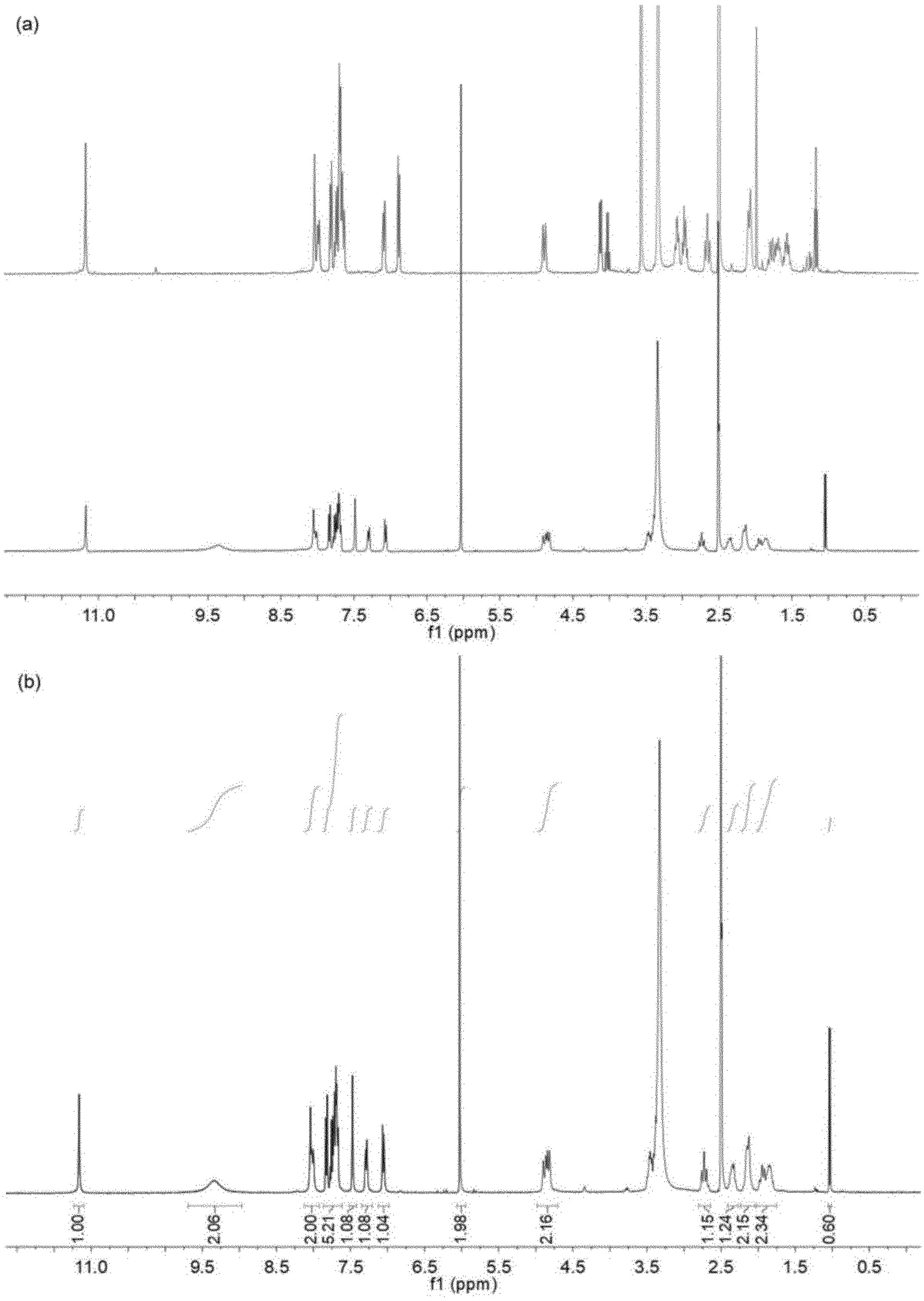
(a)
11.0
9.5
8.5
7.5
6.5
5.5
4.5
3.5
2.5
1.5
0.5
f1 (ppm)
(b)
1.00
2.06
2.00
5.21
1.08
1.08
1.04
1.98
2.16
1.15
1.24
2.15
2.34
0.60
11.0
9.5
8.5
7.5
6.5
5.5
4.5
3.5
2.5
1.5
0.5
f1 (ppm)

FIG. 3

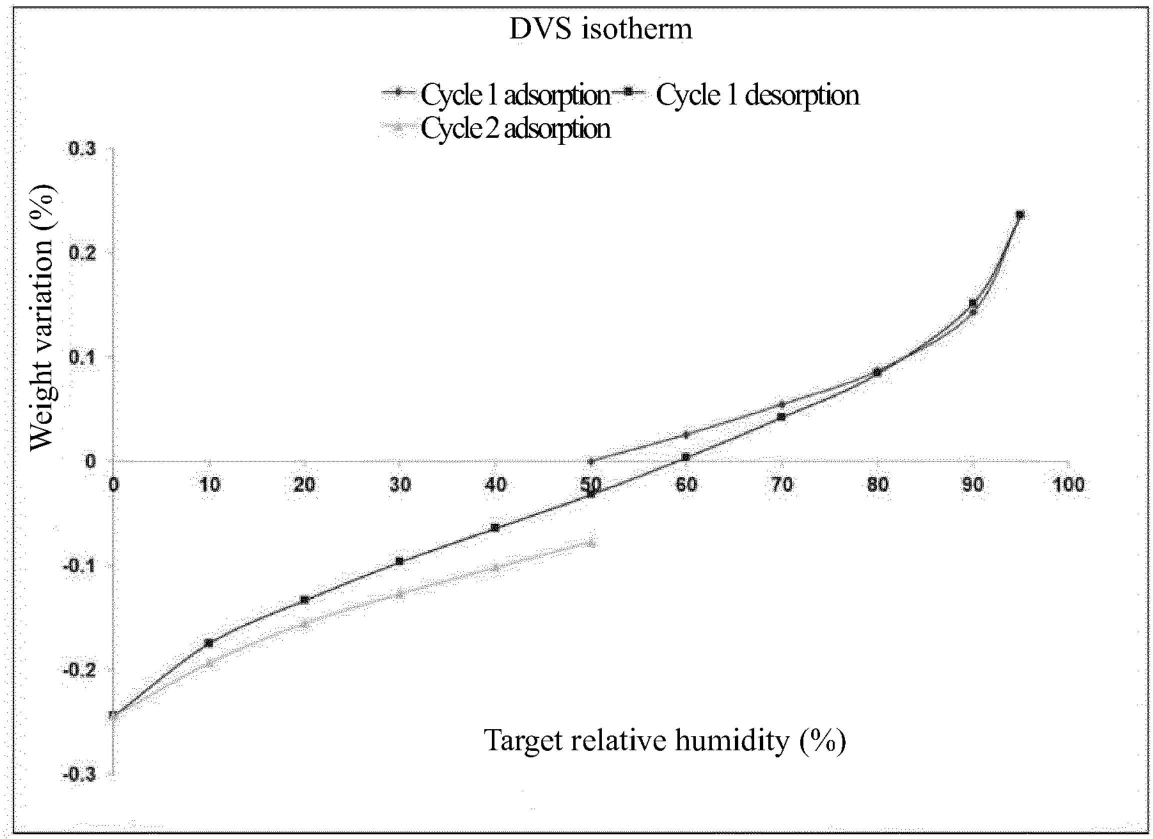
DVS isotherm
Cycle 1 adsorption
Cycle 1 desorption
Cycle 2 adsorption
Weight variation (%)
0.3
0.2
0.1
0
-0.1
-0.2
-0.3
0
10
20
30
40
50
60
70
80
90
100
Target relative humidity (%)

FIG. 4

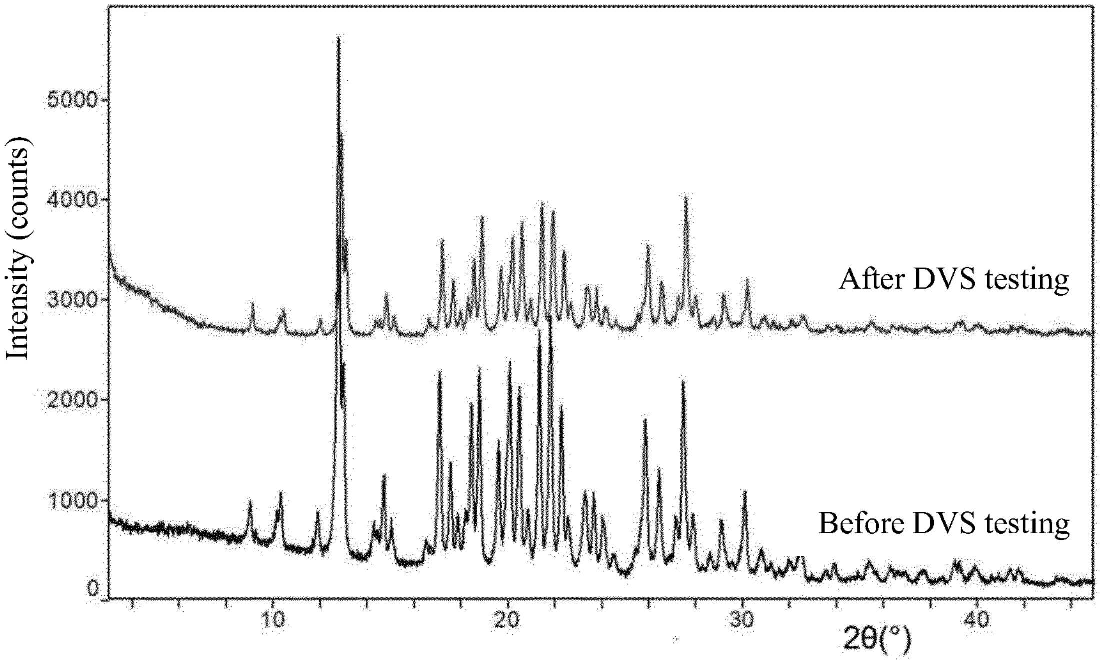
Intensity (counts)
After DVS testing
Before DVS testing
2θ(°)

FIG. 5

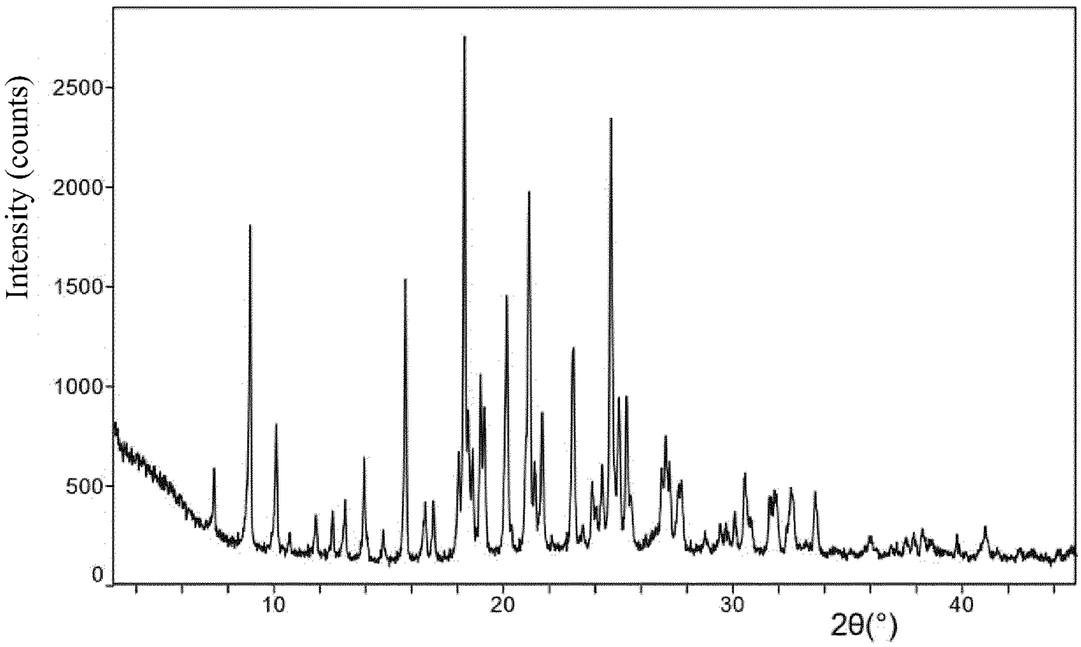
Intensity (counts)
2θ(°)

FIG. 6

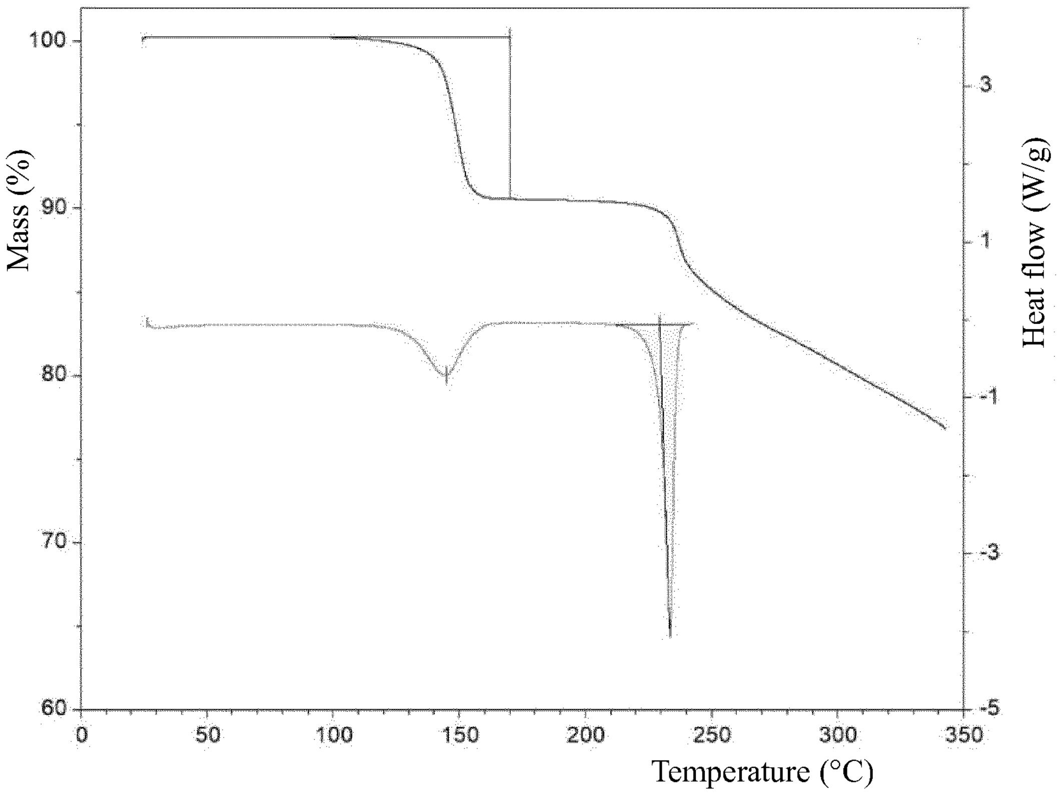
Mass (%)
Heat flow (W/g)
Temperature (°C)
100
90
80
70
60
3
1
-1
-3
-5
0
50
100
150
200
250
300
350

FIG. 7

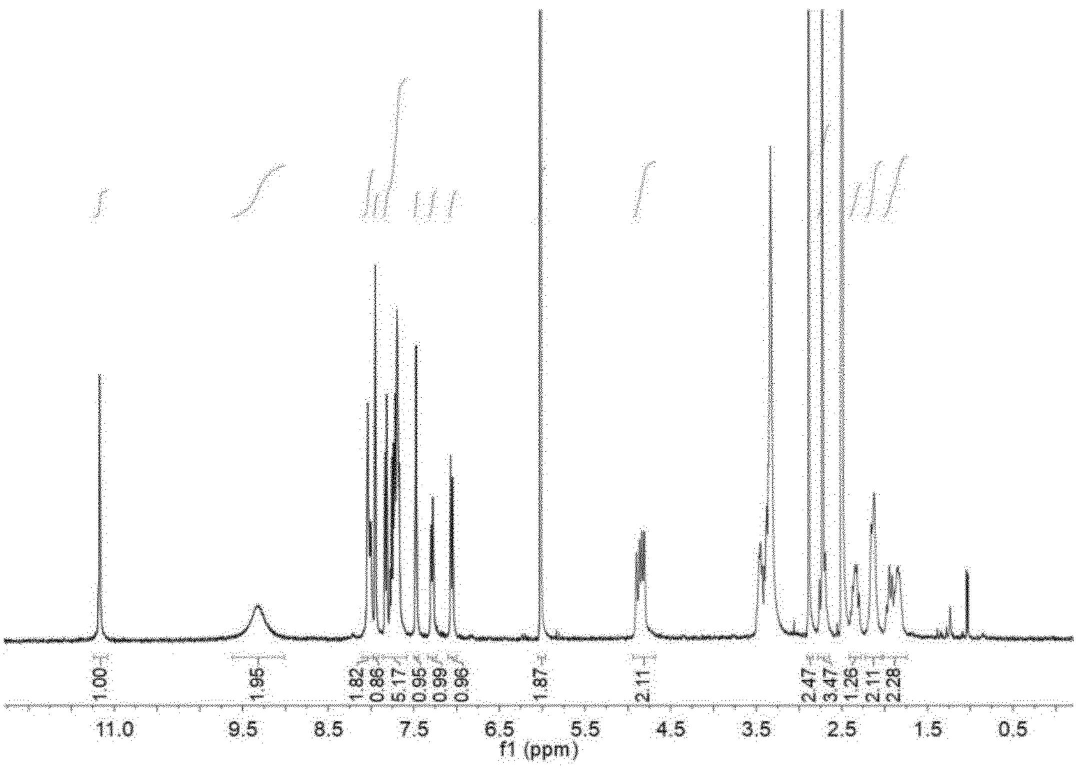
1.00
1.95
1.82
0.86
5.17
0.95
0.99
0.96
1.87
2.11
2.47
3.47
1.26
2.11
2.28
11.0
9.5
8.5
7.5
6.5
5.5
4.5
3.5
2.5
1.5
0.5
f1 (ppm)

FIG. 8

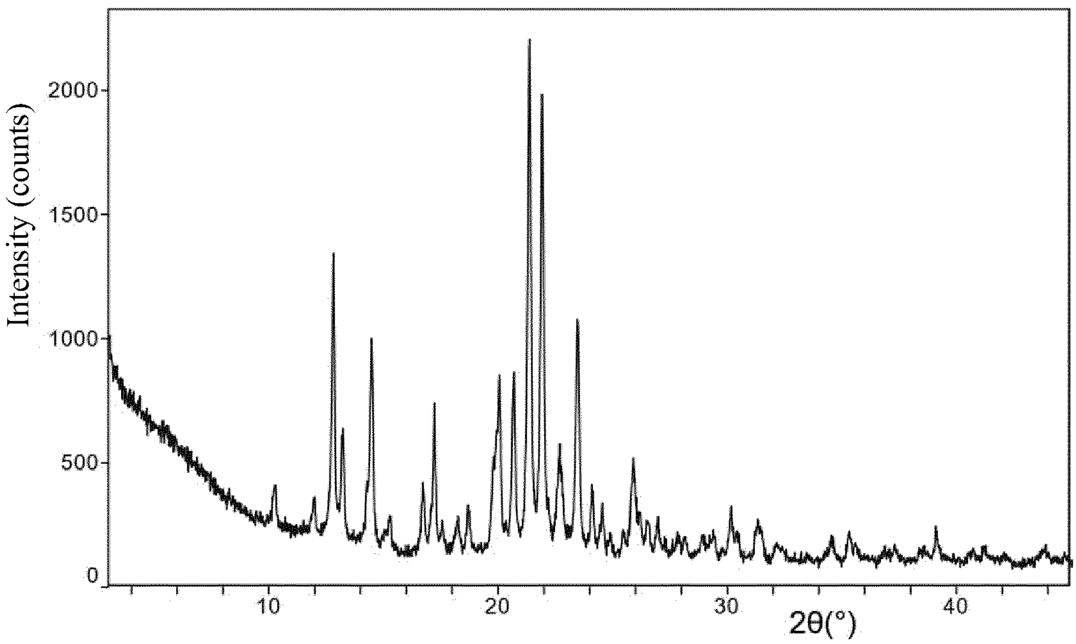
Intensity (counts)
2000
1500
1000
500
0
10
20
30
40
2θ(°)

FIG. 9

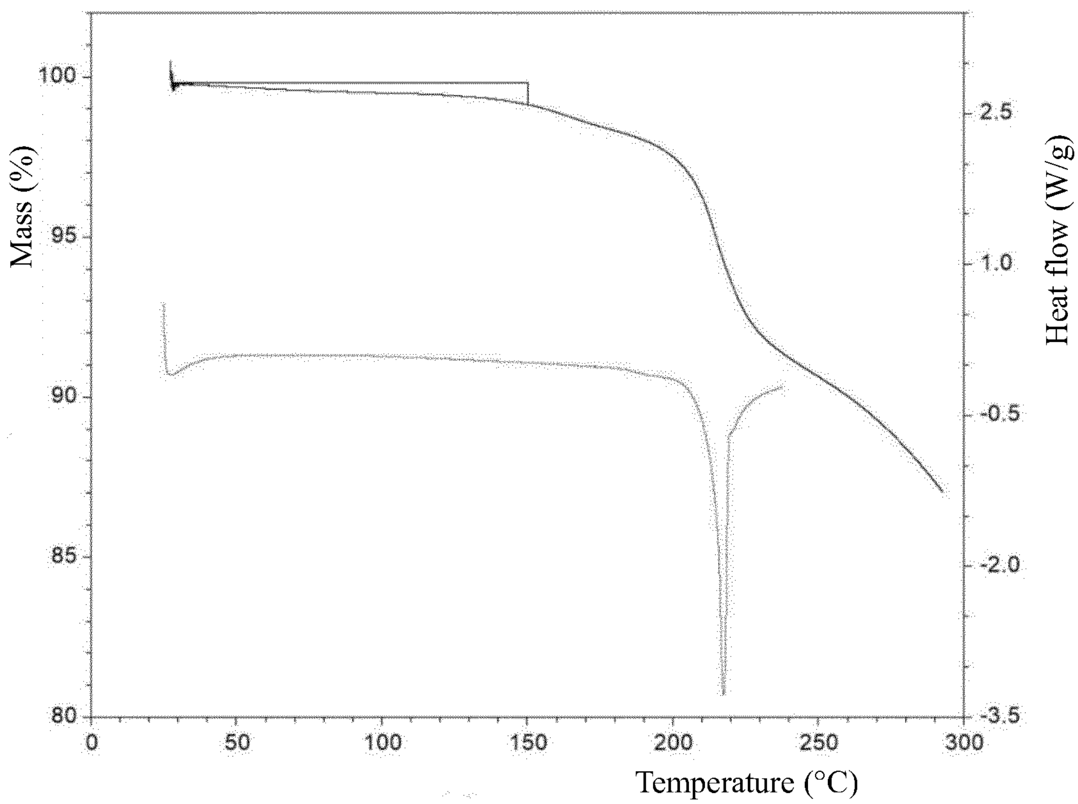
Mass (%)
100
95
90
85
80
Heat flow (W/g)
2.5
1.0
-0.5
-2.0
-3.5
0
50
100
150
200
250
300
Temperature (°C)

FIG. 10

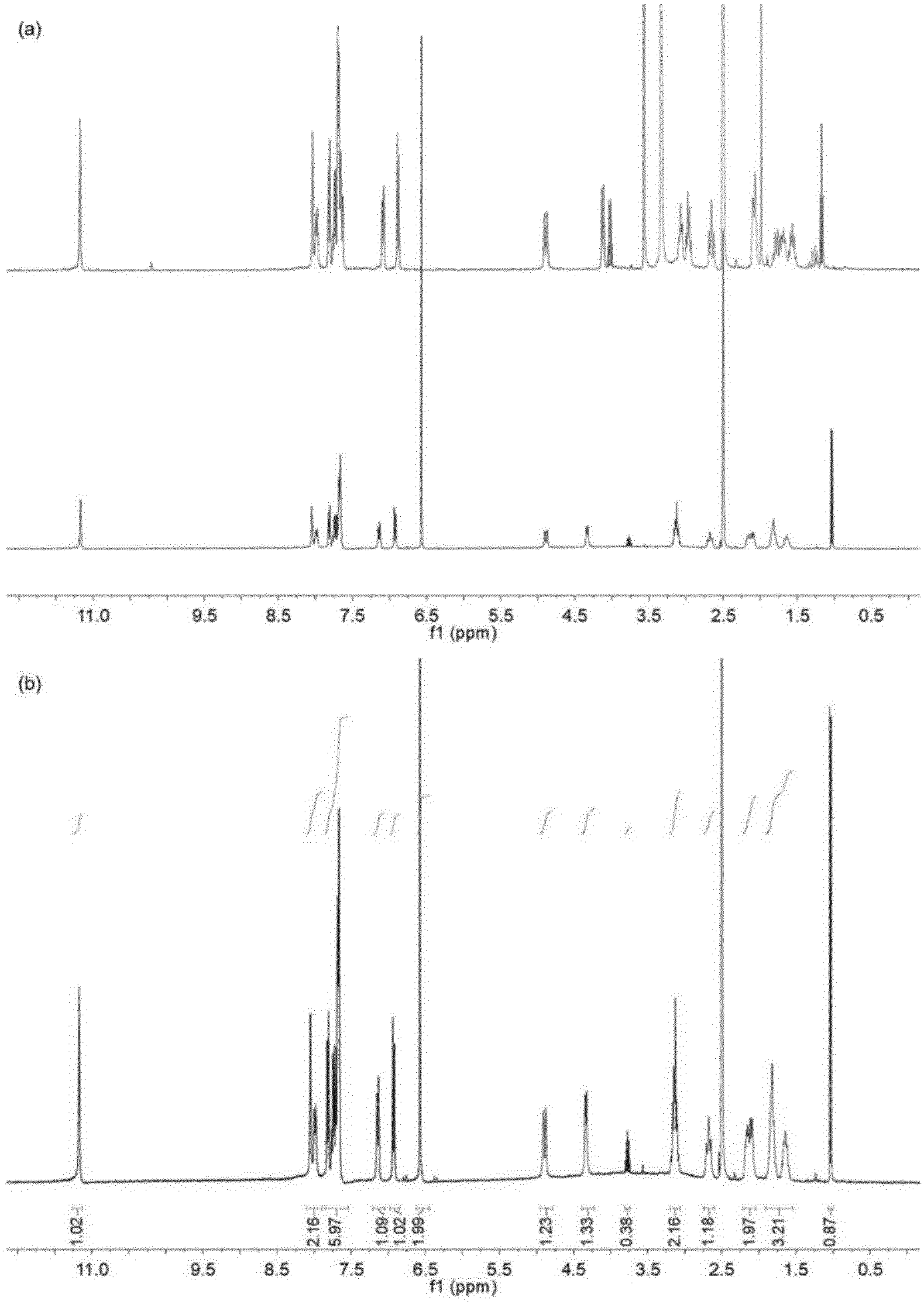
(a)
11.0
9.5
8.5
7.5
6.5
5.5
4.5
3.5
2.5
1.5
0.5
f1 (ppm)
(b)
1.02
2.16
5.97
1.09
1.02
1.99
1.23
1.33
0.38
2.16
1.18
1.97
3.21
0.87
11.0
9.5
8.5
7.5
6.5
5.5
4.5
3.5
2.5
1.5
0.5
f1 (ppm)

FIG. 11

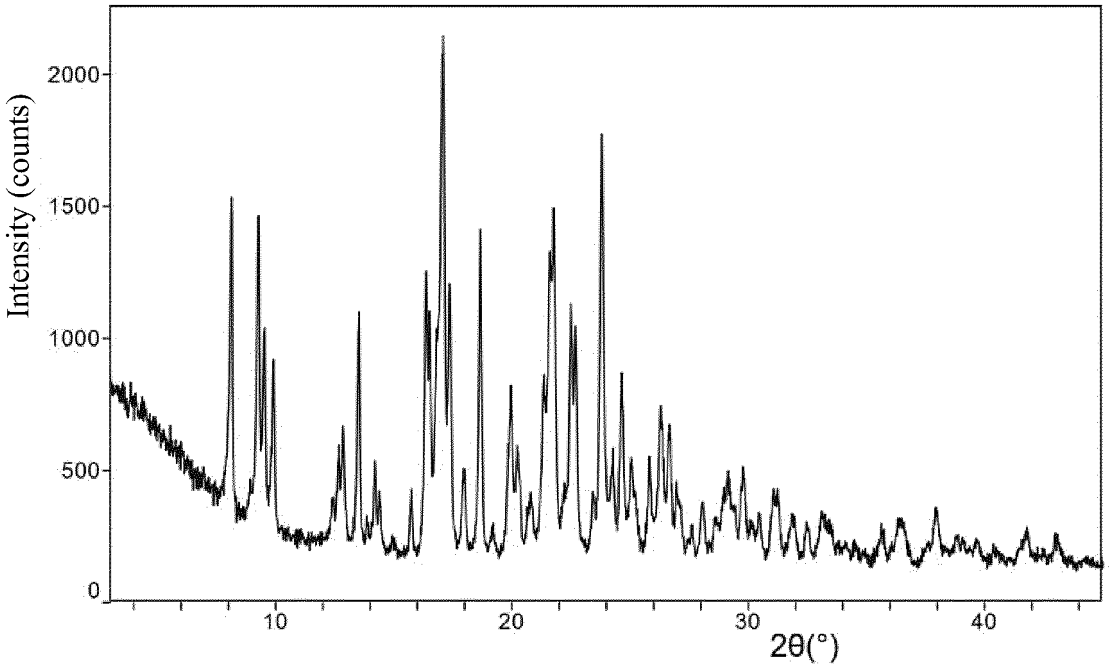


FIG. 12

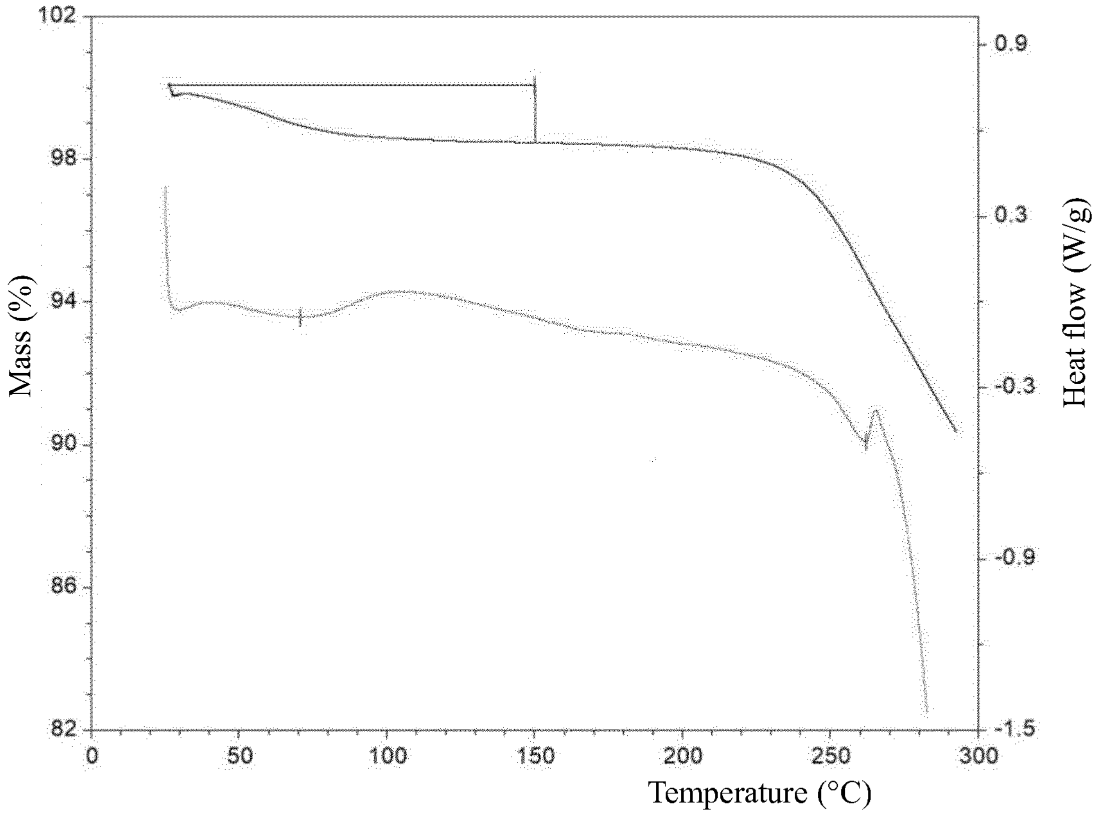
102
98
94
90
86
82
0
50
100
150
200
250
300
0.9
0.3
-0.3
-0.9
-1.5
Mass (%)
Heat flow (W/g)
Temperature (°C)

FIG. 13

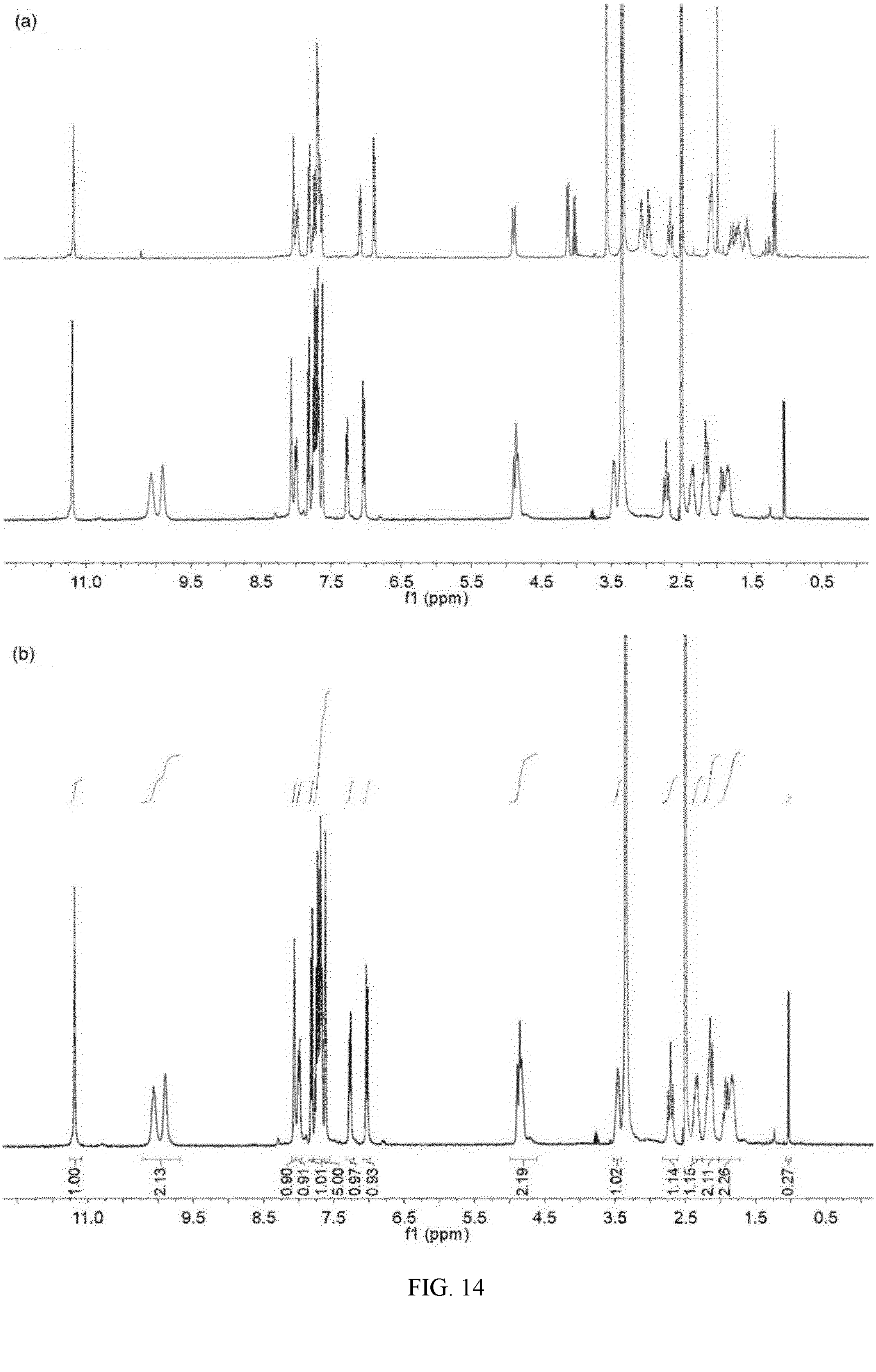
(a)
11.0
9.5
8.5
7.5
6.5
5.5
4.5
3.5
2.5
1.5
0.5
f1 (ppm)
(b)
1.00
2.13
0.90
0.91
1.01
5.00
0.97
0.93
2.19
1.02
1.14
1.15
2.11
2.26
0.27
11.0
9.5
8.5
7.5
6.5
5.5
4.5
3.5
2.5
1.5
0.5
f1 (ppm)

FIG. 14

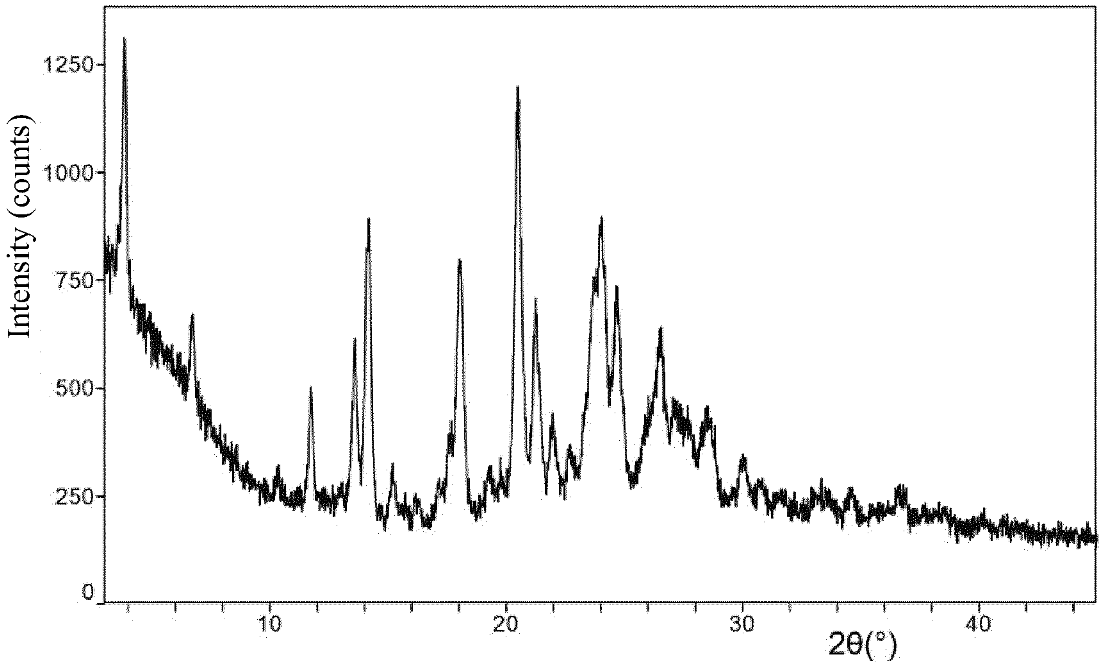
1250
1000
750
500
250
0
Intensity (counts)
10
20
30
40
2θ(°)

FIG. 15

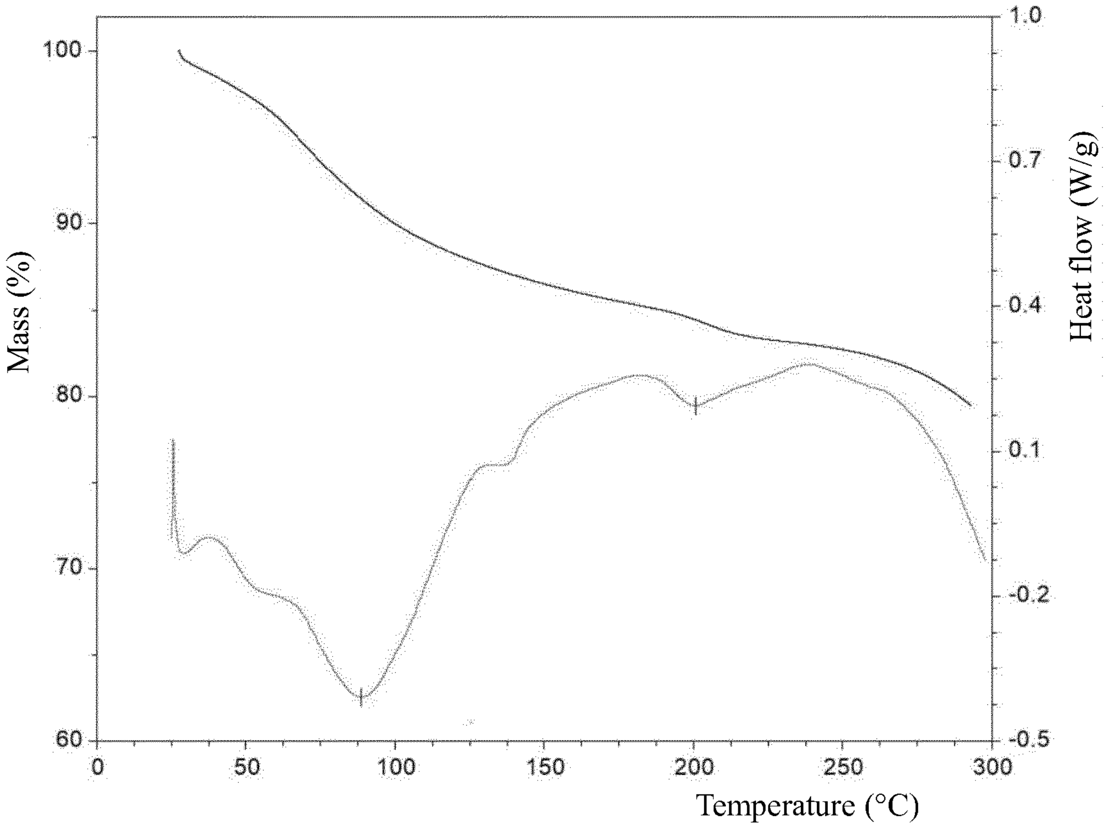
100
90
80
70
60
Mass (%)
1.0
0.7
0.4
0.1
-0.2
-0.5
Heat flow (W/g)
0
50
100
150
200
250
300
Temperature (°C)

FIG. 16

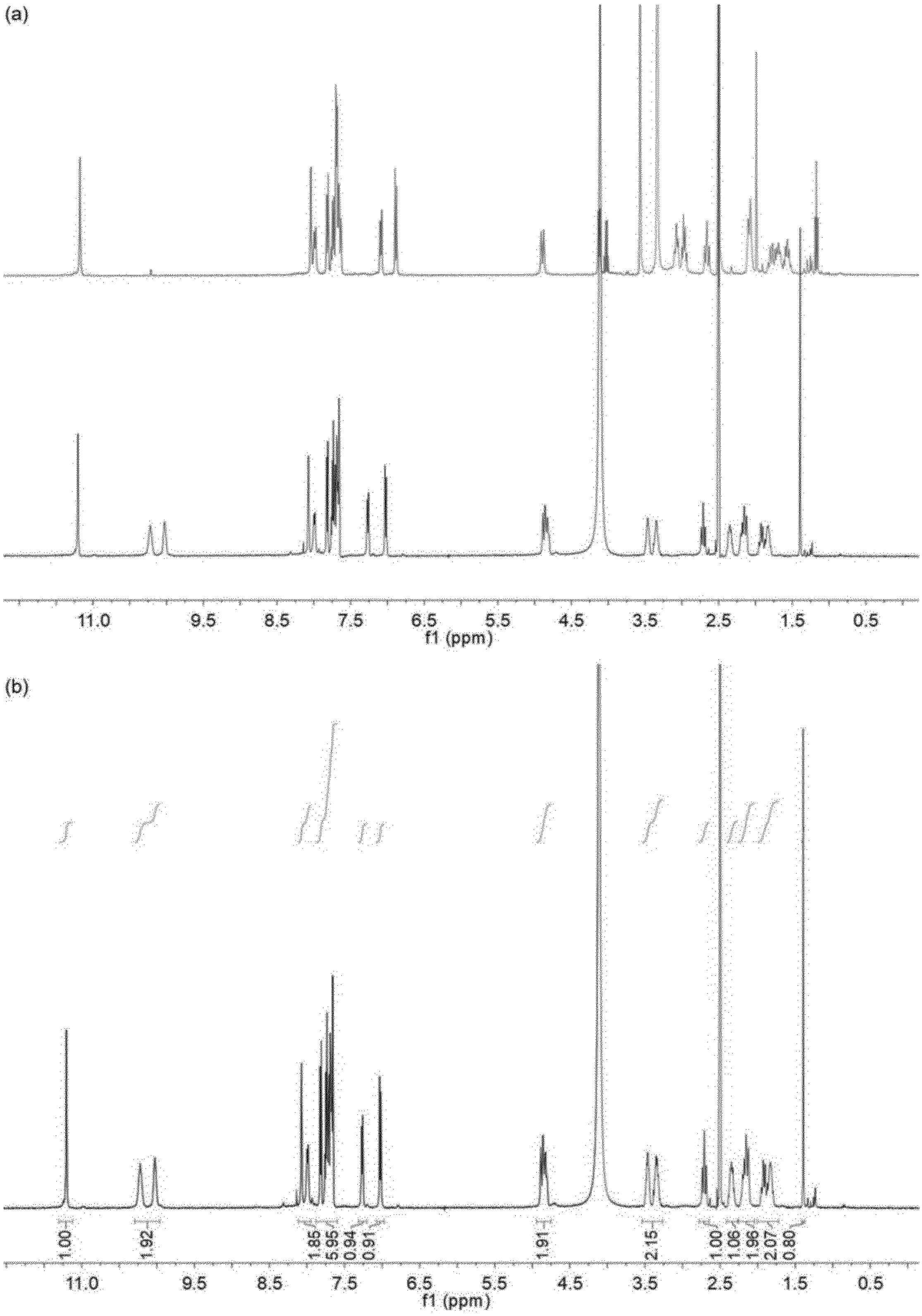
(a)
11.0
9.5
8.5
7.5
6.5
5.5
4.5
3.5
2.5
1.5
0.5
f1 (ppm)
(b)
1.00
1.92
1.85
5.95
0.94
0.91
1.91
2.15
1.00
1.06
1.96
2.07
0.80
11.0
9.5
8.5
7.5
6.5
5.5
4.5
3.5
2.5
1.5
0.5
f1 (ppm)

FIG. 17

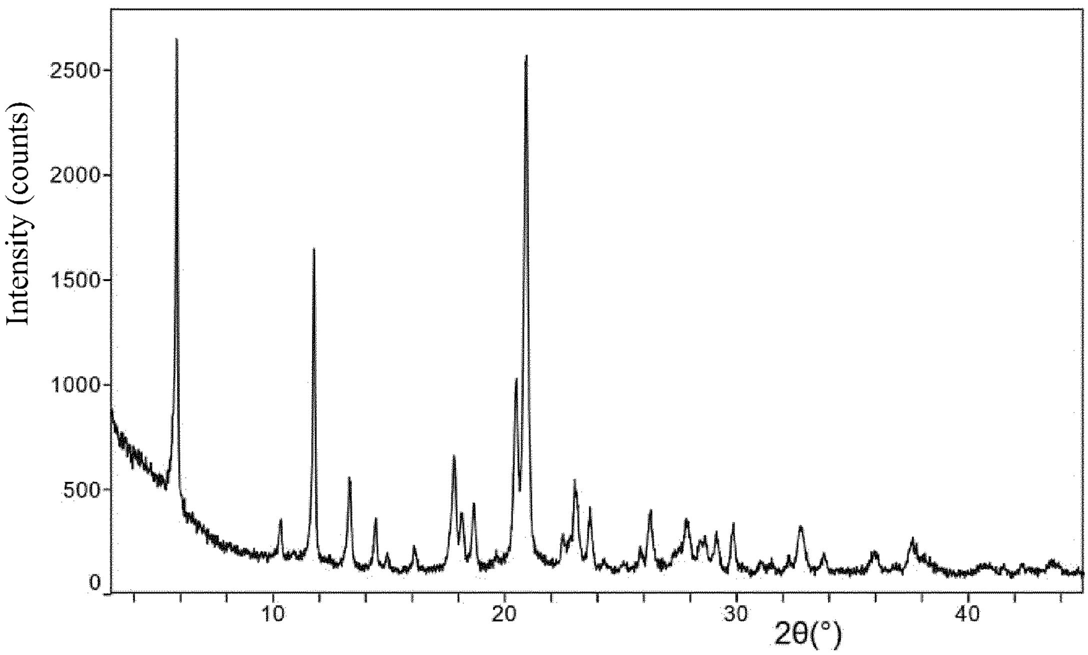
Intensity (counts)
2500
2000
1500
1000
500
0
10
20
30
40
2θ(°)

FIG. 18

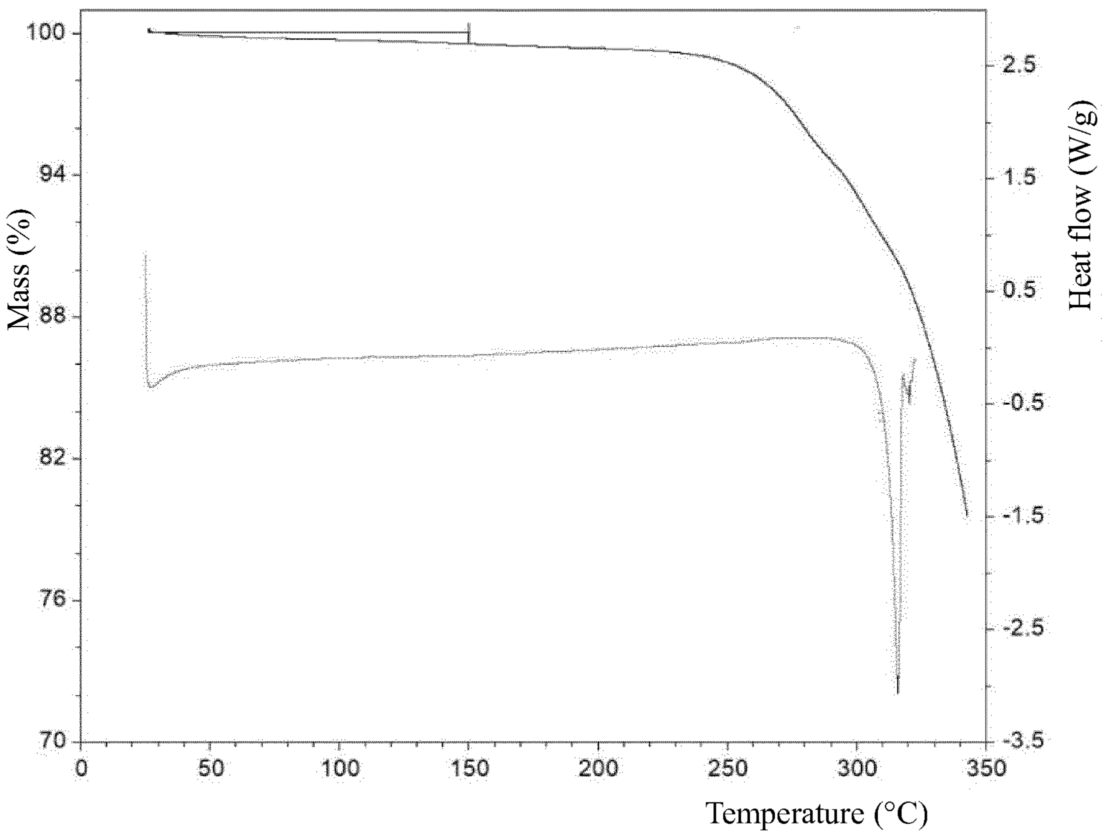
Mass (%)
100
94
88
82
76
70
Heat flow (W/g)
2.5
1.5
0.5
-0.5
-1.5
-2.5
-3.5
0
50
100
150
200
250
300
350
Temperature (°C)

FIG. 19

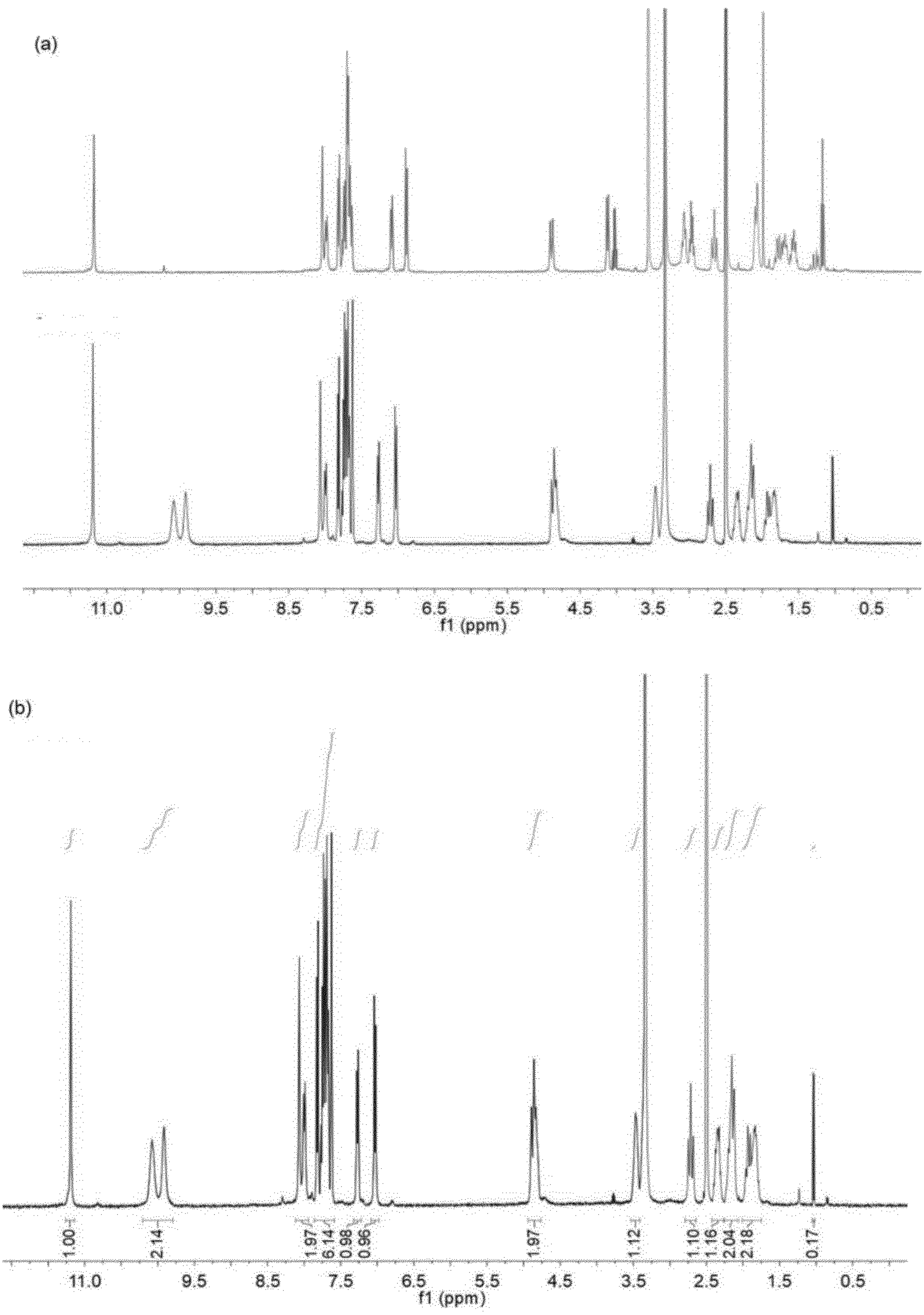
(a)
11.0
9.5
8.5
7.5
6.5
5.5
4.5
3.5
2.5
1.5
0.5
f1 (ppm)
(b)
1.00
2.14
1.97
6.14
0.98
0.96
1.97
1.12
1.10
1.16
2.04
2.18
0.17
11.0
9.5
8.5
7.5
6.5
5.5
4.5
3.5
2.5
1.5
0.5
f1 (ppm)

FIG. 20

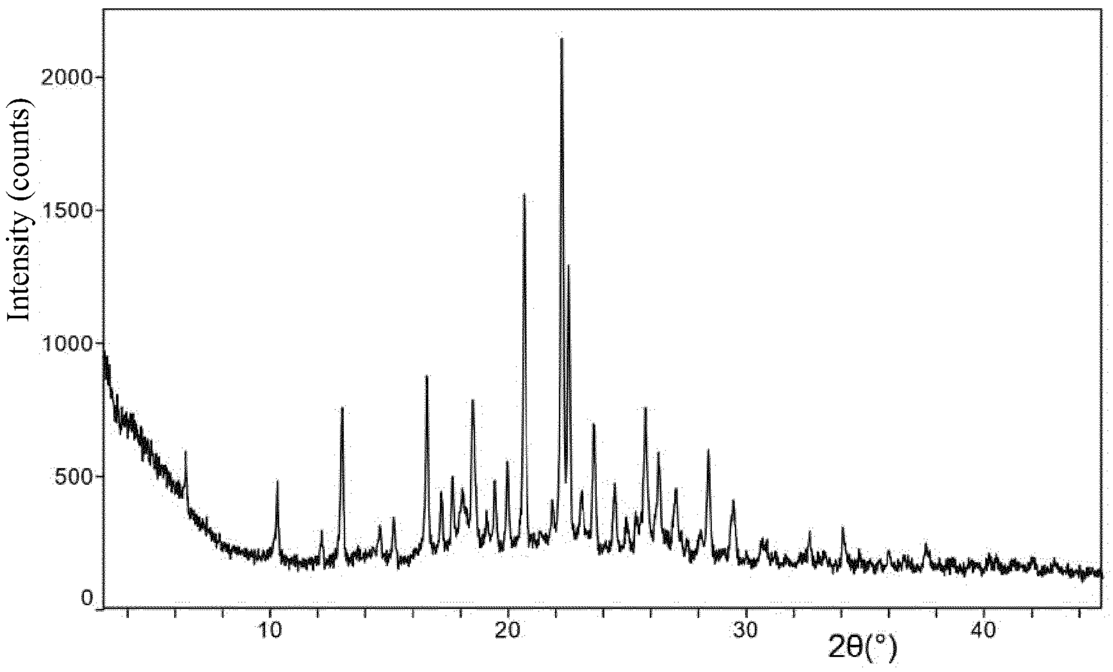
Intensity (counts)
2000
1500
1000
500
0
10
20
30
40
2θ(°)

FIG. 21

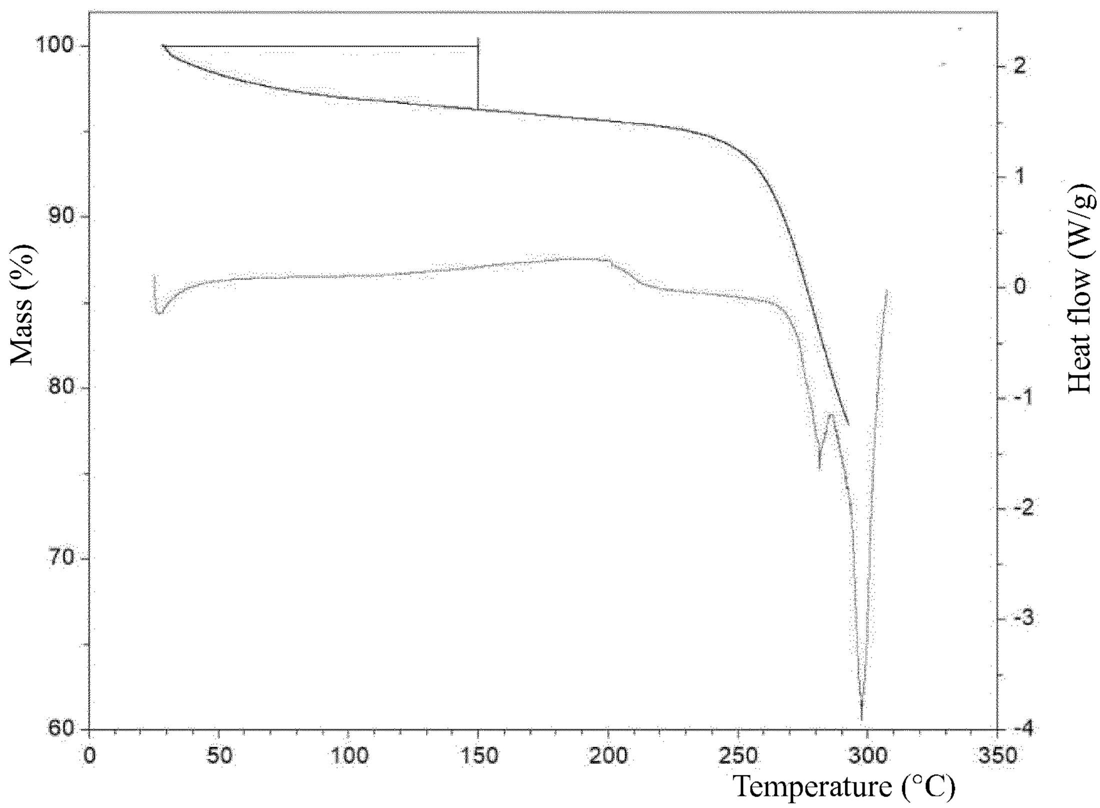
100
90
80
70
60
Mass (%)
0
50
100
150
200
250
300
350
Temperature (°C)
2
1
0
-1
-2
-3
-4
Heat flow (W/g)

FIG. 22

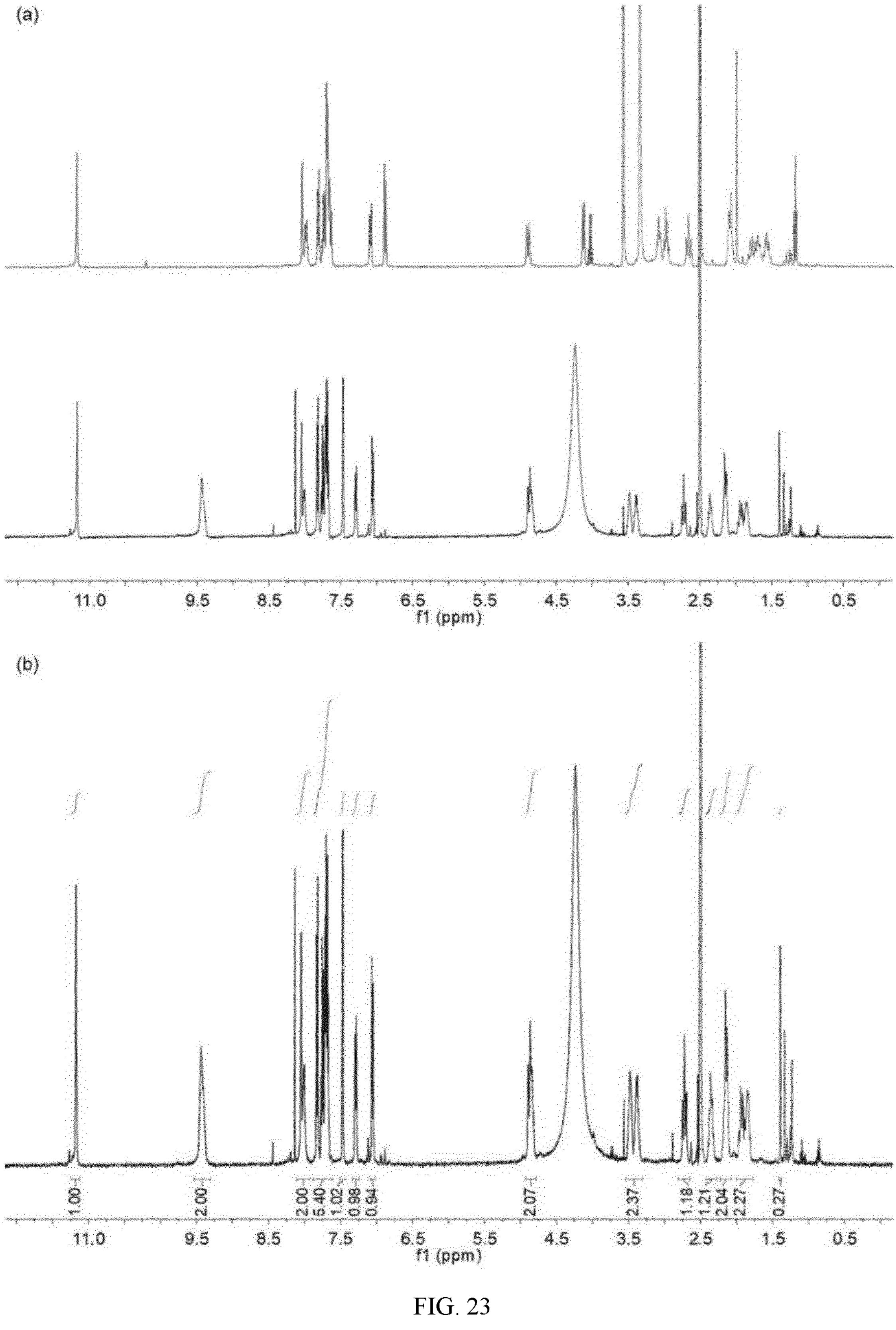
(a)
11.0
9.5
8.5
7.5
6.5
5.5
4.5
3.5
2.5
1.5
0.5
f1 (ppm)
(b)
1.00
2.00
2.00
5.40
1.02
0.98
0.94
2.07
2.37
1.18
1.21
2.04
2.27
0.27
11.0
9.5
8.5
7.5
6.5
5.5
4.5
3.5
2.5
1.5
0.5
f1 (ppm)

FIG. 23

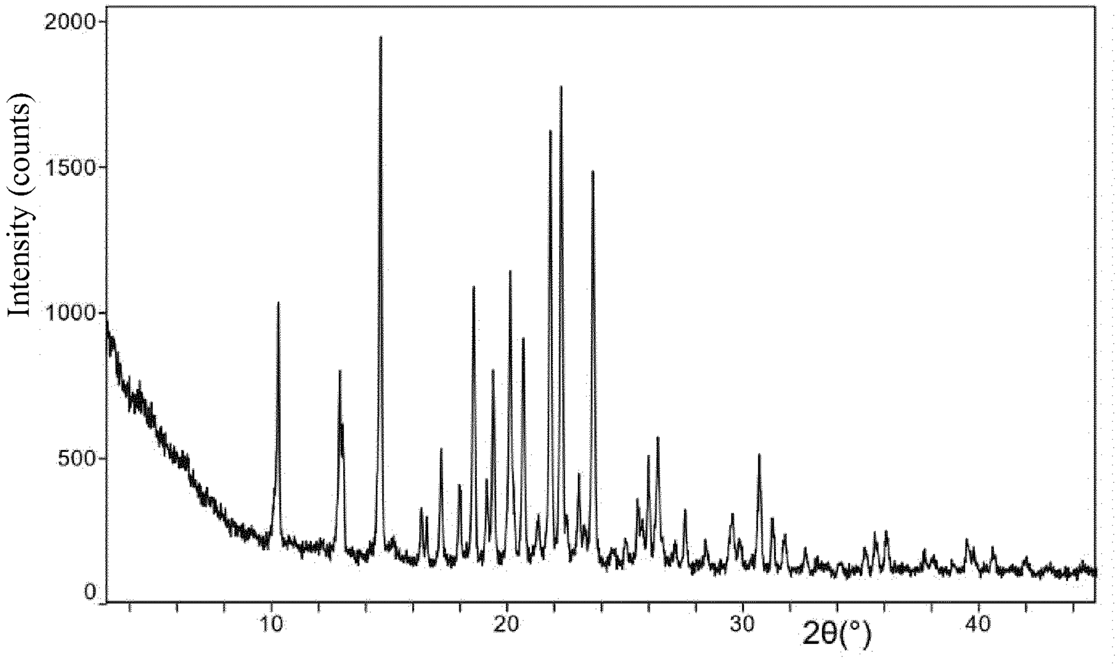
2000
1500
1000
500
0
Intensity (counts)
10
20
30
40
2θ(°)

FIG. 24

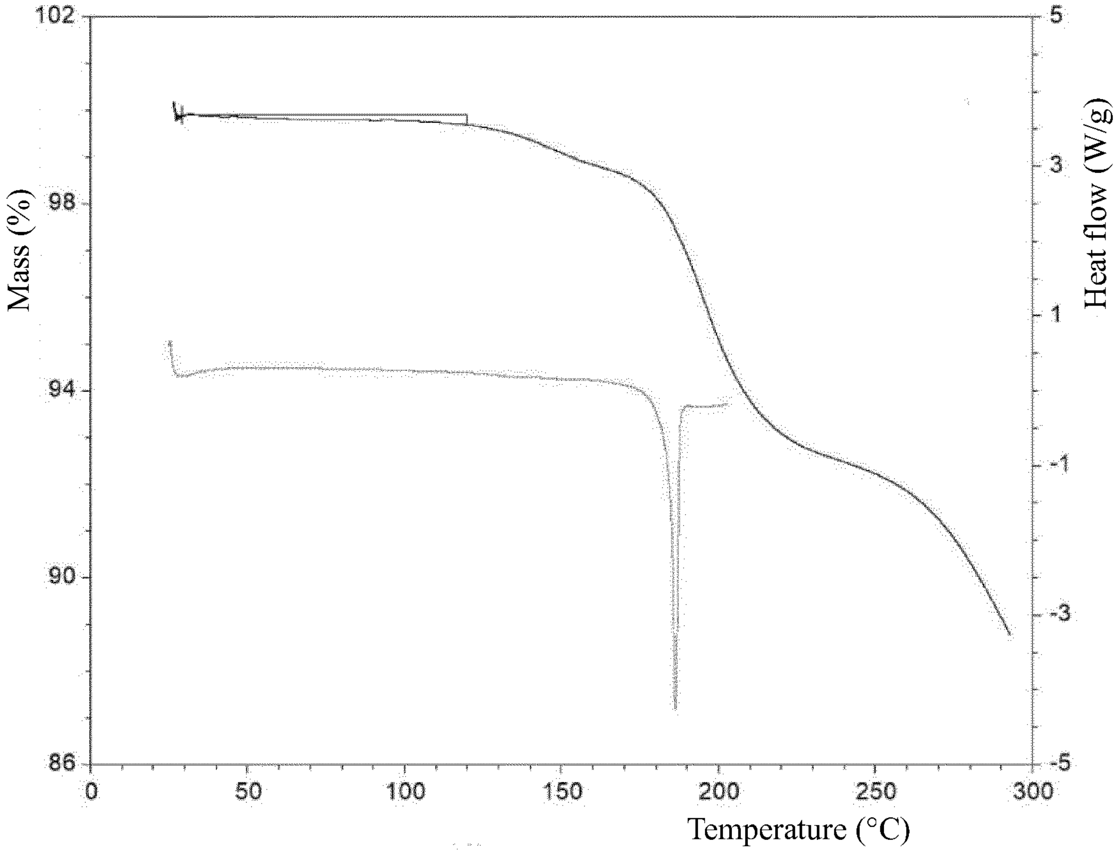
102
98
94
90
86
Mass (%)
5
3
1
-1
-3
-5
Heat flow (W/g)
0
50
100
150
200
250
300
Temperature (°C)

FIG. 25

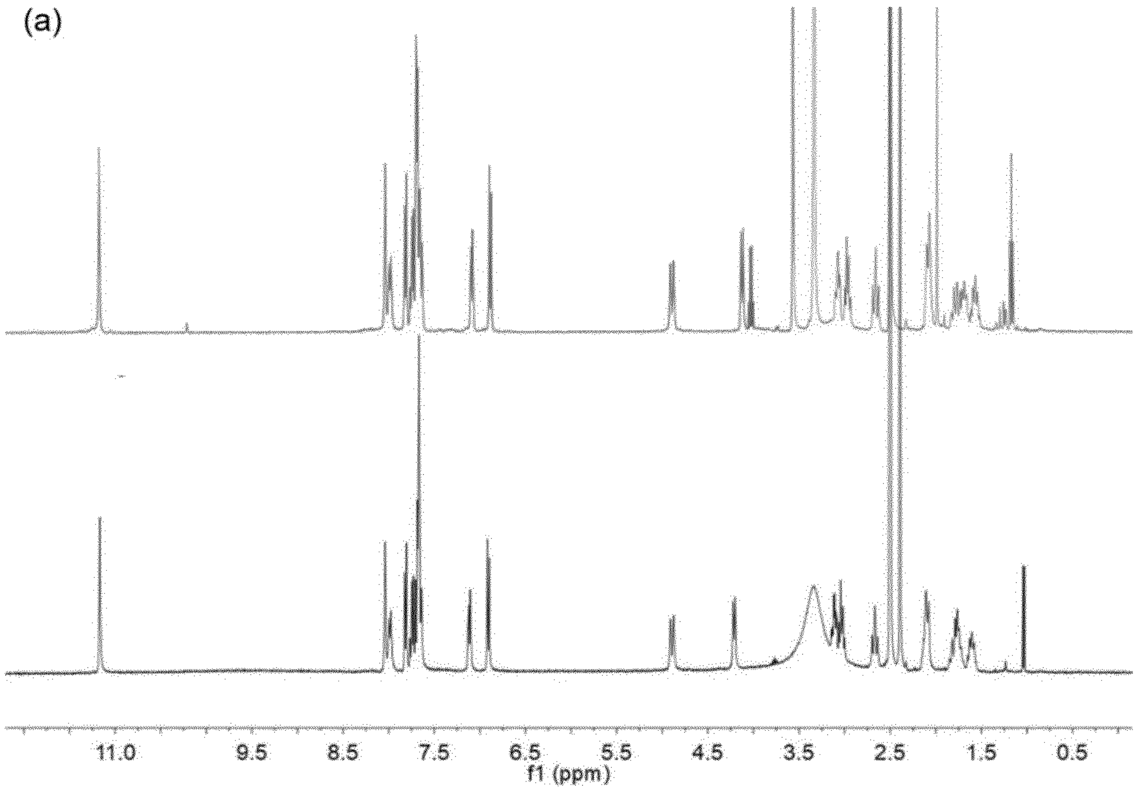
(a)
11.0
9.5
8.5
7.5
6.5
5.5
4.5
3.5
2.5
1.5
0.5
f1 (ppm)

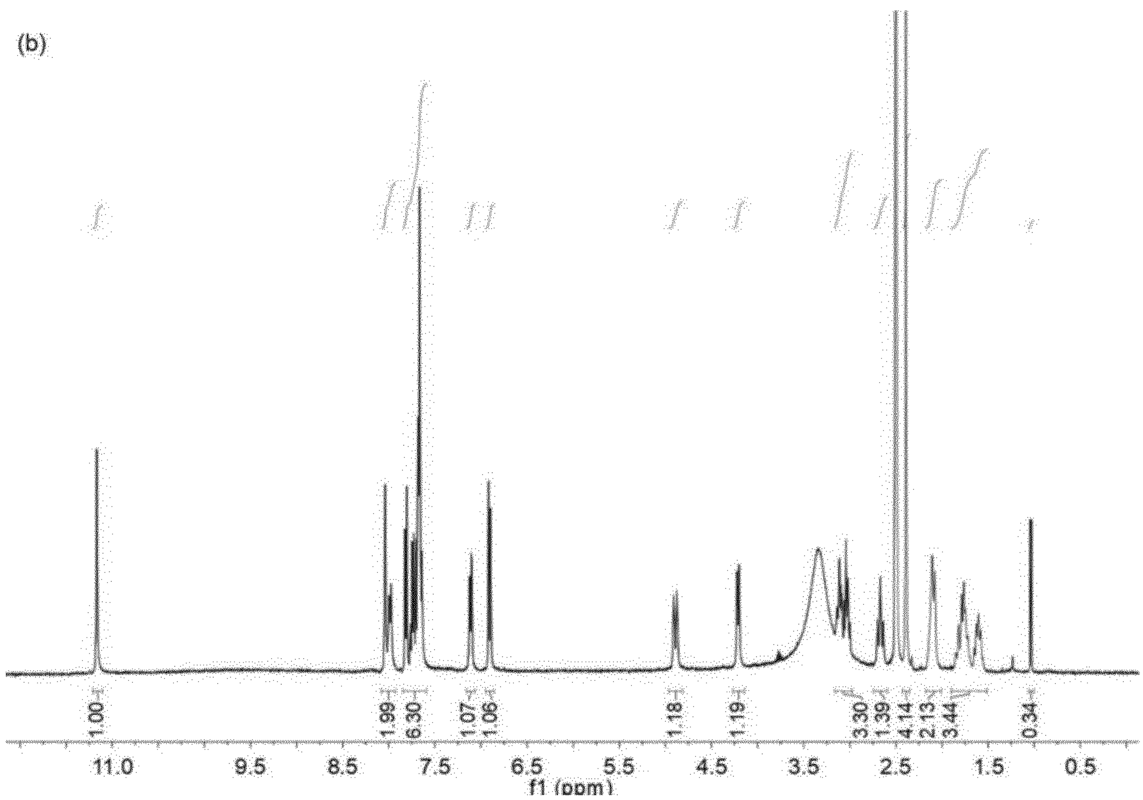
(b)
1.00
1.99
6.30
1.07
1.06
1.18
1.19
3.30
1.39
4.14
2.13
3.44
0.34
11.0
9.5
8.5
7.5
6.5
5.5
4.5
3.5
2.5
1.5
0.5
f1 (ppm)

FIG. 26

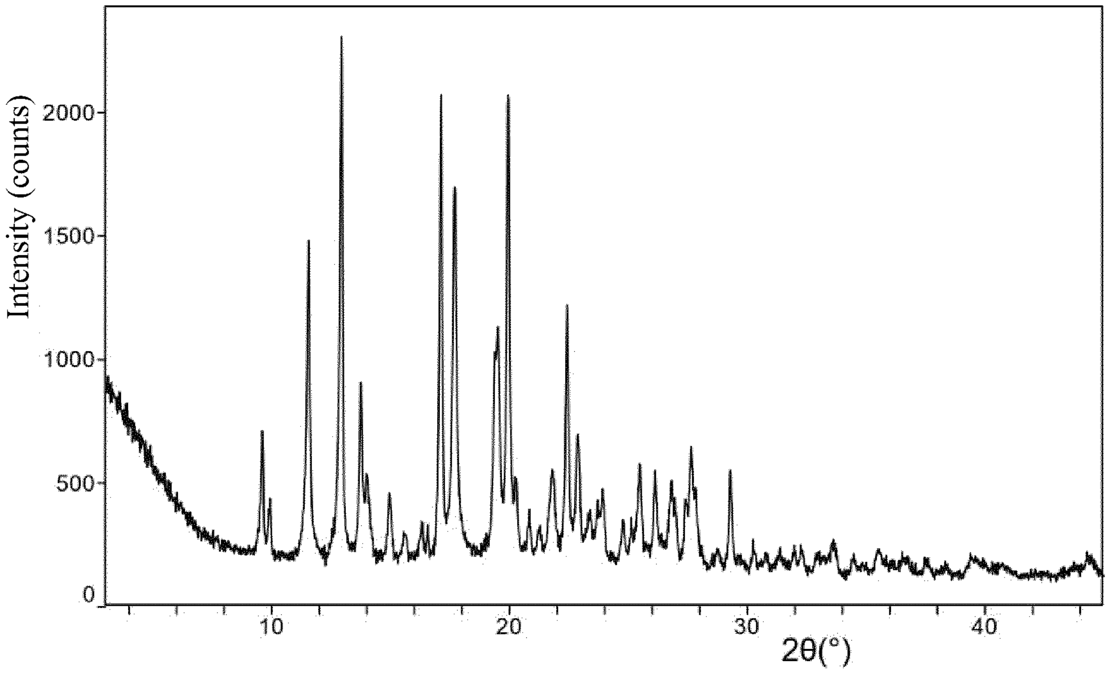
Intensity (counts)
2000
1500
1000
500
0
10
20
30
40
2θ(°)

FIG. 27

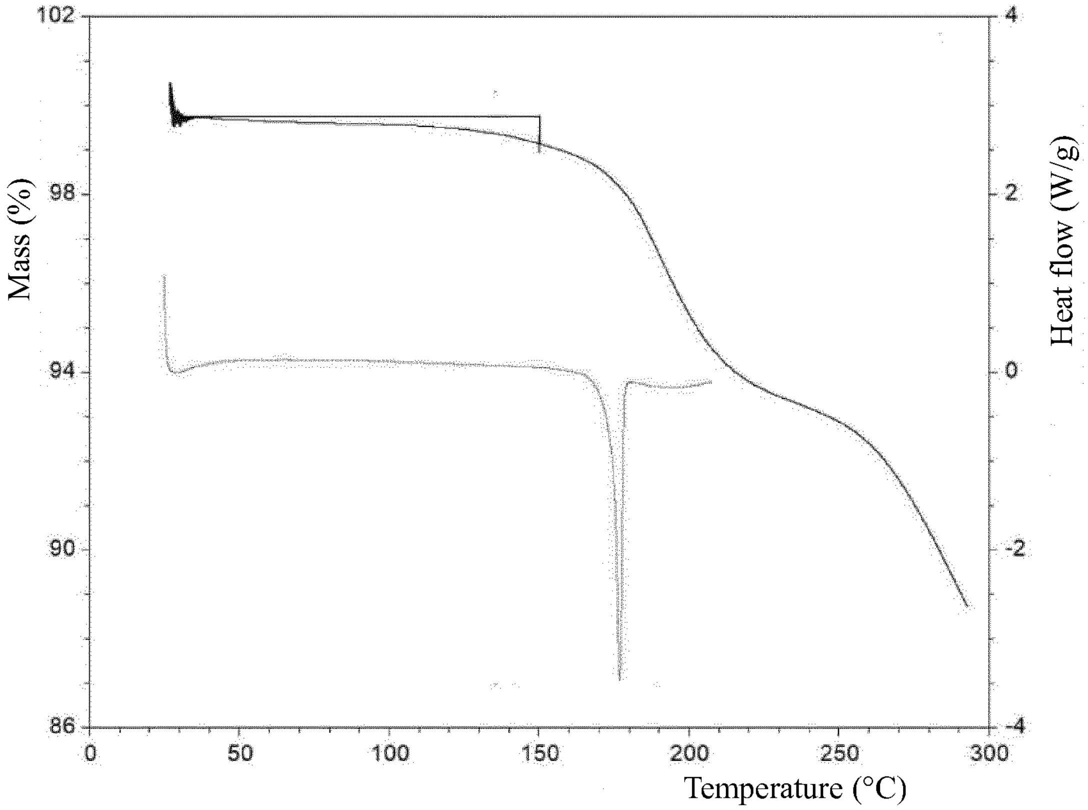
102
98
94
90
86
Mass (%)
4
2
0
-2
-4
Heat flow (W/g)
0
50
100
150
200
250
300
Temperature (°C)

FIG. 28

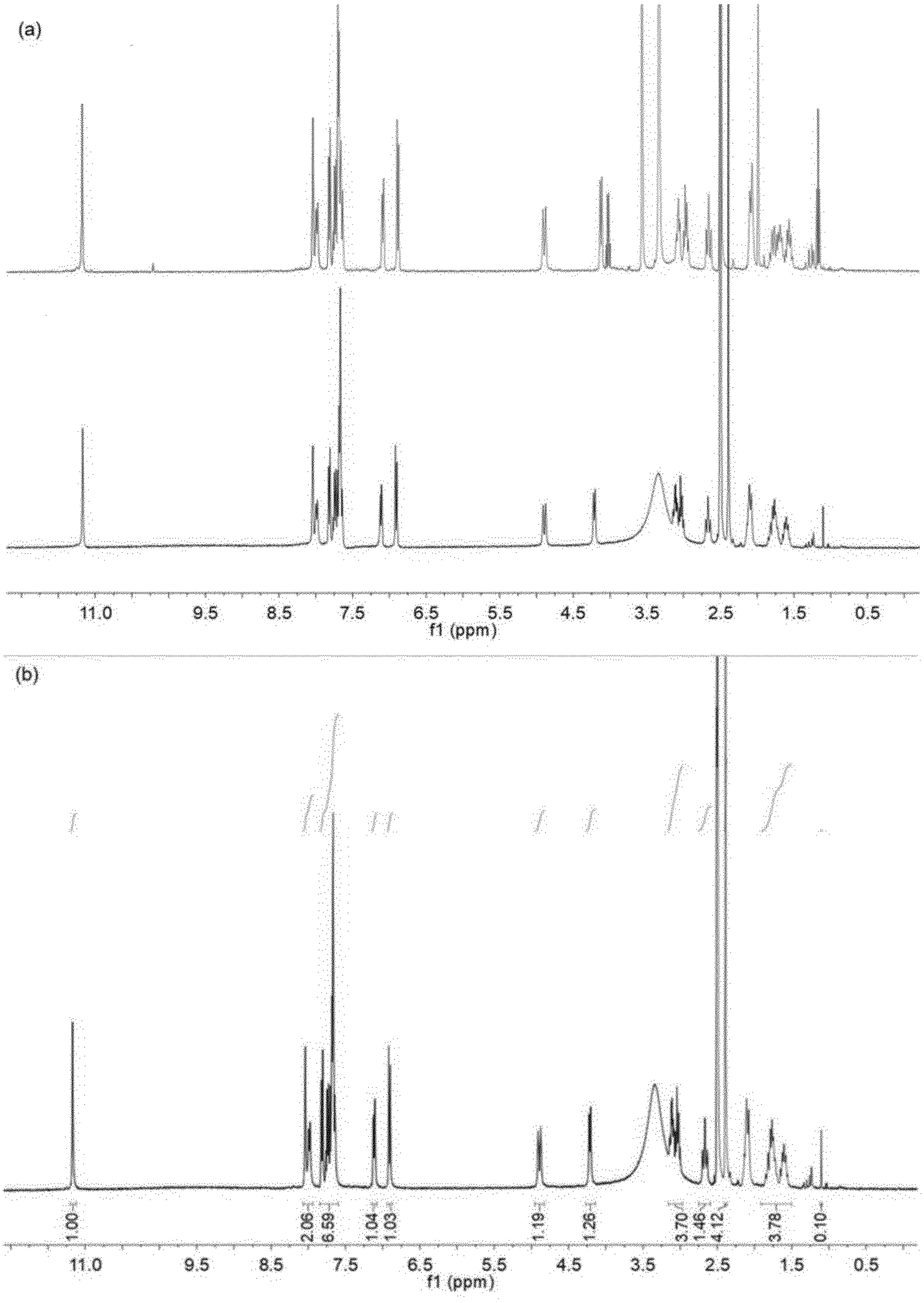
(a)
11.0
9.5
8.5
7.5
6.5
5.5
4.5
3.5
2.5
1.5
0.5
f1 (ppm)
(b)
1.00
2.06
6.59
1.04
1.03
1.19
1.26
3.70
1.46
4.12
3.78
0.10
11.0
9.5
8.5
7.5
6.5
5.5
4.5
3.5
2.5
1.5
0.5
f1 (ppm)

FIG. 29

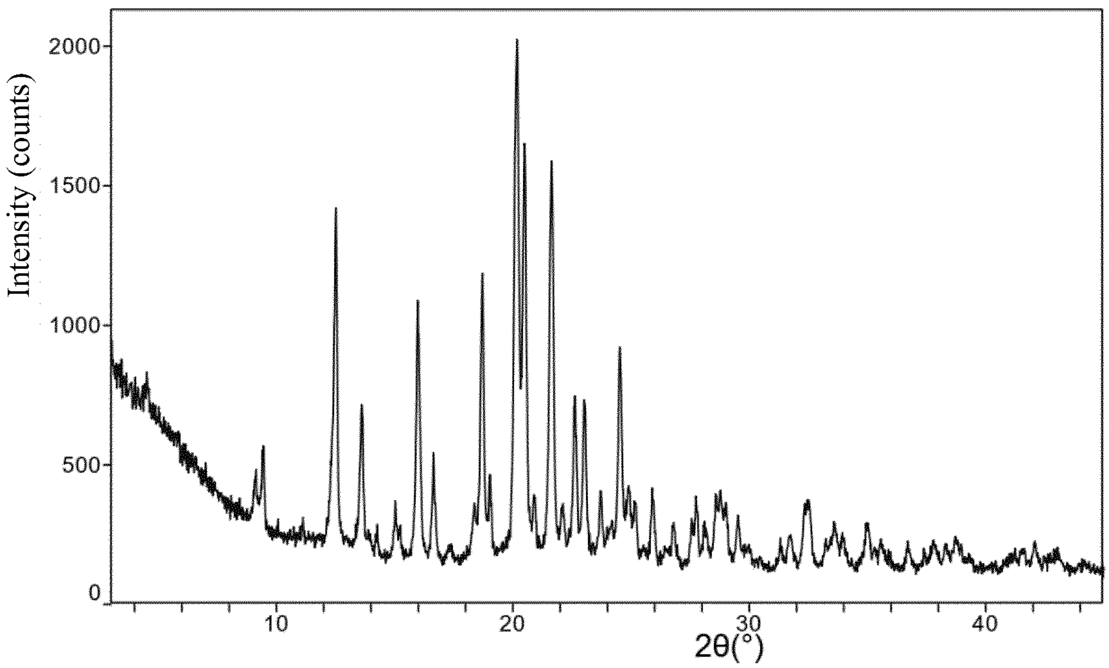
Intensity (counts)
2000
1500
1000
500
0
10
20
30
40
2θ(°)

FIG. 30

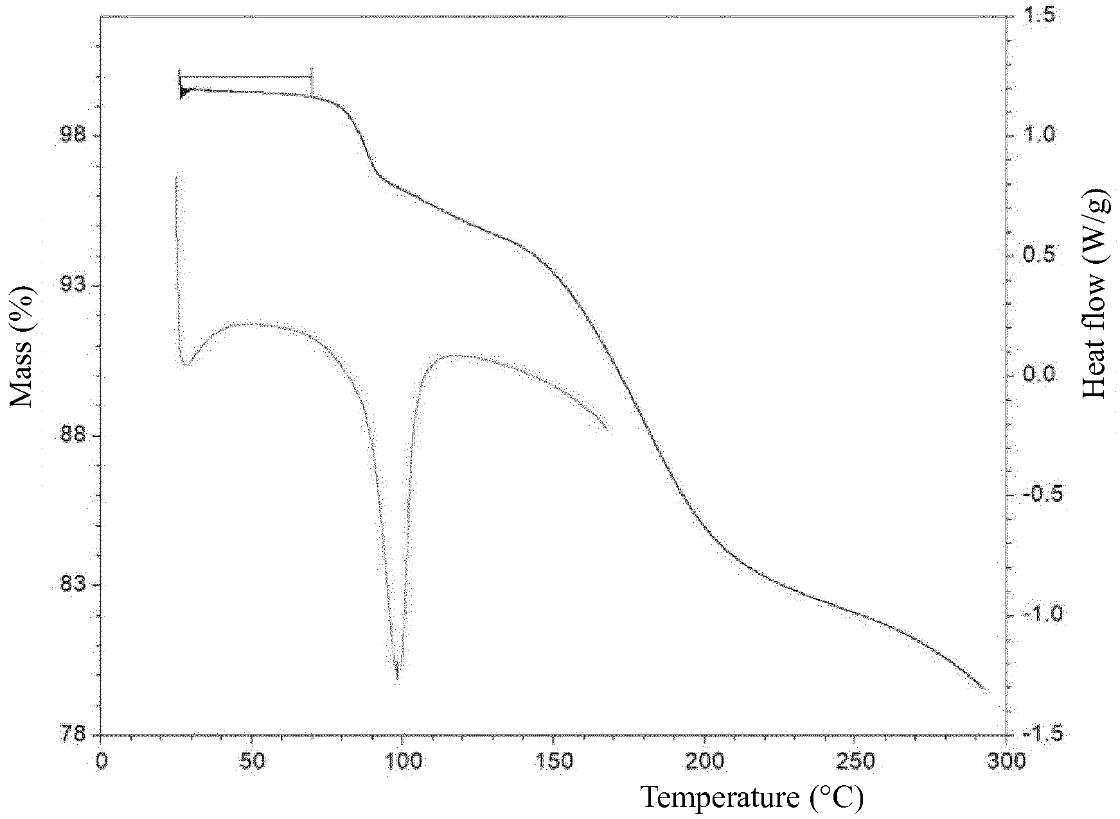
Mass (%)
98
93
88
83
78
Heat flow (W/g)
1.5
1.0
0.5
0.0
-0.5
-1.0
-1.5
0
50
100
150
200
250
300
Temperature (°C)

FIG. 31

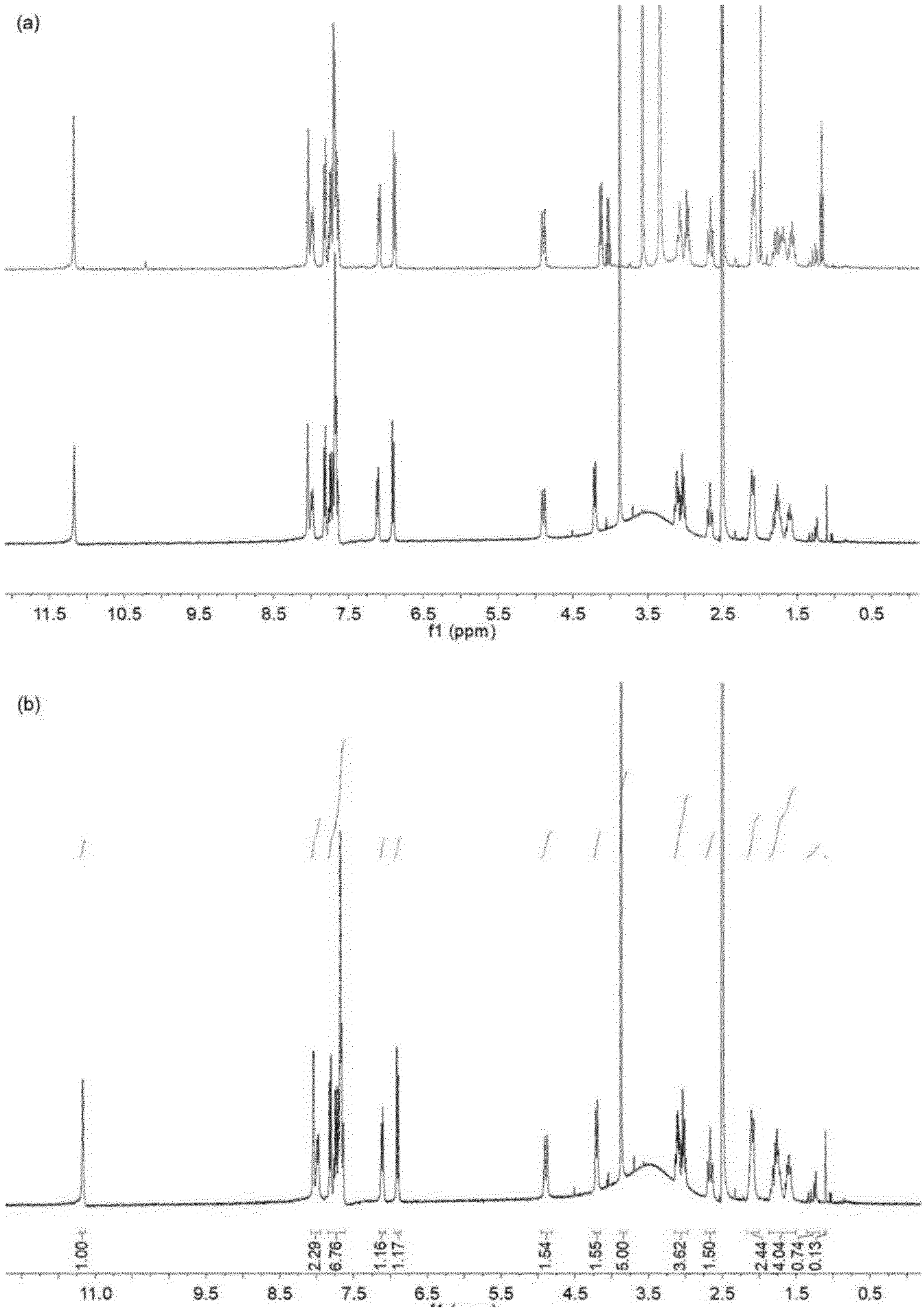
(a)
11.5
10.5
9.5
8.5
7.5
6.5
5.5
4.5
3.5
2.5
1.5
0.5
f1 (ppm)
(b)
1.00
2.29
6.76
1.16
1.17
1.54
1.55
5.00
3.62
1.50
2.44
4.04
0.74
0.13
11.0
9.5
8.5
7.5
6.5
5.5
4.5
3.5
2.5
1.5
0.5

FIG. 32

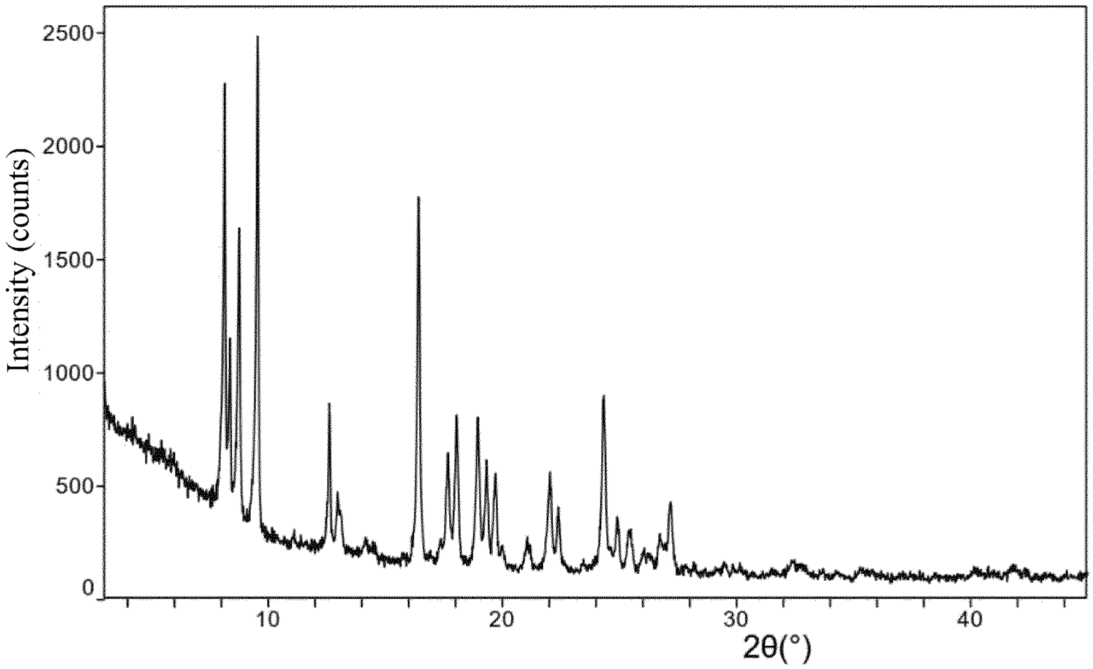

2500
2000
1500
1000
500
0
Intensity (counts)
10
20
30
40
2θ(°)


FIG. 33

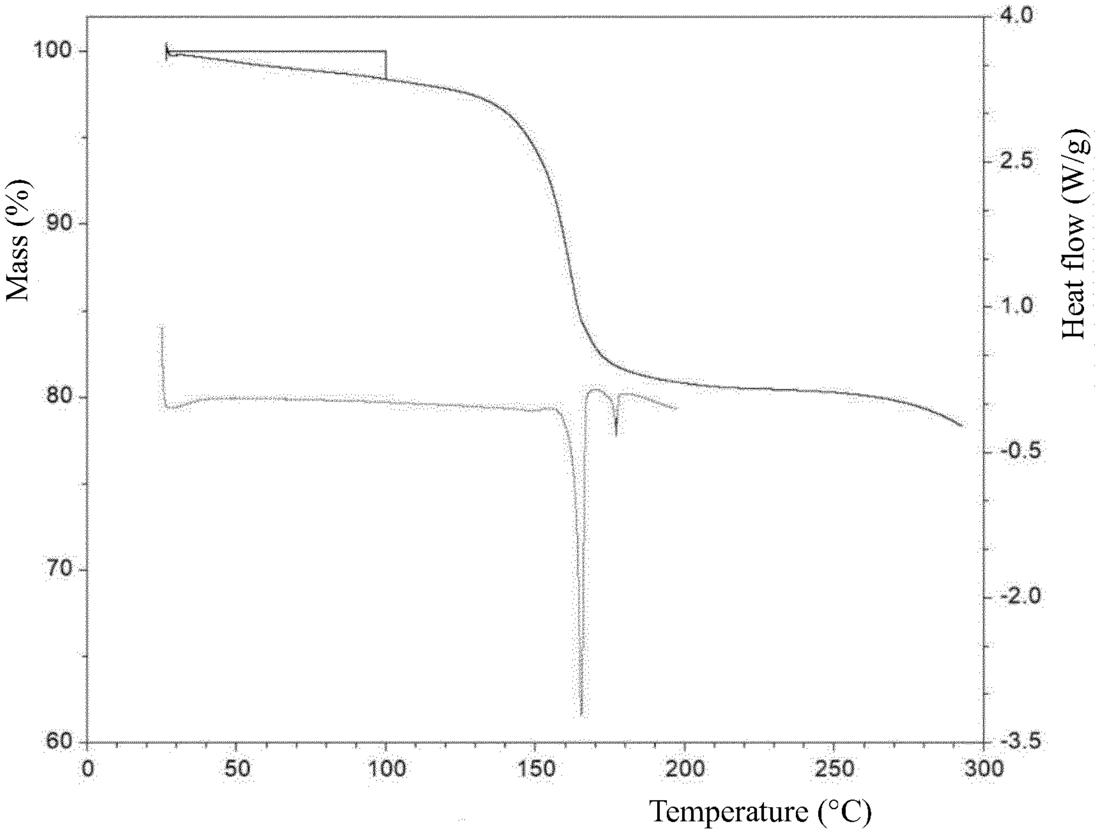

100
90
80
70
60
Mass (%)
4.0
2.5
1.0
-0.5
-2.0
-3.5
Heat flow (W/g)
0
50
100
150
200
250
300
Temperature (°C)


FIG. 34

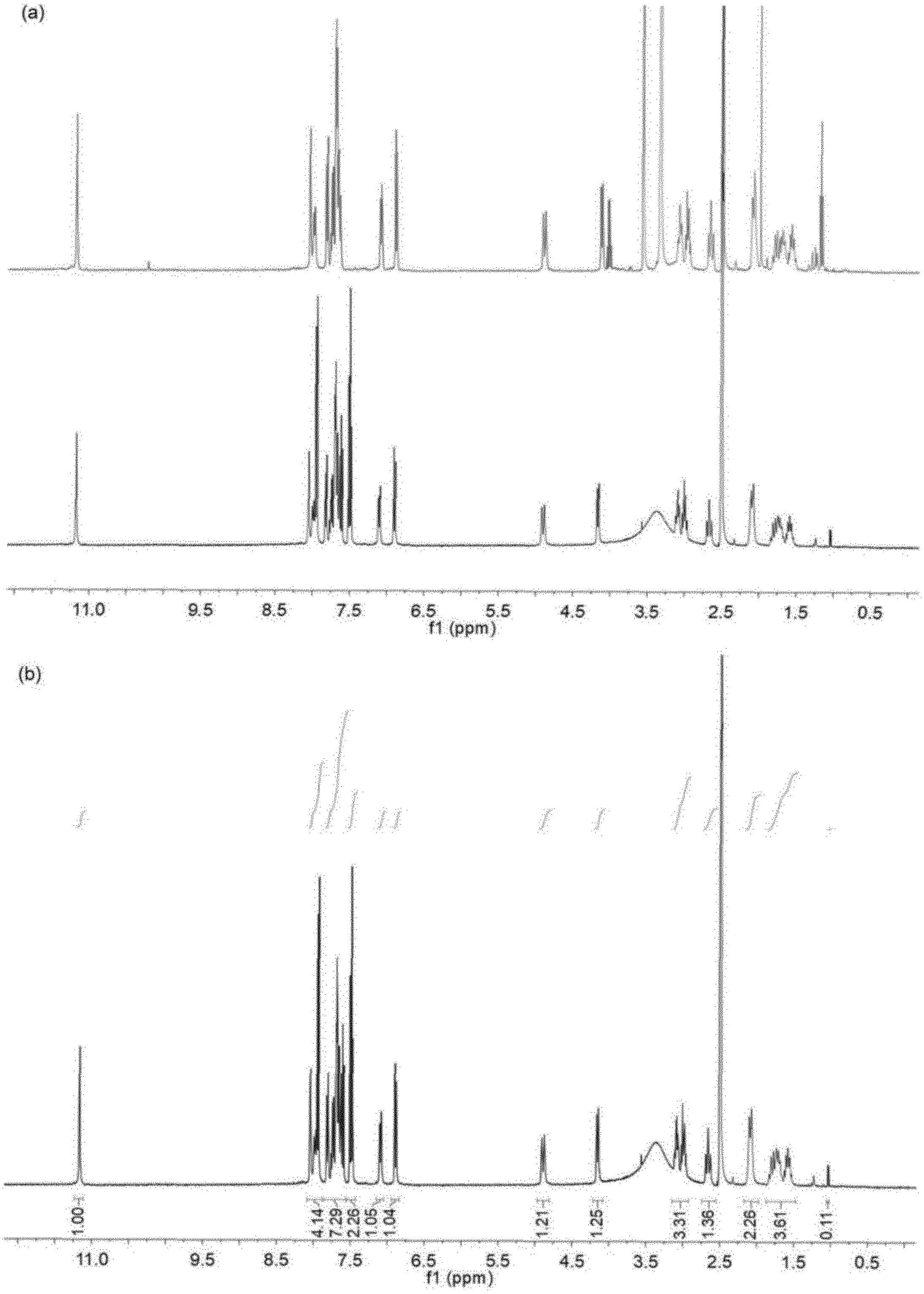
(a)
11.0
9.5
8.5
7.5
6.5
5.5
4.5
3.5
2.5
1.5
0.5
f1 (ppm)
(b)
1.00
4.14
7.29
2.26
1.05
1.04
1.21
1.25
3.31
1.36
2.26
3.61
0.11
11.0
9.5
8.5
7.5
6.5
5.5
4.5
3.5
2.5
1.5
0.5
f1 (ppm)

FIG. 35

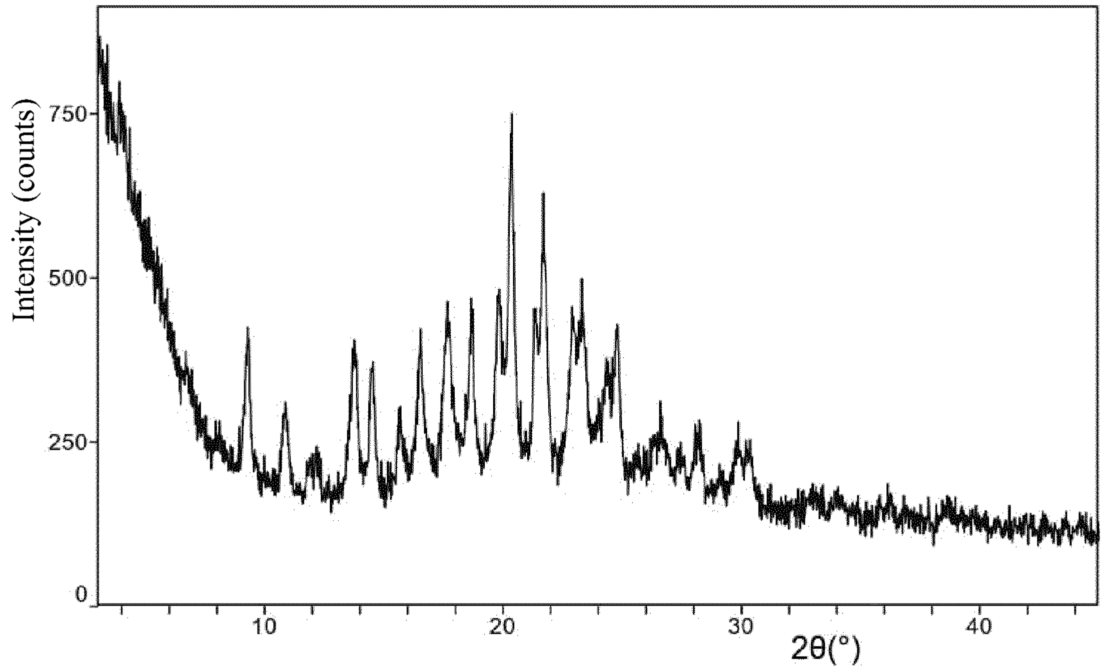
Intensity (counts)
750
500
250
0
10
20
30
40
2θ(°)

FIG. 36

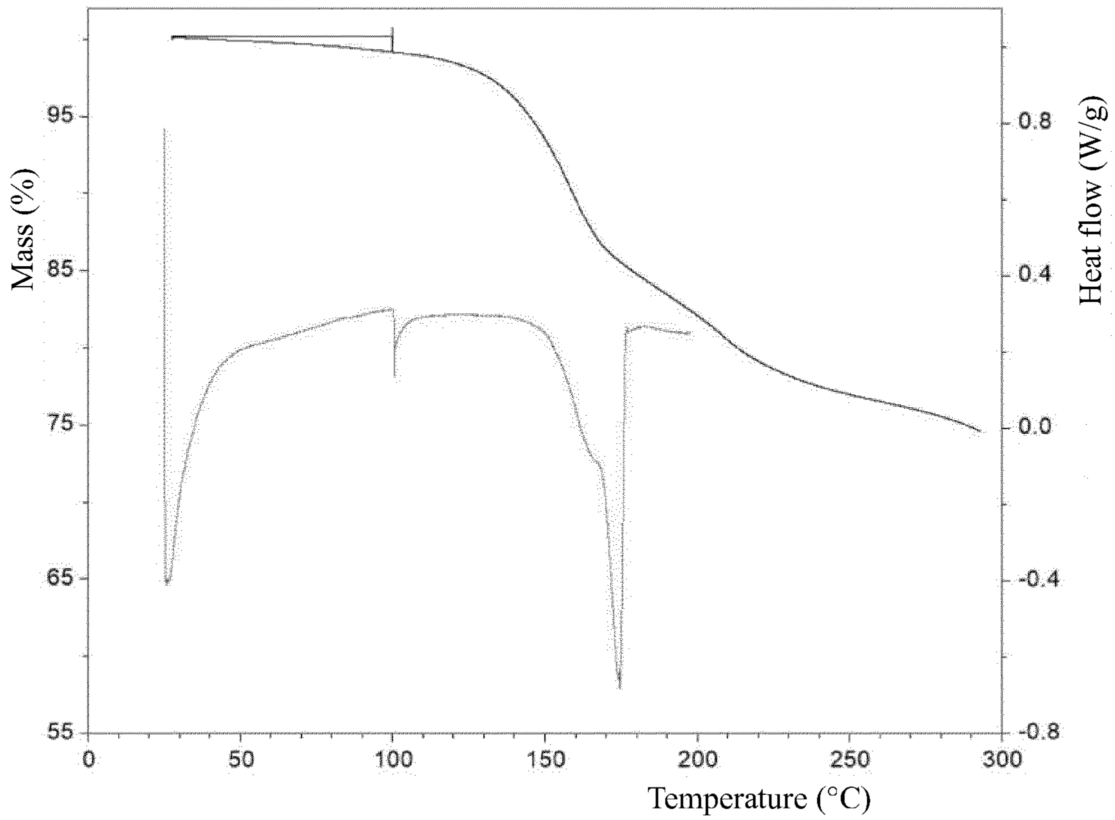
Mass (%)
95
85
75
65
55
Heat flow (W/g)
0.8
0.4
0.0
-0.4
-0.8
0
50
100
150
200
250
300
Temperature (°C)

FIG. 37

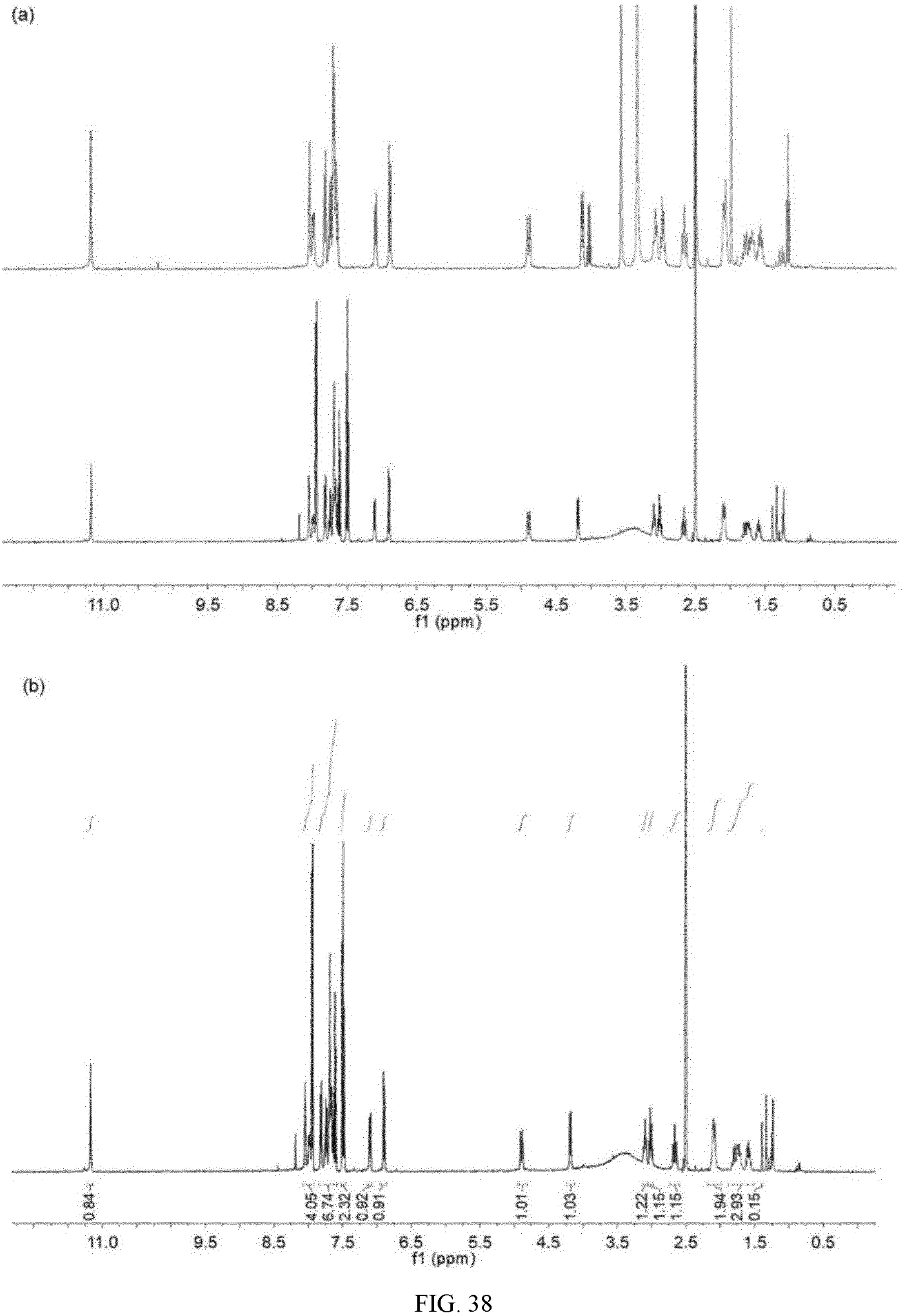
(a)
11.0
9.5
8.5
7.5
6.5
5.5
4.5
3.5
2.5
1.5
0.5
f1 (ppm)
(b)
0.84
4.05
6.74
2.32
0.92
0.91
1.01
1.03
1.22
1.15
1.15
1.94
2.93
0.15
11.0
9.5
8.5
7.5
6.5
5.5
4.5
3.5
2.5
1.5
0.5
f1 (ppm)

FIG. 38

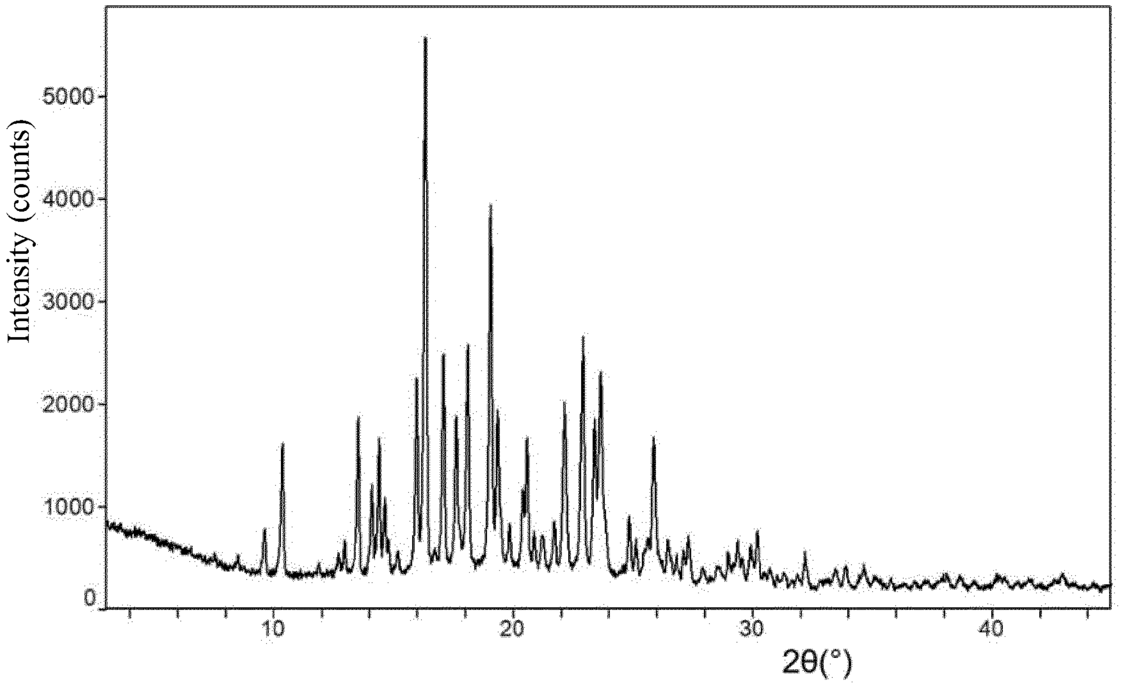
Intensity (counts)
5000
4000
3000
2000
1000
0
10
20
30
40
2θ(°)

FIG. 39

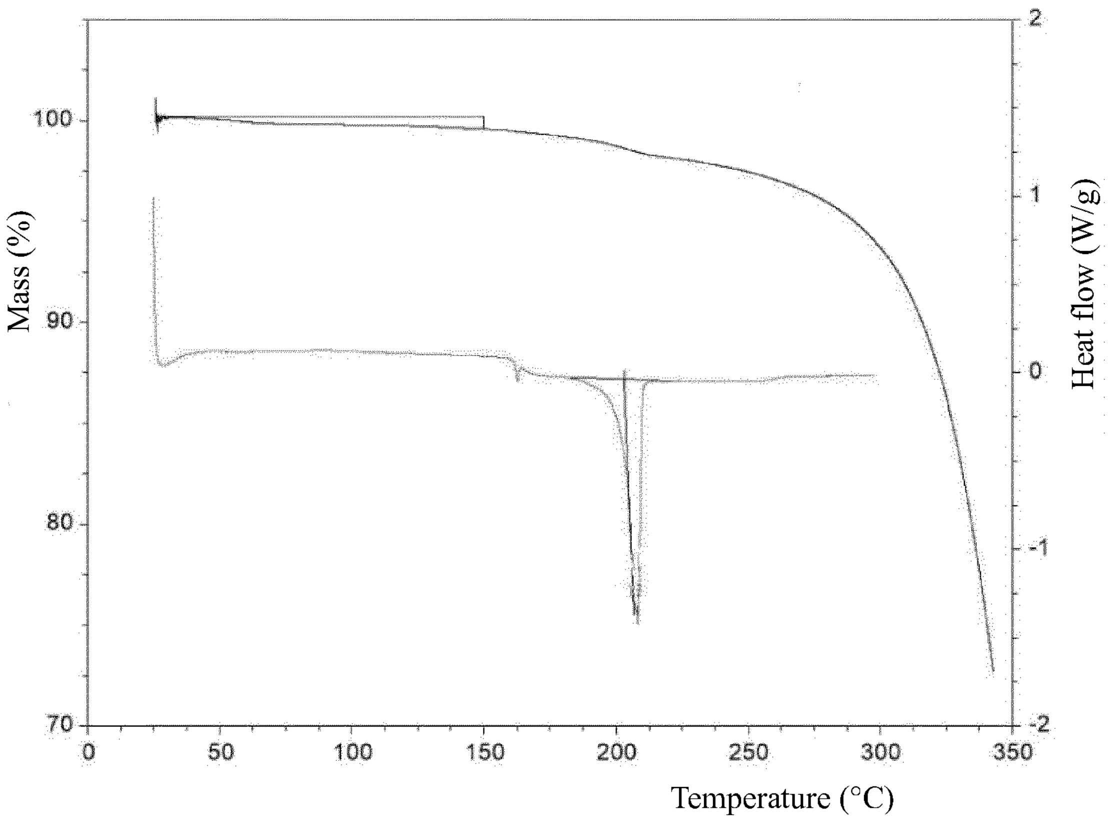
Mass (%)
100
90
80
70
Heat flow (W/g)
2
1
0
-1
-2
0
50
100
150
200
250
300
350
Temperature (°C)

FIG. 40

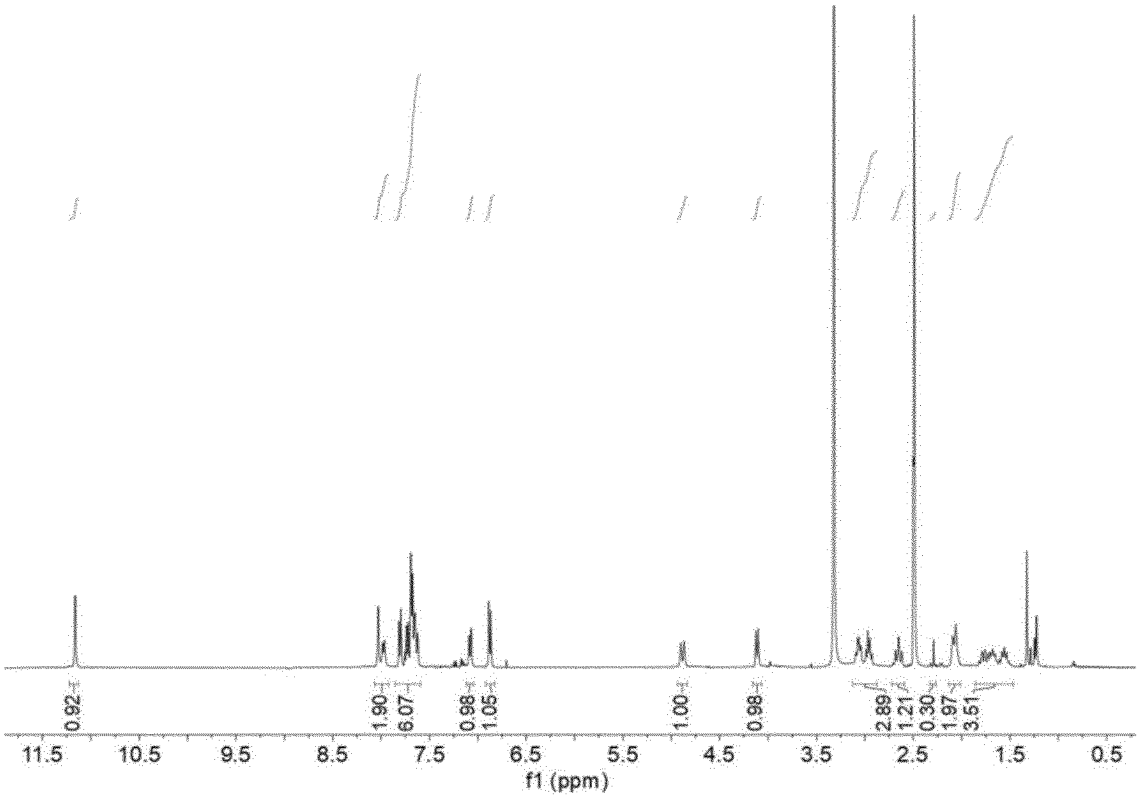
0.92
1.90
6.07
0.98
1.05
1.00
0.98
2.89
1.21
0.30
1.97
3.51
11.5
10.5
9.5
8.5
7.5
6.5
5.5
4.5
3.5
2.5
1.5
0.5
f1 (ppm)

FIG. 41

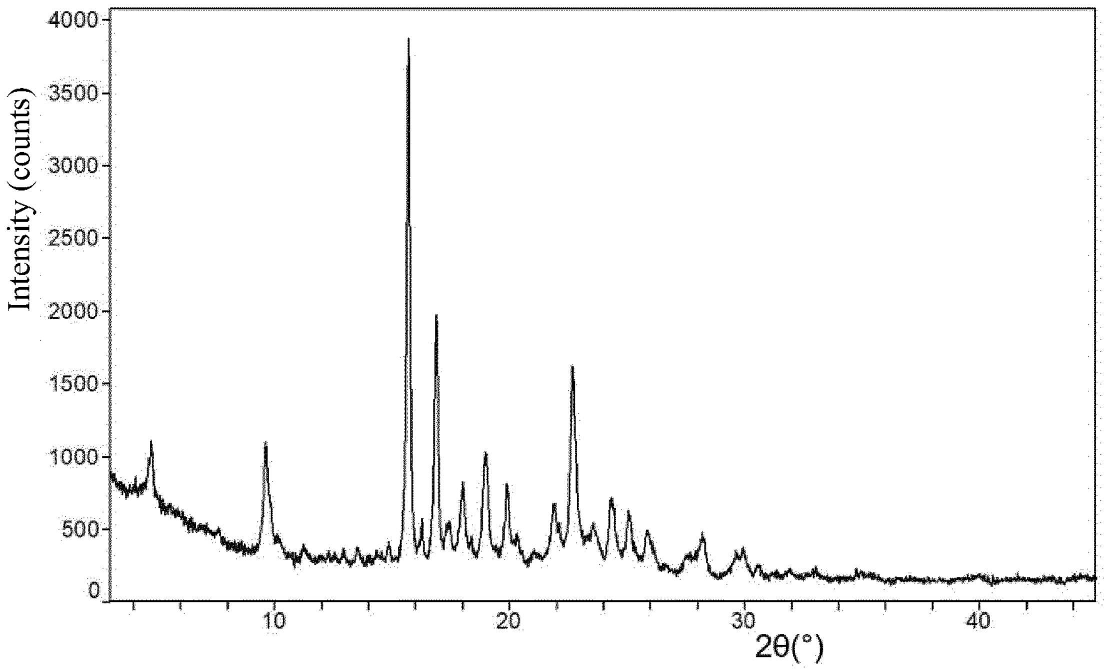
4000
3500
3000
2500
2000
1500
1000
500
0
Intensity (counts)
10
20
30
40
2θ(°)

FIG. 42

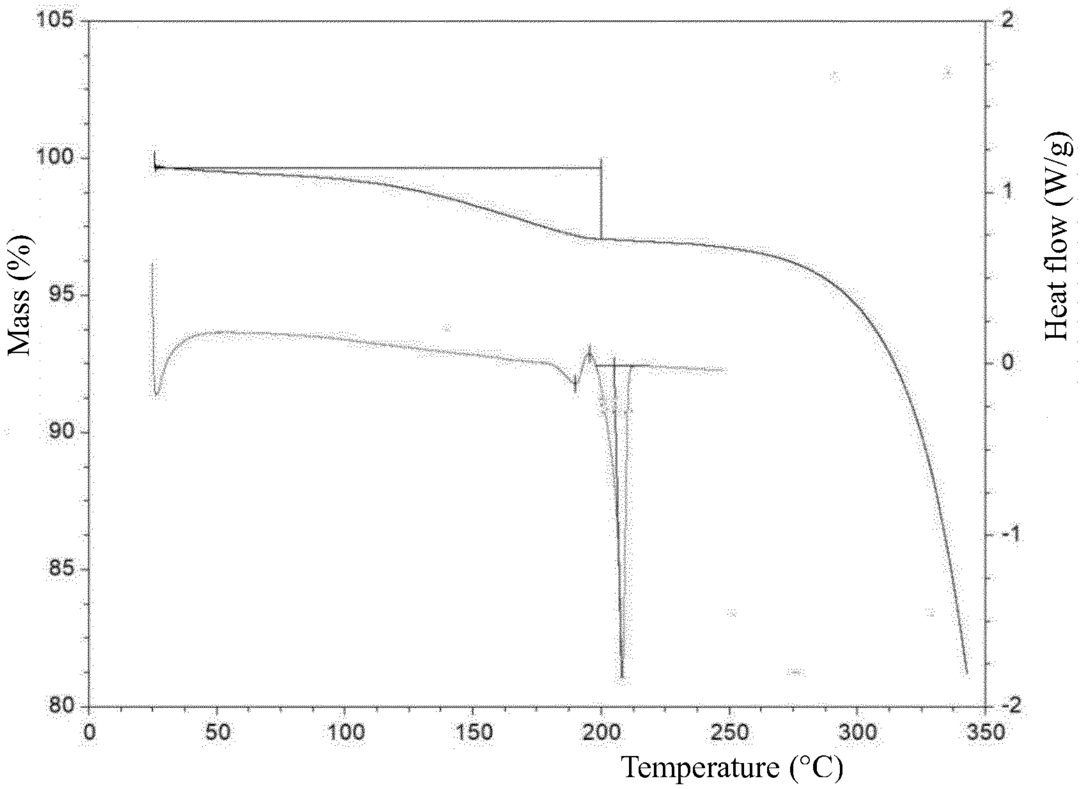
105
100
95
90
85
80
Mass (%)
2
1
0
-1
-2
Heat flow (W/g)
0
50
100
150
200
250
300
350
Temperature (°C)

FIG. 43

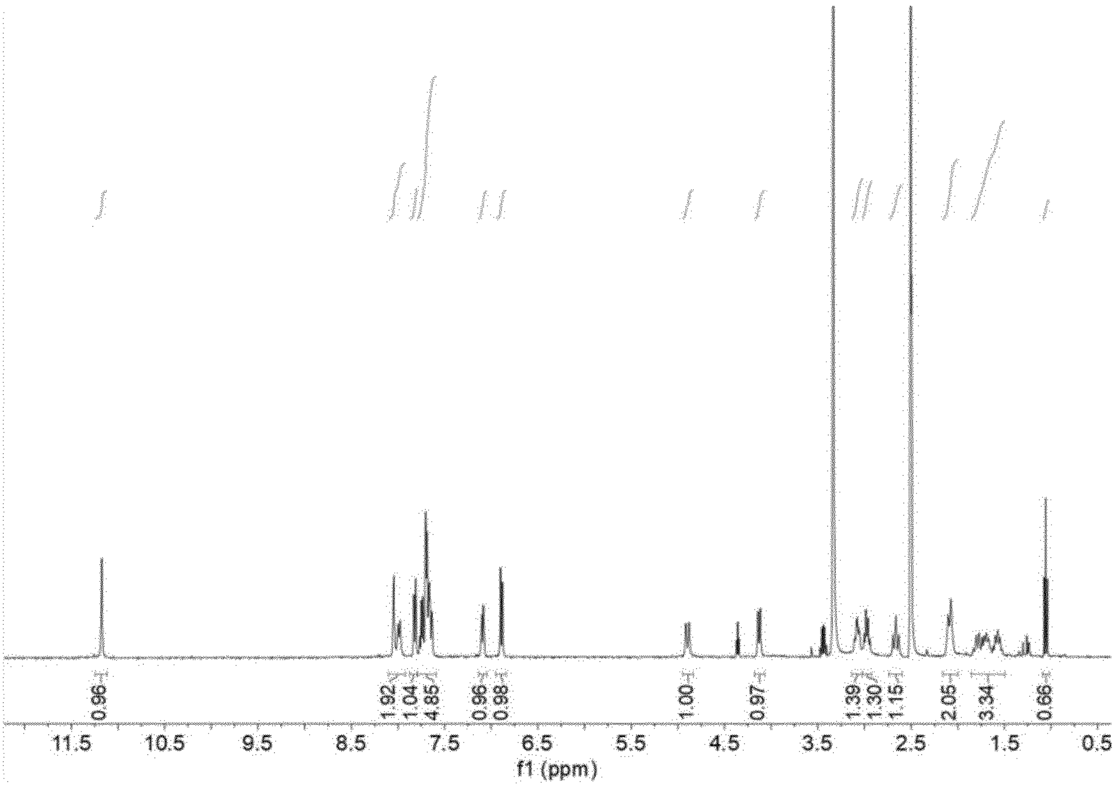
0.96
1.92
1.04
4.85
0.96
0.98
1.00
0.97
1.39
1.30
1.15
2.05
3.34
0.66
11.5
10.5
9.5
8.5
7.5
6.5
5.5
4.5
3.5
2.5
1.5
0.5
f1 (ppm)

FIG. 44

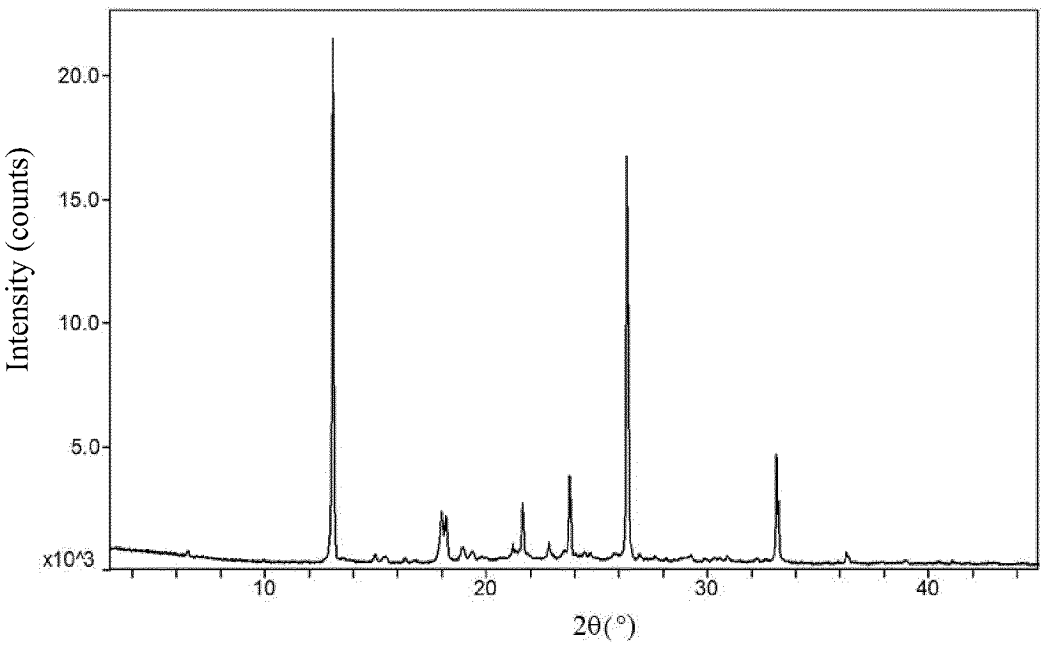
Intensity (counts)
20.0
15.0
10.0
5.0
x10^3
10
20
30
40
2θ(°)

FIG. 45

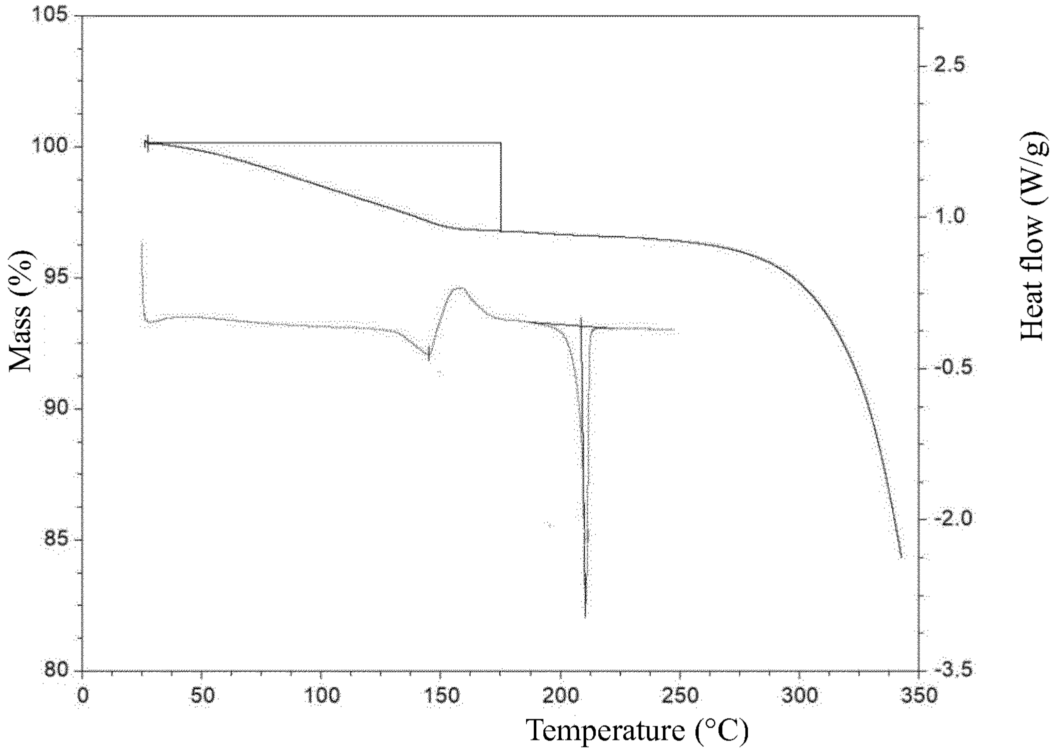

105
100
95
90
85
80
Mass (%)
2.5
1.0
-0.5
-2.0
-3.5
Heat flow (W/g)
0
50
100
150
200
250
300
350
Temperature (°C)


FIG. 46

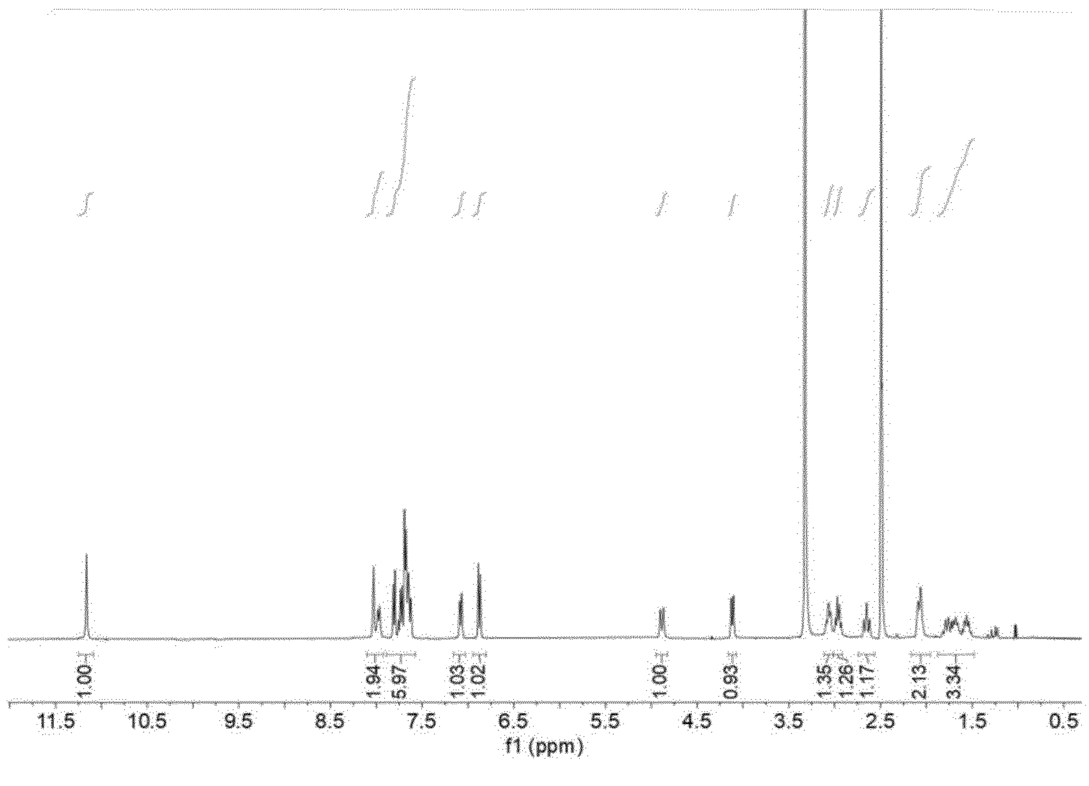

1.00
1.94
5.97
1.03
1.02
1.00
0.93
1.35
1.26
1.17
2.13
3.34
11.5
10.5
9.5
8.5
7.5
6.5
5.5
4.5
3.5
2.5
1.5
0.5
f1 (ppm)


FIG. 47

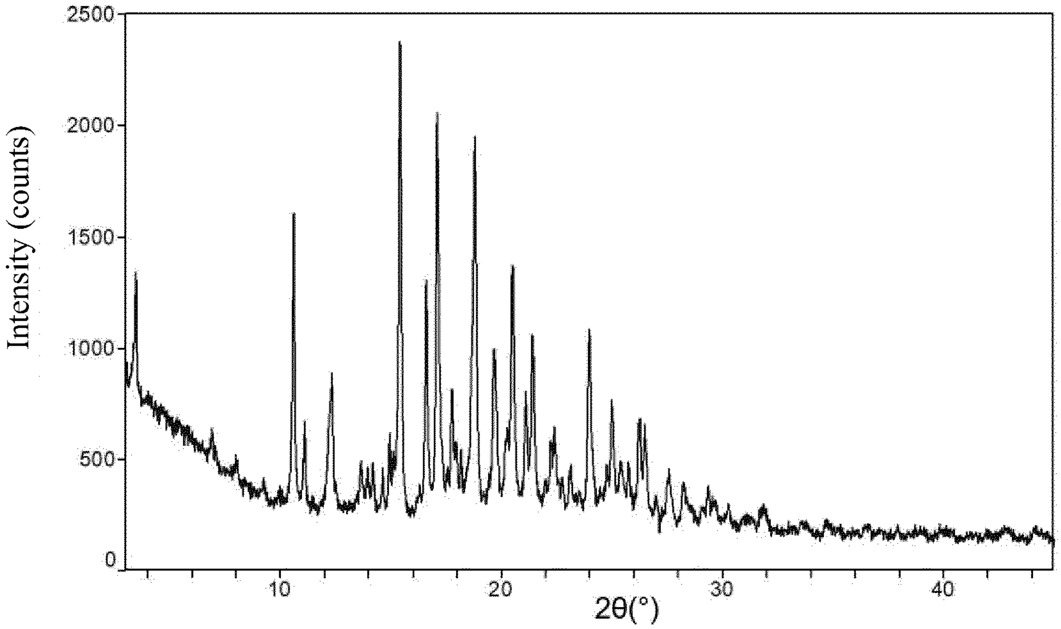
Intensity (counts)
2500
2000
1500
1000
500
0
10
20
30
40
2θ(°)

FIG. 48

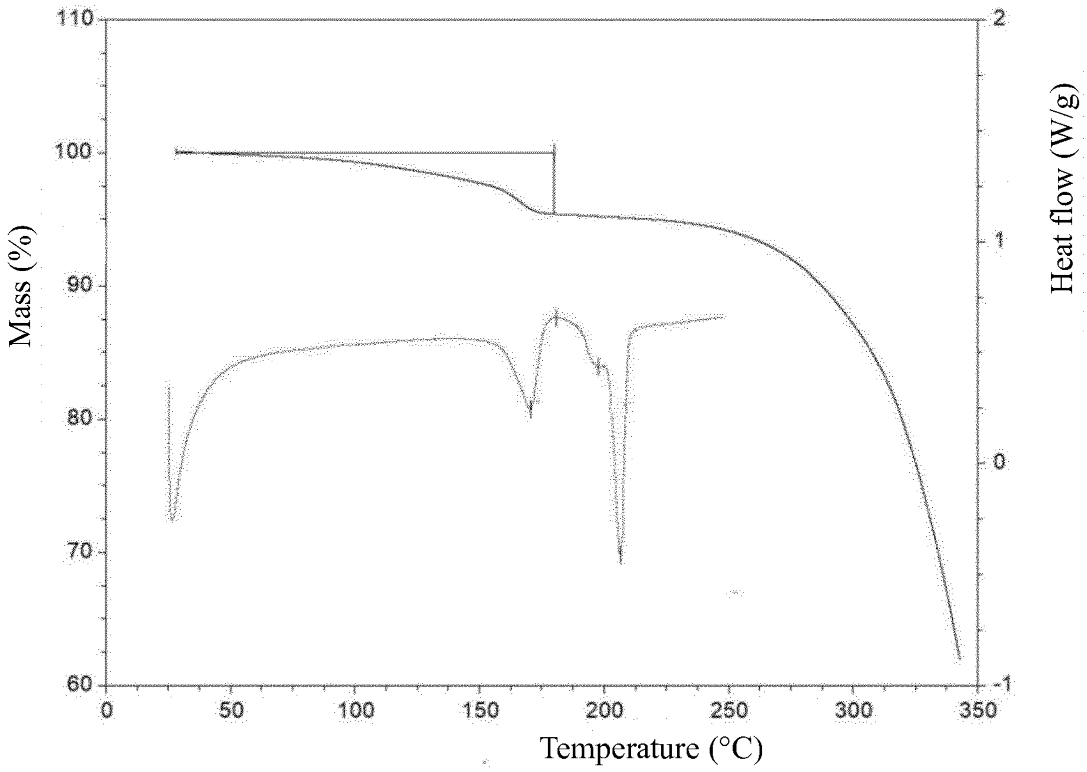
Mass (%)
110
100
90
80
70
60
Heat flow (W/g)
2
1
0
-1
0
50
100
150
200
250
300
350
Temperature (°C)

FIG. 49

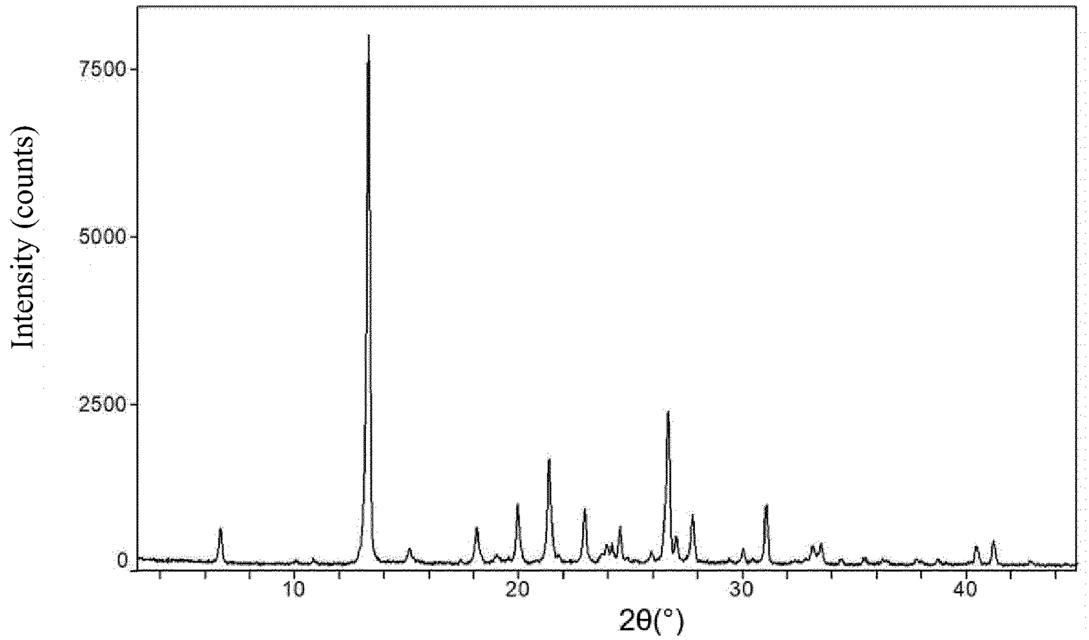
7500
5000
2500
0
Intensity (counts)
10
20
30
40
2θ(°)

FIG. 50

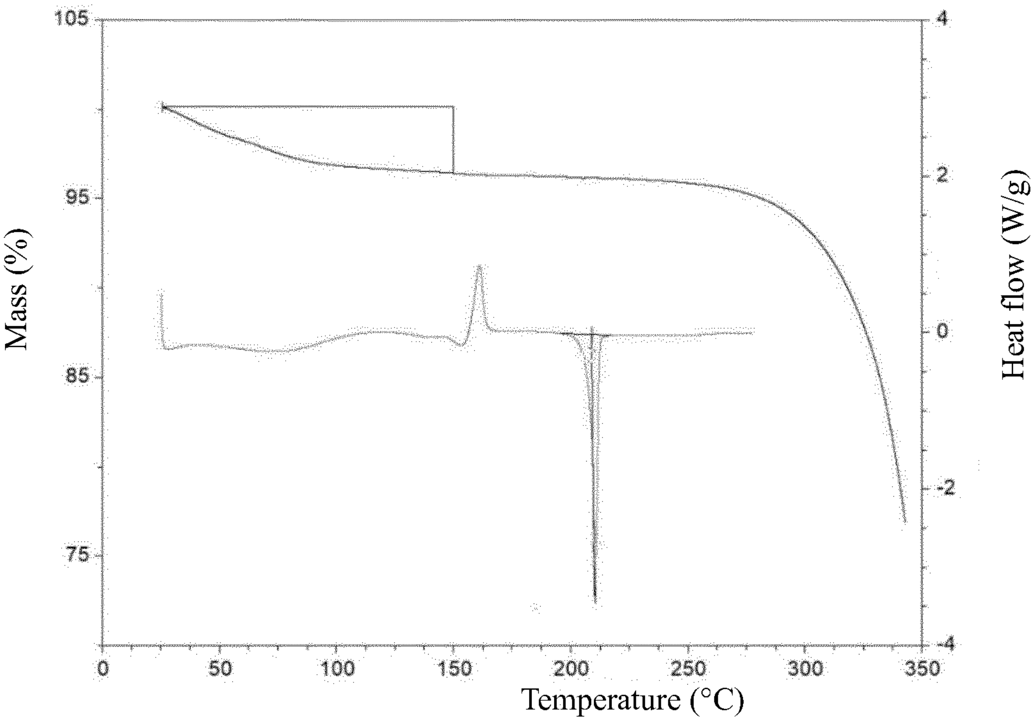
105
95
85
75
Mass (%)
4
2
0
-2
-4
Heat flow (W/g)
0
50
100
150
200
250
300
350
Temperature (°C)

FIG. 51

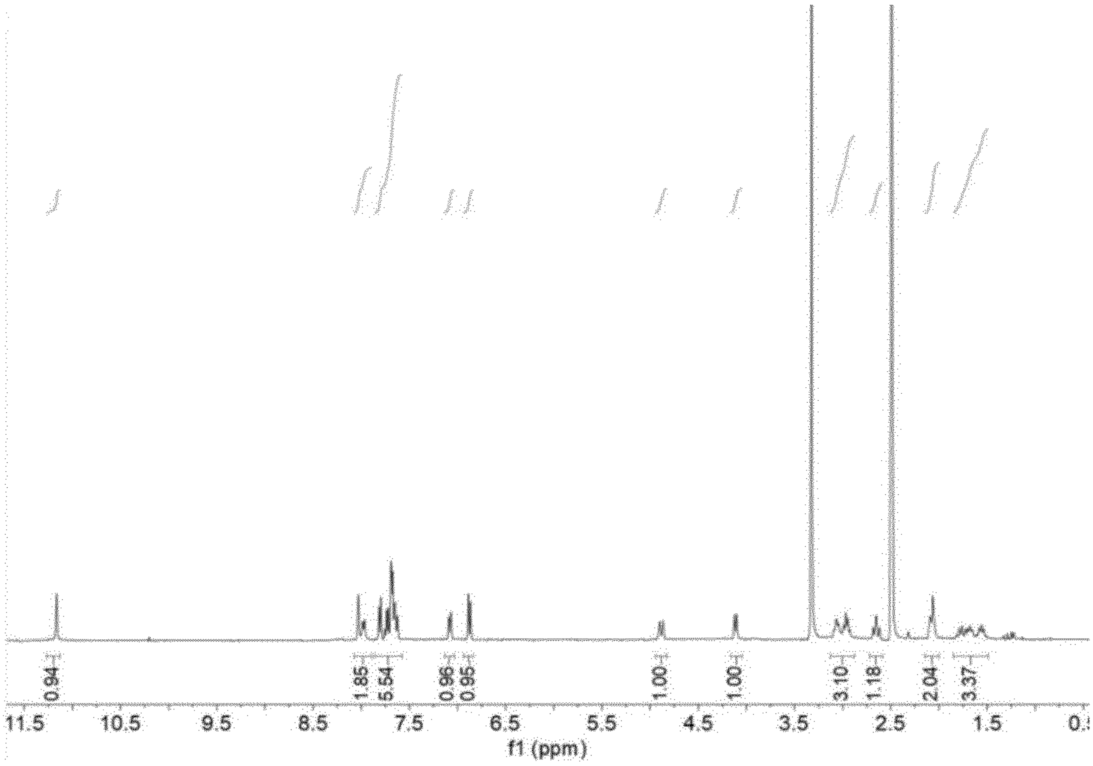
0.94
1.85
5.54
0.96
0.95
1.00
1.00
3.10
1.18
2.04
3.37
11.5 10.5 9.5 8.5 7.5 6.5 5.5 4.5 3.5 2.5 1.5
f1 (ppm)

FIG. 52

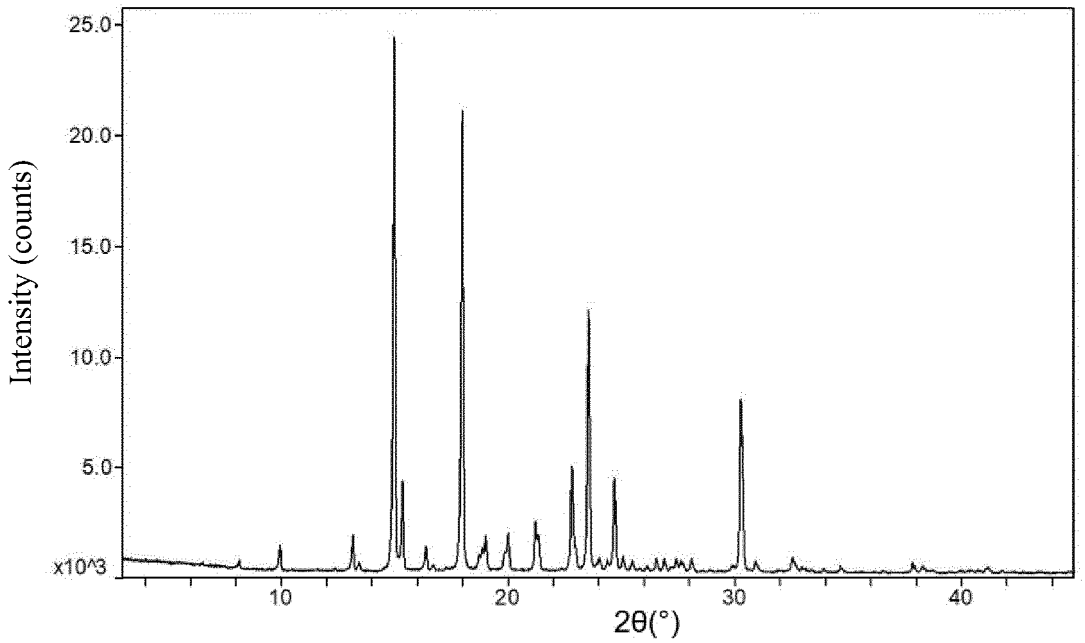
Intensity (counts)
25.0
20.0
15.0
10.0
5.0
x10^3
10 20 30 40
2θ(°)

FIG. 53

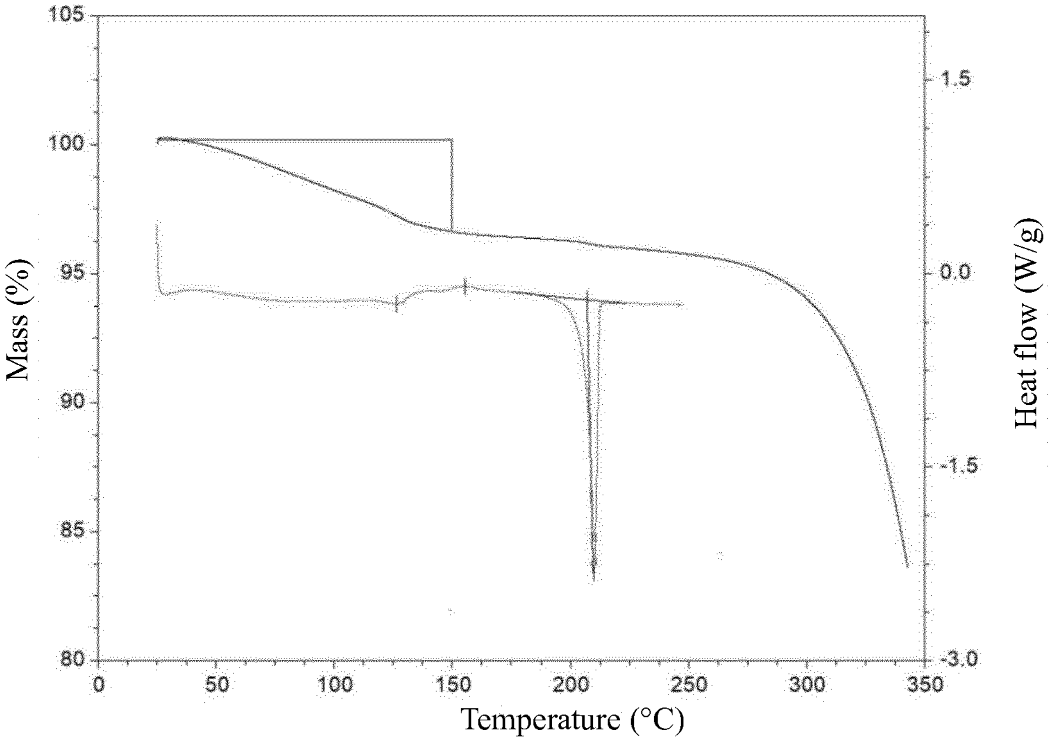
105
100
95
90
85
80
Mass (%)
1.5
0.0
-1.5
-3.0
Heat flow (W/g)
0
50
100
150
200
250
300
350
Temperature (°C)

FIG. 54

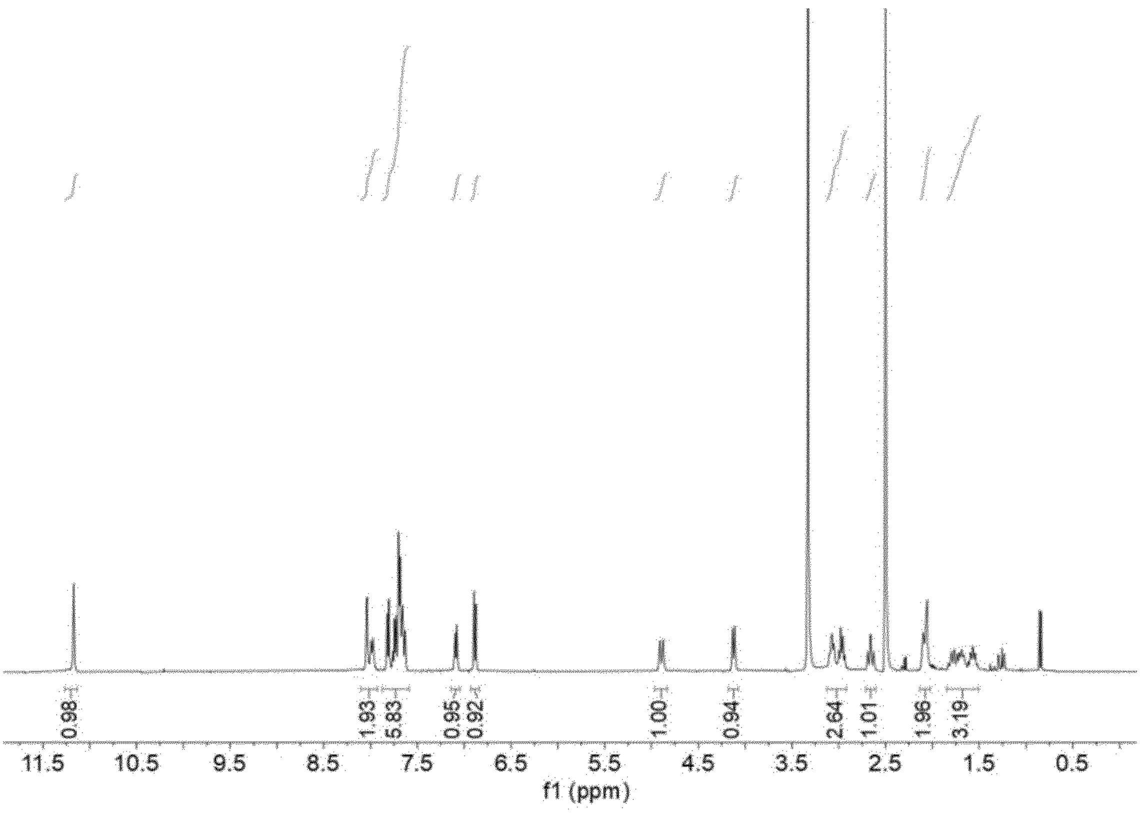
0.98
1.93
5.83
0.95
0.92
1.00
0.94
2.64
1.01
1.96
3.19
11.5
10.5
9.5
8.5
7.5
6.5
5.5
4.5
3.5
2.5
1.5
0.5
f1 (ppm)

FIG. 55

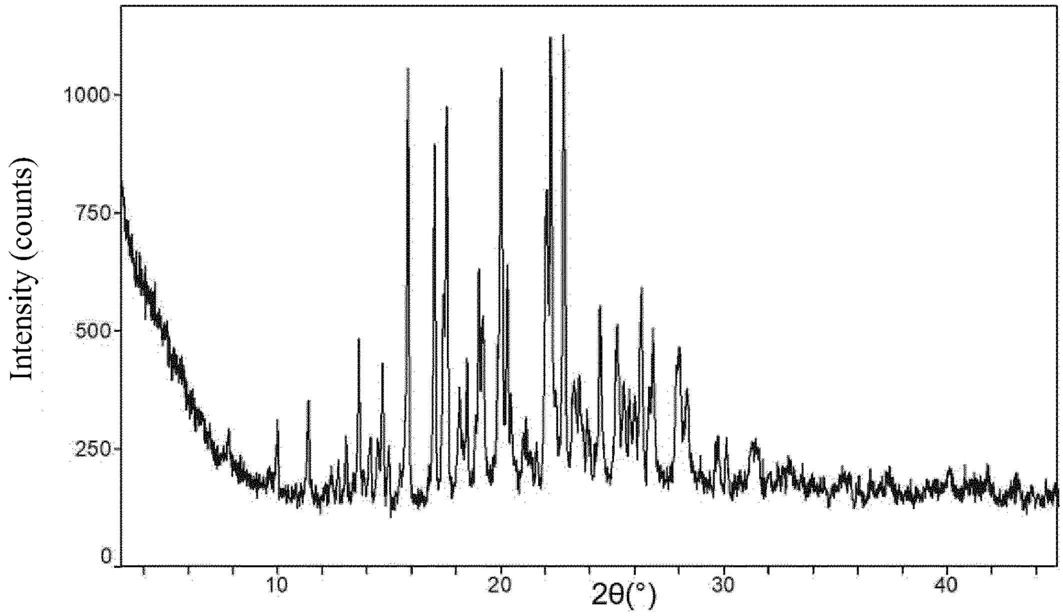
Intensity (counts)
1000
750
500
250
0
10
20
2θ(°)
30
40

FIG. 56

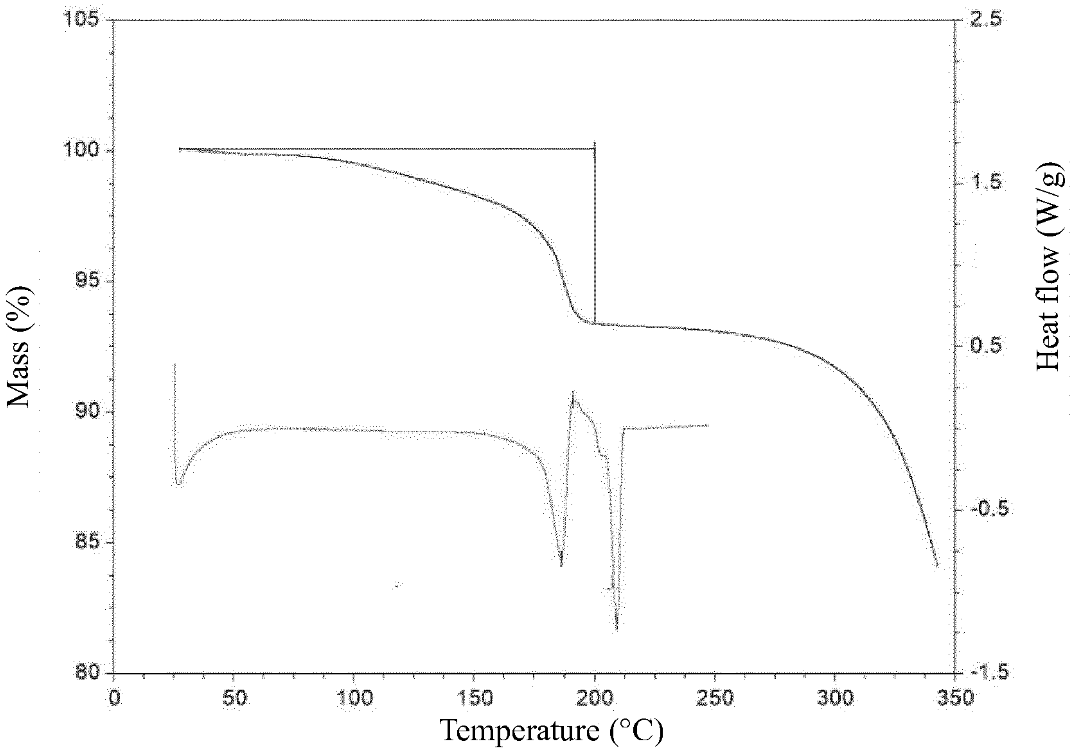
105
100
95
90
85
80
Mass (%)
2.5
1.5
0.5
-0.5
-1.5
Heat flow (W/g)
0
50
100
150
200
250
300
350
Temperature (°C)

FIG. 57

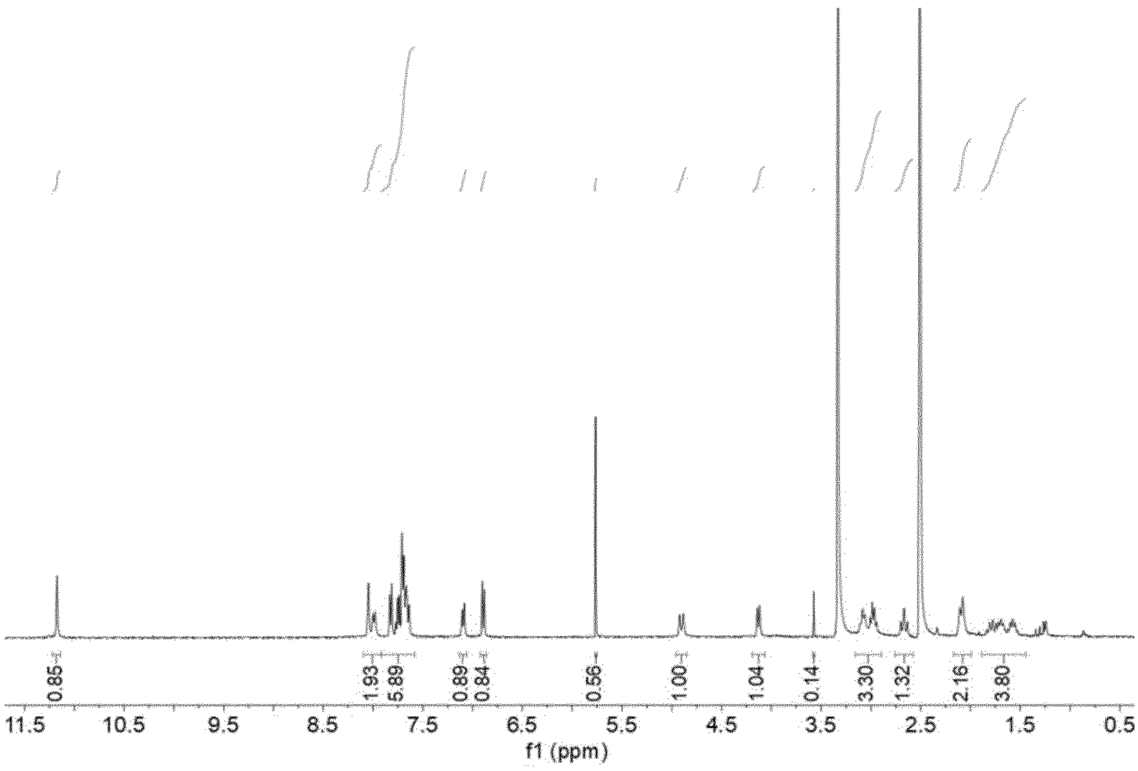
0.85
1.93
5.89
0.89
0.84
0.56
1.00
1.04
0.14
3.30
1.32
2.16
3.80
11.5 10.5 9.5 8.5 7.5 6.5 5.5 4.5 3.5 2.5 1.5 0.5
f1 (ppm)

FIG. 58

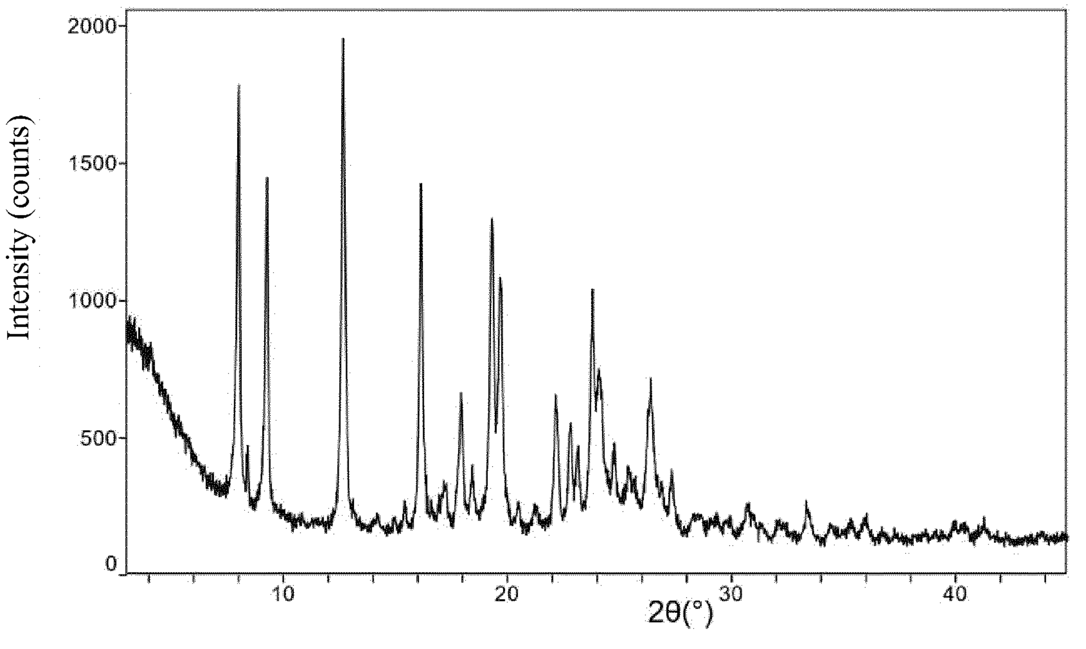
Intensity (counts)
2000
1500
1000
500
0
10
20
30
40
2θ(°)

FIG. 59

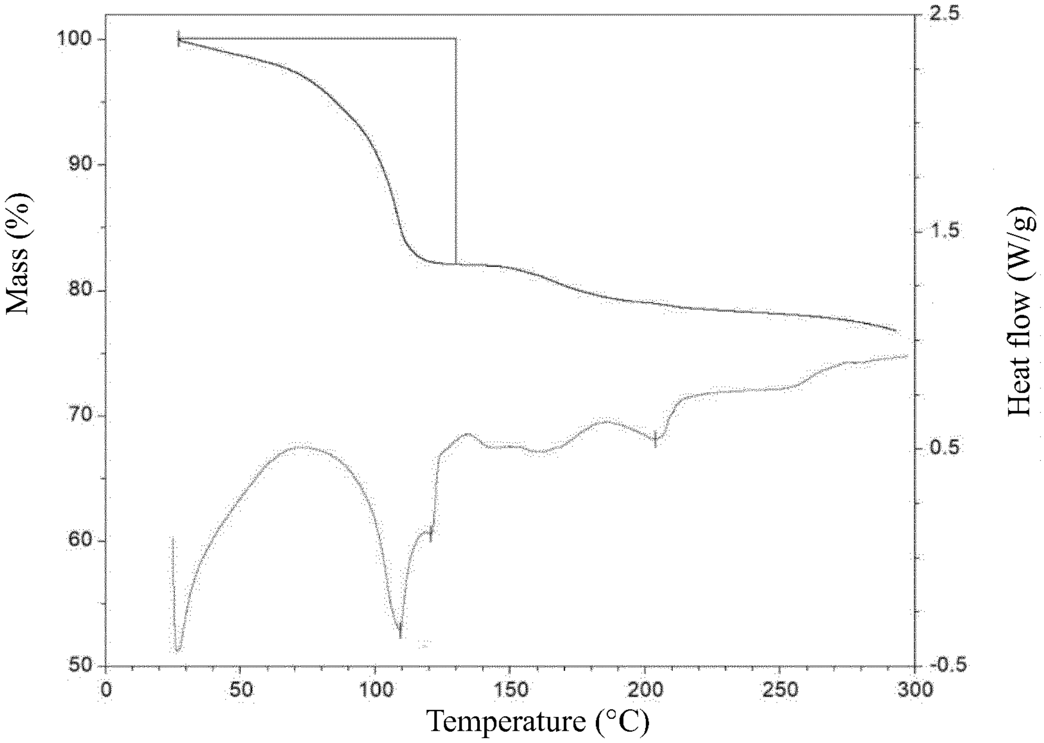
Mass (%)
Heat flow (W/g)
Temperature (°C)
100
90
80
70
60
50
2.5
1.5
0.5
-0.5
0
50
100
150
200
250
300

FIG. 60

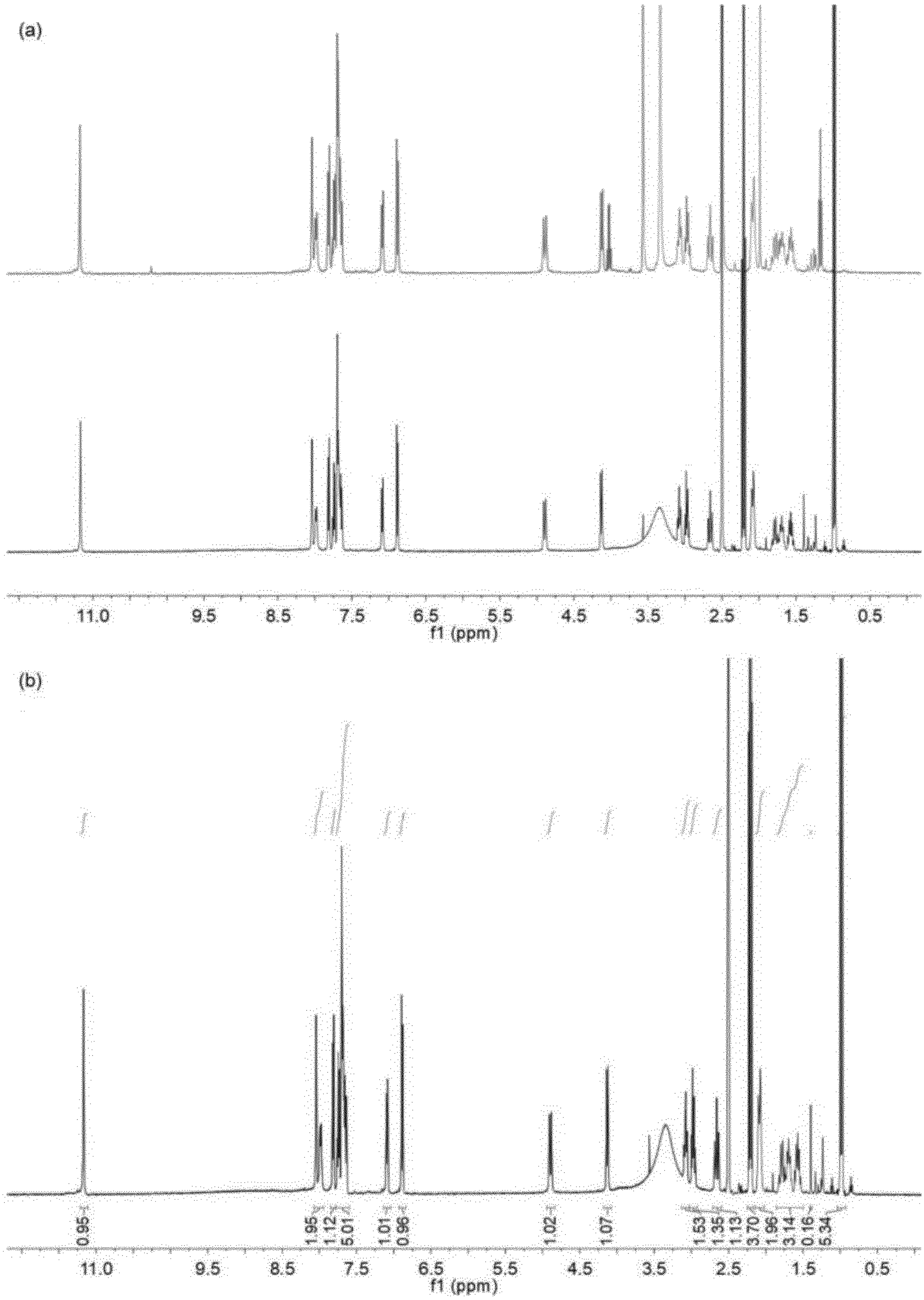
(a)
11.0
9.5
8.5
7.5
6.5
5.5
4.5
3.5
2.5
1.5
0.5
f1 (ppm)
(b)
0.95
1.95
1.12
5.01
1.01
0.96
1.02
1.07
1.53
1.35
1.13
3.70
1.96
3.14
0.16
5.34
11.0
9.5
8.5
7.5
6.5
5.5
4.5
3.5
2.5
1.5
0.5
f1 (ppm)

FIG. 61

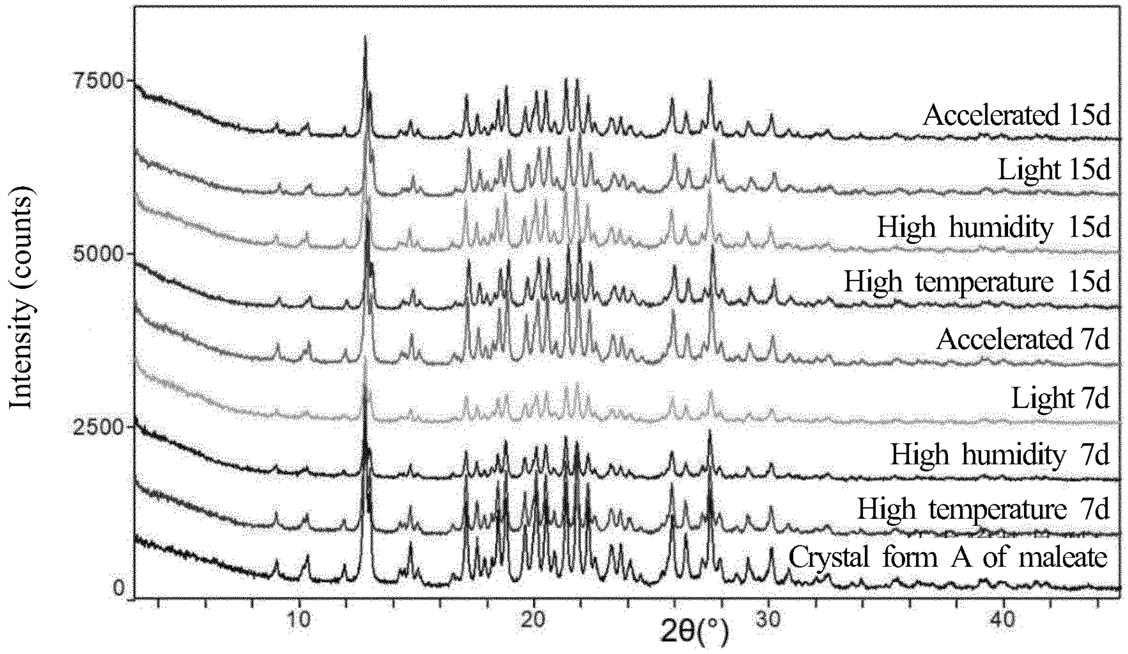

7500
5000
2500
0
Intensity (counts)
10
20
30
40
2θ(°)
Accelerated 15d
Light 15d
High humidity 15d
High temperature 15d
Accelerated 7d
Light 7d
High humidity 7d
High temperature 7d
Crystal form A of maleate


FIG. 62

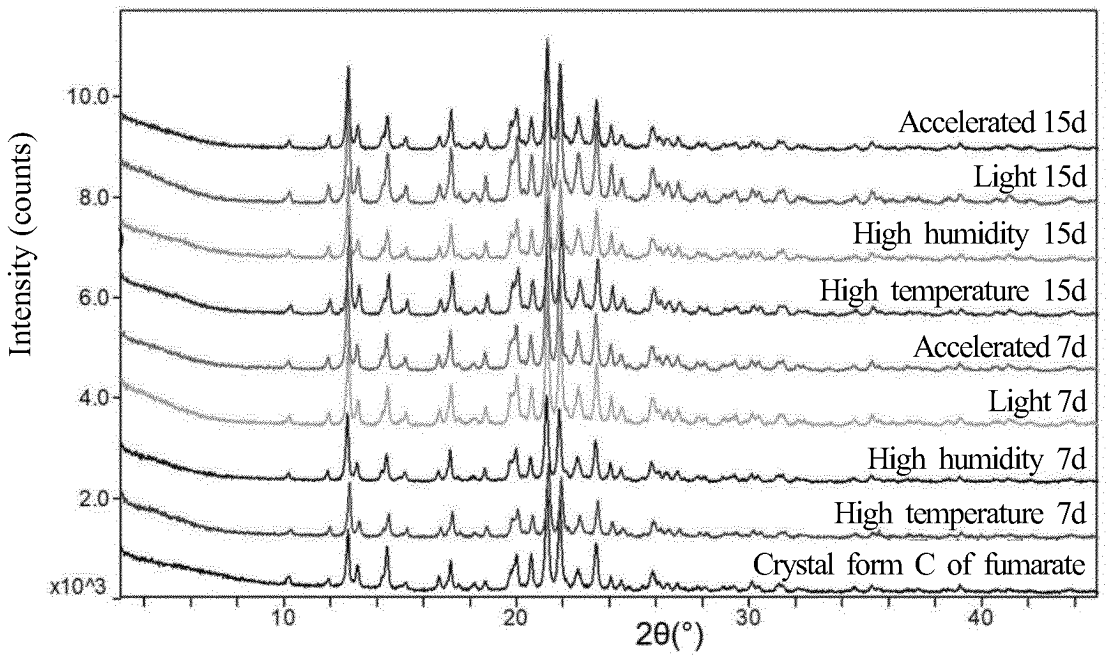

10.0
8.0
6.0
4.0
2.0
x10^3
Intensity (counts)
10
20
30
40
2θ(°)
Accelerated 15d
Light 15d
High humidity 15d
High temperature 15d
Accelerated 7d
Light 7d
High humidity 7d
High temperature 7d
Crystal form C of fumarate


FIG. 63

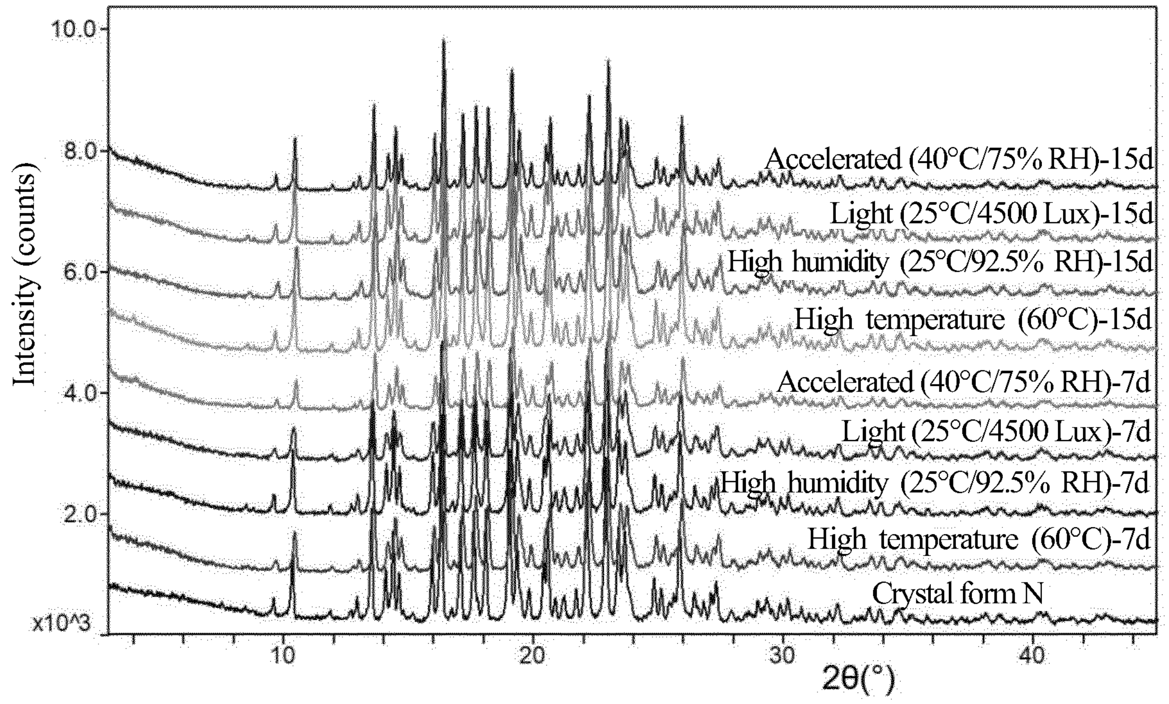
10.0
8.0
6.0
4.0
2.0
x10^3
Intensity (counts)
Accelerated (40°C/75% RH)-15d
Light (25°C/4500 Lux)-15d
High humidity (25°C/92.5% RH)-15d
High temperature (60°C)-15d
Accelerated (40°C/75% RH)-7d
Light (25°C/4500 Lux)-7d
High humidity (25°C/92.5% RH)-7d
High temperature (60°C)-7d
Crystal form N
10
20
30
40
2θ(°)

FIG. 64

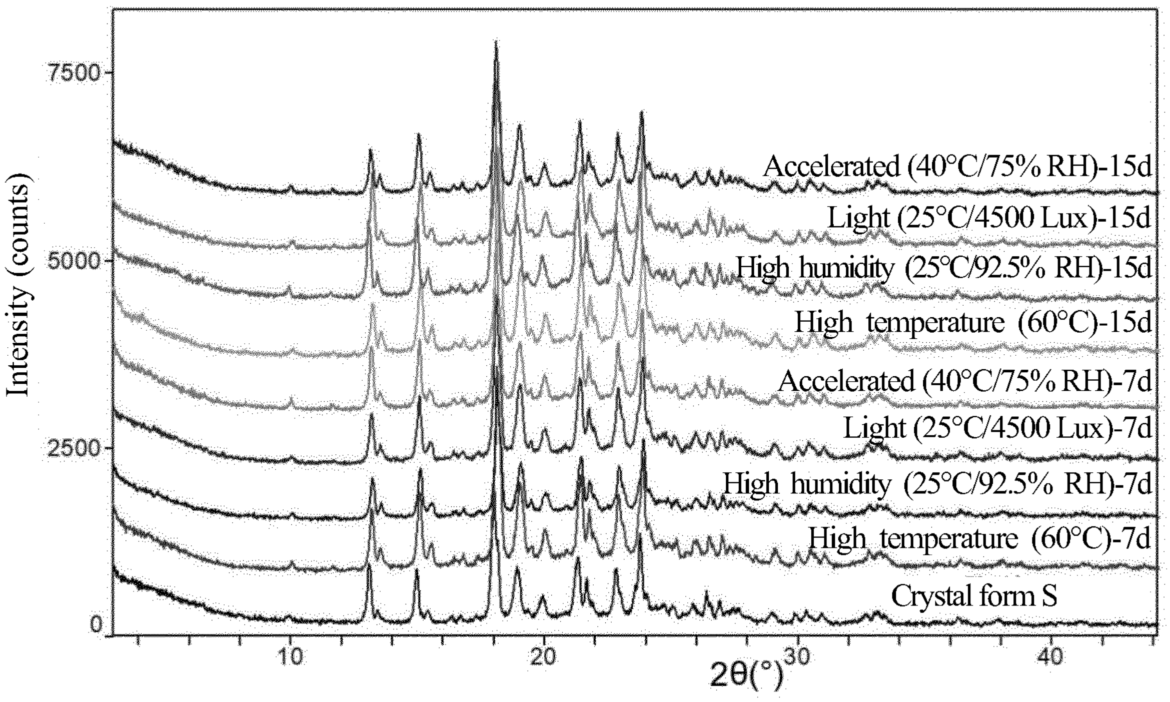
7500
5000
2500
0
Intensity (counts)
Accelerated (40°C/75% RH)-15d
Light (25°C/4500 Lux)-15d
High humidity (25°C/92.5% RH)-15d
High temperature (60°C)-15d
Accelerated (40°C/75% RH)-7d
Light (25°C/4500 Lux)-7d
High humidity (25°C/92.5% RH)-7d
High temperature (60°C)-7d
Crystal form S
10
20
30
40
2θ(°)

FIG. 65

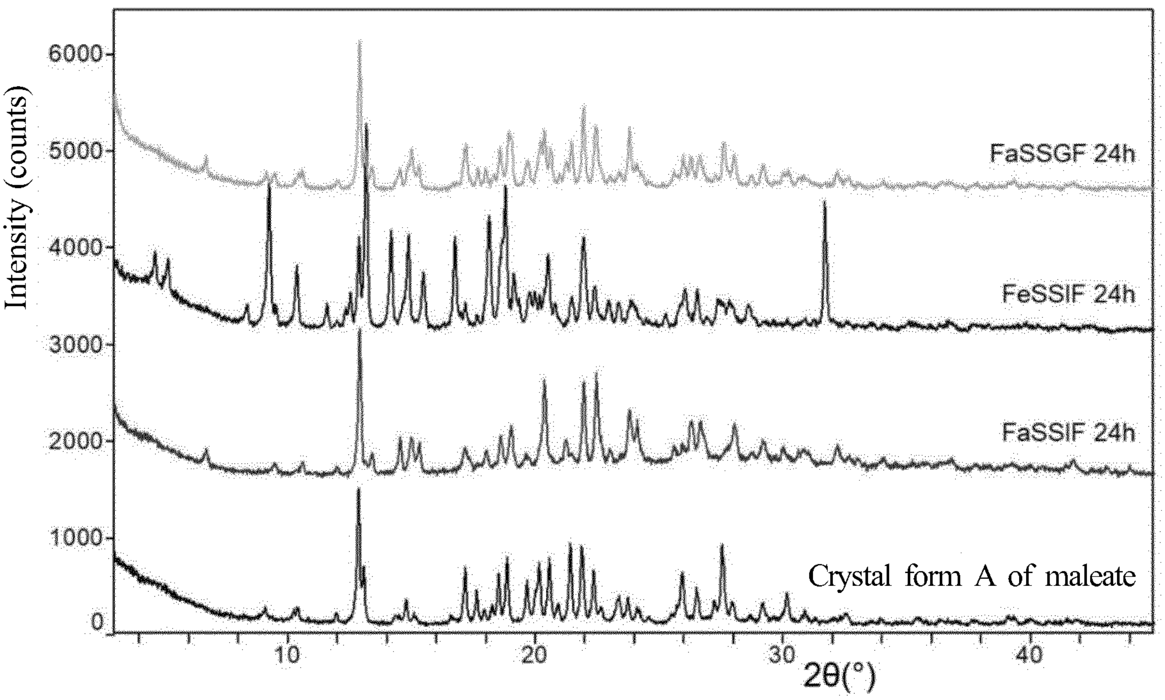
Intensity (counts)
6000
5000
4000
3000
2000
1000
0
FaSSGF 24h
FeSSIF 24h
FaSSIF 24h
Crystal form A of maleate
10
20
30
40
2θ(°)

FIG. 66

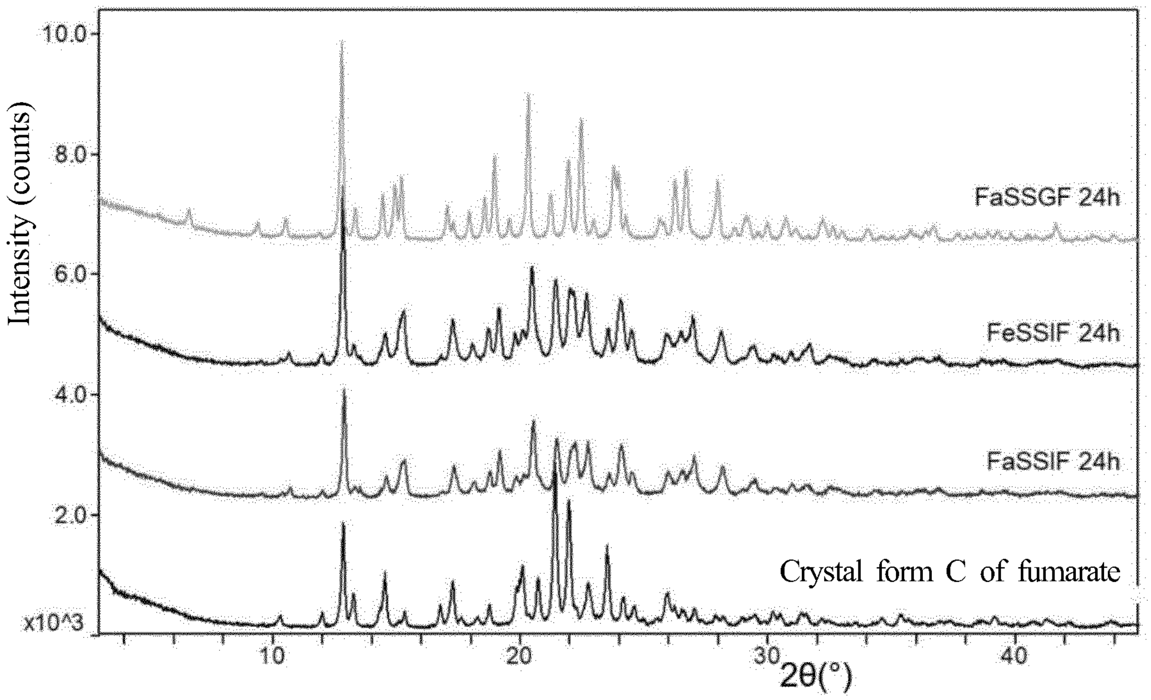
Intensity (counts)
10.0
8.0
6.0
4.0
2.0
x10^3
FaSSGF 24h
FeSSIF 24h
FaSSIF 24h
Crystal form C of fumarate
10
20
30
40
2θ(°)

FIG. 67

## INTERNATIONAL SEARCH REPORT

International application No.
**PCT/CN2023/116489**

### A. CLASSIFICATION OF SUBJECT MATTER

C07D 487/04 (2006.01)i；C07D223/16(2006.01)i；A61K31/55(2006.01)i

According to International Patent Classification (IPC) or to both national classification and IPC

### B. FIELDS SEARCHED

Minimum documentation searched (classification system followed by classification symbols)

IPC：C07D，A61K

Documentation searched other than minimum documentation to the extent that such documents are included in the fields searched

Electronic data base consulted during the international search (name of data base and, where practicable, search terms used)

STN(REGISTRY, CAPLUS), VEN, CNABS, CNTXT, CNKI: 精氨酸加压素, V2受体抑制剂, 盐, 晶型, 晶体, 苯并氮杂卓, 四氢吡咯, 马来酸, 富马酸, STN中的structural formula search, arginine vasopressin, V2 receptor inhibitor, salt, crystal, crystalline, polycrystal, benzazepine, tetrahydropyrrole, maleic acid, fumaric acid

### C. DOCUMENTS CONSIDERED TO BE RELEVANT

| Category* | Citation of document, with indication, where appropriate, of the relevant passages | Relevant to claim No. |
|---|---|---|
| A | US 5258510 A (OTSUKA PHARMACEUTICAL CO., LTD.) 02 November 1993 (1993-11-02)<br>description, columns 1-4 and 362 | 1-26 |
| A | CN 1106802 A (AMERICAN CYANAMID COMPANY) 16 August 1995 (1995-08-16)<br>description, pages 1-14 and 70 | 1-26 |
| A | US 5532235 A (AMERICAN CYANAMID COMPANY) 02 July 1996 (1996-07-02)<br>description, columns 1-6 and 87 | 1-26 |
| A | US 2007185323 A1 (ZARD SAMIR et al.) 09 August 2007 (2007-08-09)<br>description, pages 1-4 | 1-26 |
| A | CN 101258134 A (ORGANON & CO. et al.) 03 September 2008 (2008-09-03)<br>description, pages 1-4 | 1-26 |
| PX | WO 2022184172 A1 (SHANGHAI JEMINCARE PHARMACEUTICAL CO., LTD. et al.) 09 September 2022 (2022-09-09)<br>claims 1-14 | 1-26 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 October 2023** | **02 November 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | <br>Telephone No. |

Form PCT/ISA/210 (second sheet) (July 2022)

**INTERNATIONAL SEARCH REPORT**
**Information on patent family members**

International application No.
**PCT/CN2023/116489**

| Patent document cited in search report | | | Publication date (day/month/year) | Patent family member(s) | | | Publication date (day/month/year) |
|---|---|---|---|---|---|---|---|
| US | 5258510 | A | 02 November 1993 | US | 5559230 | A | 24 September 1996 |
| CN | 1106802 | A | 16 August 1995 | PE | 17995 | A1 | 18 July 1995 |
| | | | | IL | 110436 | A0 | 21 October 1994 |
| | | | | IL | 110436 | A | 10 December 2003 |
| | | | | EP | 0640592 | A1 | 01 March 1995 |
| | | | | EP | 0640592 | B1 | 30 December 1998 |
| | | | | HU | 9402223 | D0 | 28 September 1994 |
| | | | | HUT | 71548 | A | 28 December 1995 |
| | | | | HU | 221017 | B1 | 29 July 2002 |
| | | | | PL | 304496 | A1 | 06 February 1995 |
| | | | | PL | 181918 | B1 | 31 October 2001 |
| | | | | DK | 0640592 | T3 | 30 August 1999 |
| | | | | TW | 402592 | B | 21 August 2000 |
| | | | | GR | 3029758 | T3 | 30 June 1999 |
| | | | | ECSP | 941135 | A | 27 February 1995 |
| | | | | RU | 94027580 | A | 27 May 1997 |
| | | | | RU | 2149160 | C1 | 20 May 2000 |
| | | | | HK | 1011362 | A1 | 09 July 1999 |
| | | | | KR | 950003294 | A | 16 February 1995 |
| | | | | KR | 100347478 | B1 | 06 December 2002 |
| | | | | CZ | 179894 | A3 | 15 February 1995 |
| | | | | NZ | 299340 | A | 25 August 2000 |
| | | | | FI | 20011102 | A | 25 May 2001 |
| | | | | FI | 111076 | B | 30 May 2003 |
| | | | | FI | 20011100 | A | 25 May 2001 |
| | | | | FI | 111077 | B | 30 May 2003 |
| | | | | AU | 6877694 | A | 09 February 1995 |
| | | | | AU | 676737 | B2 | 20 March 1997 |
| | | | | BR | 1100538 | A | 08 August 2000 |
| | | | | SG | 52312 | A1 | 28 September 1998 |
| | | | | ZA | 945604 | B | 09 March 1995 |
| | | | | NO | 942817 | D0 | 28 July 1994 |
| | | | | NO | 942817 | L | 30 January 1995 |
| | | | | NO | 308601 | B1 | 02 October 2000 |
| | | | | JPH | 07179430 | A | 18 July 1995 |
| | | | | JP | 3630449 | B2 | 16 March 2005 |
| | | | | US | 5512563 | A | 30 April 1996 |
| | | | | ZA | 9405604 | B | 09 March 1995 |
| | | | | NZ | 264116 | A | 26 November 1996 |
| | | | | CA | 2128955 | A1 | 30 January 1995 |
| | | | | CA | 2128955 | C | 14 November 2006 |
| | | | | DE | 69415614 | D1 | 11 February 1999 |
| | | | | DE | 69415614 | T2 | 26 August 1999 |
| | | | | FI | 943542 | A0 | 28 July 1994 |
| | | | | FI | 943542 | A | 30 January 1995 |
| | | | | FI | 108433 | B | 31 January 2002 |
| | | | | FI | 20011101 | A | 25 May 2001 |
| | | | | FI | 111075 | B | 30 May 2003 |
| | | | | ATE | 175198 | T1 | 15 January 1999 |
| | | | | ES | 2125377 | T3 | 01 March 1999 |

Form PCT/ISA/210 (patent family annex) (July 2022)

**INTERNATIONAL SEARCH REPORT**
**Information on patent family members**

International application No.
**PCT/CN2023/116489**

| Patent document cited in search report | Publication date (day/month/year) | Patent family member(s) | Publication date (day/month/year) |
|---|---|---|---|
| | | SK 88094 A3 | 10 May 1995 |
| | | SK 281194 B6 | 18 January 2001 |
| US 5532235 A | 02 July 1996 | ZA 96299 B | 15 July 1997 |
| | | EP 0804440 A1 | 05 November 1997 |
| | | EP 0804440 B1 | 19 December 2007 |
| | | JPH 10512867 A | 08 December 1998 |
| | | JP 4219978 B2 | 04 February 2009 |
| | | WO 9622295 A1 | 25 July 1996 |
| | | ES 2297835 T3 | 01 May 2008 |
| | | MX 9705415 A | 29 November 1997 |
| | | BR 9607186 A | 11 November 1997 |
| | | BR 9607186 B1 | 13 January 2009 |
| | | NZ 302159 A | 28 January 2000 |
| | | IL 116773 A0 | 14 May 1996 |
| | | IL 116773 A | 06 December 2000 |
| | | AR 002268 A1 | 11 March 1998 |
| | | TW 426684 B | 21 March 2001 |
| | | CA 2210632 A1 | 25 July 1996 |
| | | CZ 224897 A3 | 15 April 1998 |
| | | DE 69637369 D1 | 31 January 2008 |
| | | DE 69637369 T2 | 22 January 2009 |
| | | AT 381570 T | 15 January 2008 |
| | | HU 9702219 A2 | 28 July 1998 |
| | | HU 9702219 A3 | 28 May 1999 |
| | | US 5719278 A | 17 February 1998 |
| | | EA 199700111 A1 | 30 December 1997 |
| | | EA 000607 B1 | 29 December 1999 |
| | | AU 4775596 A | 07 August 1996 |
| | | DK 0804440 T3 | 31 March 2008 |
| US 2007185323 A1 | 09 August 2007 | FR 2867187 A1 | 09 September 2005 |
| | | FR 2867187 B1 | 12 October 2007 |
| | | EP 1720827 A1 | 15 November 2006 |
| | | JP 2007526288 A | 13 September 2007 |
| | | WO 2005085183 A1 | 15 September 2005 |
| | | CA 2557731 A1 | 15 September 2005 |
| | | IL 177649 A0 | 31 December 2006 |
| CN 101258134 A | 03 September 2008 | ZA 200707090 A | 29 October 2008 |
| WO 2022184172 A1 | 09 September 2022 | CA 3210848 A1 | 09 September 2022 |
| | | TW 202246273 A | 01 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202211080826
- CN 2022079350 W

### Non-patent literature cited in the description

- Test standard: Chinese Pharmacopoeia, vol. IV, 0451